# EUROPEAN PATENT APPLICATION

(11) **EP 4 438 726 A2**
(43) Date of publication of application: **02.10.2024**
(21) Application number: 24162711.6
(22) Date of filing: 11.03.2024
(51) Int. Cl.: C12N 9/22, C12N 9/88, C12N 15/82

(54) **COMPOSITIONS AND METHODS COMPRISING PLANTS WITH INCREASED SEED AMINO ACID CONTENT**

(30) Priority: 10.03.2023 US 202363489568 P
(71) Applicant: Benson Hill, Inc., St. Louis MO 63132 (US)
(72) Inventor: Begemann, Matthew Brett, St. Louis, 63132 (US); Goettel, Herbert Wolfgang, St. Louis, 63132 (US); Neumann, Gina Christine, St. Louis, 63132 (US); Zess, Erin, St. Louis, 63132 (US); Moshiri, Farhad, St. Louis, 63132 (US)
(74) Representative: Inspicos P/S

(57) **Abstract**

Provided herein are genome edited plants and plant parts that comprise increased amino acid content. In particular, the amino acids include tryptophan (Trp) and methionine (Met). Seed of the genome edited plants and plant parts can comprise increased Trp and/or Met. The genome edited plants and plant parts have genetic mutations in an anthranilate synthase gene including a gene encoding an alpha subunit of anthranilate synthase and/or a homocysteine S-methyltransferase gene. The genome edited plants and plant parts include soybean and pea. Also provided herein are compositions and methods of producing such plants and plant parts, and plant products including compositions comprising increased Trp and/or Met content.

## Description

### RELATED APPLICATION

This application claims priority to U.S. Provisional Application No. 63/489,568, filed March 10, 2023, which is incorporated herein in its entirety.

### REFERENCE TO A SEQUENCE LISTING SUBMITTED

### ELECTRONICALLY AS AN XML FILE

The instant application contains a Sequence Listing which has been submitted in xml ST.26 format via USPTO Patent Center and is hereby incorporated by reference in its entirety. Said xml copy, created on March 8, 2024, is named 2024-03-08_B88552_1530US_SL and is 77.0 KB in size.

### FIELD OF THE INVENTION

The present disclosure relates to plants and plant parts having increased seed amino acid content, and associated methods and compositions thereof.

### BACKGROUND OF THE INVENTION

Animals raised for food lack the ability to manufacture a number of amino acids and therefore are required to obtain these amino acids from their diet. The amino acids which must be obtained from the diet are referred to as essential amino acids. Plants are able to synthesize all twenty of the essential amino acids and therefore serve as the primary source of these amino acids for animals.

Soybean is an economically important legume with an estimated 2021 world-wide production of 383 million metric tons and estimated US crop value of $57.5 billion. Soybean seeds have an average protein content of about 40%, high as compared to other seed crops. Because of their high protein content, soybeans are extensively used as a major ingredient in animal feed with the majority of soybean meal produced used for providing an amino acid and protein source in feed for poultry, swine, cattle, fish, and other farm animals. Although the amino acid profile of soybean meal complements that of cereal grains to help meet amino acid requirements for animals, soybean meal is still deficient in essential amino acids such as tryptophan (Trp) and methionine (Met). To overcome this limitation, the animal industry supplements soybean-based feed with synthetic Trp and/or Met, a process that significantly adds to cost of the animal feed. Additionally,
when soybean meal is formulated in feed rations, the inclusion rate is typically calculated based on satisfying the most limiting essential amino acids. This formulation results in the remaining essential amino acids being formulated in excess of dietary requirements. The excess amino acids end up as waste.

Thus, there is a need for soybean plants that produce elevated levels of essential amino acids such as Trp and Met to improve quality of feed sources for animals.

### SUMMARY OF THE INVENTION

Plants and plant parts comprising a genetic mutation that increases tryptophan (Trp) and/or methionine (Met) seed amino acid content are provided. The genetic mutation is located in at least one endogenous anthranilate synthase (*AS*) gene or homolog thereof and/or in at least one endogenous homocysteine S-methyltransferase (*HMT*) gene or homolog thereof. Compositions and methods for producing such plants and plant parts, and products produced from such plants and plant parts are also provided.

In one aspect, the present disclosure provides a plant or plant part comprising a genetic mutation in at least one endogenous *AS* gene or homolog thereof encoding an anthranilate synthase and/or *HMT* gene or homolog thereof encoding a homocysteine *S-*methyltransferase, wherein the mutated plant or plant part comprises increased tryptophan (Trp) and/or methionine (Met) amino acid content as compared to a control.

In some embodiments of the above aspect, the plant or plant part is a seed.

In some embodiments of the above aspect, the genetic mutation comprises one or more insertions, substitutions, or deletions in the at least one endogenous *AS* gene or homolog thereof and/or *HMT* gene or homolog thereof.

In some embodiments of the above aspect, the *AS* gene or homolog thereof comprises an *ASA* gene or homolog thereof encoding an anthranilate synthase alpha subunit. In some embodiments, the genetic mutation comprises an in-frame deletion in the *ASA* gene or homolog thereof. In some embodiments, the in-frame deletion comprises a deletion of 3 to 30 nucleotides.

In some embodiments of the above aspect, the genetic mutation is located in an enzyme regulatory region of the encoded ASA protein. In some embodiments, the enzyme regulatory region comprises a region that binds Trp. In some embodiments, the *AS* gene or homolog thereof comprising the genetic mutation encodes an anthranilate synthase that is insensitive to or has reduced sensitivity to Trp feedback inhibition.

In some embodiments of the above aspect, the genetic mutation is located in a *ASA1* gene or homolog thereof: (i) comprising a nucleic acid sequence having at least 80% sequence identity to the nucleic acid sequence set forth as SEQ ID NO: 1 and encoding an active anthranilate synthase; (ii) comprising the nucleic acid sequence set forth as SEQ ID NO: 1; (iii) encoding an anthranilate synthase alpha subunit comprising an amino acid sequence having at least 80% sequence identity to an amino acid sequence set forth as SEQ ID NO: 2, wherein the anthranilate synthase alpha subunit is active; and/or (iv) encoding an anthranilate synthase alpha subunit comprising an amino acid sequence set forth as SEQ ID NO: 2.

In some embodiments of the above aspect, the genetic mutation comprises a deletion in the *HMT* gene or homolog thereof. In some embodiments, the deletion comprises a deletion of 1 to 70 nucleotides.

In some embodiments of the above aspect, the genetic mutation is located in a *HMT1* gene or homolog thereof: (i) comprising a nucleic acid sequence having at least 80% sequence identity to the nucleic acid sequence set forth as SEQ ID NO: 9 and encoding an active homocysteine *S*-methyltransferase; (ii) comprising the nucleic acid sequence set forth as SEQ ID NO: 9; (iii) encoding a homocysteine *S*-methyltransferase comprising an amino acid sequence having at least 80% sequence identity to an amino acid sequence set forth as SEQ ID NO: 10, wherein the homocysteine *S*-methyltransferase is active; and/or (iv) encoding a homocysteine *S*-methyltransferase comprising an amino acid sequence set forth as SEQ ID NO: 10.

In some embodiments of the above aspect, the genetic mutation is located in a *HMT3* gene or homolog thereof: (i) comprising a nucleic acid sequence having at least 80% sequence identity to the nucleic acid sequence set forth as SEQ ID NO: 13 and encodes an active homocysteine *S*-methyltransferase; (ii) comprising the nucleic acid sequence set forth as SEQ ID NO: 13; (iii) encoding a homocysteine *S*-methyltransferase comprising an amino acid sequence having at least 80% sequence identity to an amino acid sequence set forth as SEQ ID NO: 14, wherein the homocysteine *S*-methyltransferase is active; and/or (iv) encoding a homocysteine *S*-methyltransferase comprising an amino acid sequence set forth as SEQ ID NO: 14.

In some embodiments of the above aspect, the plant or plant part comprises: (i) an in-frame deletion of nucleotides 952 to 957 of *ASA1* gene having the nucleic acid sequence set forth as SEQ ID NO: 1, or a nucleic acid sequence comprising SEQ ID NO: 3 when the deletion of (i) is introduced; (ii) an in-frame deletion of nucleotides 948 to 959 of *ASA1* gene having the nucleic acid sequence set forth as SEQ ID NO: 1, or a nucleic acid sequence comprising SEQ ID NO: 4 when the deletion of (ii) is introduced; (iii) an in-frame deletion of nucleotides 938 to 961 of *ASA1* gene having the nucleic acid sequence set forth as SEQ ID NO: 1, or a nucleic acid sequence comprising SEQ ID NO: 5 when the deletion of (iii) is introduced; (iv) a deletion of nucleotides 712 to 714 of *HMT1* gene having the nucleic acid sequence set forth as SEQ ID NO: 9, or a nucleic acid sequence comprising SEQ ID NO: 11 when the deletion of (iv) is introduced; (v) a deletion of nucleotides 695 to 760 of *HMT1* gene having the nucleic acid sequence set forth as SEQ ID NO: 9, or a nucleic acid sequence comprising SEQ ID NO: 12 when the deletion of (v) is introduced; (vi) a deletion of nucleotides 2783 to 2784 of *HMT3* gene having the nucleic acid sequence set forth as SEQ ID NO: 13, or a nucleic acid sequence comprising SEQ ID NO: 15 when the deletion of (vi) is introduced; or (vii) a deletion of nucleotides 2778 to 2790 of *HMT3* gene having the nucleic acid sequence set forth as SEQ ID NO: 13, or a nucleic acid sequence comprising SEQ ID NO: 16 when the deletion of (vii) is introduced.

In some embodiments of the above aspect, *the ASA1* gene comprising the genetic mutation encodes an anthranilate synthase alpha subunit having an amino acid sequence set forth as any one of SEQ ID NOs: 6-8.

In some embodiments of the above aspect, Trp and/or Met content in the seed is 1.1-fold to 10-fold greater than that of the control. In some embodiments of the above aspect, the S-methylmethionine (SMM) level in vegetative tissue is increased as compared to the control.

In some embodiments of the above aspect, the control comprises a corresponding plant, plant part, or seed without the genetic mutation.

In some embodiments of the above aspect, the plant or plant part is from a legume. In some embodiments, the legume is soybean *(Glycine max*) or pea *(Pisum sativum).*

In another aspect, the present disclosure provides a population of plants or plant parts comprising a plant or plant part described hereinabove. In some embodiments, the population is a population of seeds, and the plant or plant part is a seed.

In still another aspect, the present disclosure provides a seed composition produced from a plant or plant part, or a population of plants or plant parts described hereinabove.

In yet another aspect, the present disclosure provides a protein composition produced from a plant or plant part, a population of plants or plant parts, or a seed composition described hereinabove.

In another aspect, the present disclosure provides a method for increasing tryptophan (Trp) and/or methionine (Met) amino acid content produced by a plant, the method comprising: introducing editing reagents into a plant, plant part, or plant cell to produce a mutated plant, plant part, or plant cell comprising a genetic mutation at at least one target site, wherein the at least one target site comprises an endogenous *AS* gene or homolog thereof encoding an anthranilate synthase and/or an endogenous *HMT* gene or homolog thereof encoding a homocysteine *S*-methyltransferase, and wherein Trp and/or Met content in a plant or plant part generated from the mutated plant part, plant part, or plant cell, is increased as compared to a control.

In some embodiments of the above method aspect, the generated plant or plant part is a seed.

In some embodiments of the above method aspect, the editing reagents comprise: at least one RNA-guided nuclease, or a polynucleotide encoding the at least one RNA-guided nuclease; and at least one guide RNA (gRNA), or a polynucleotide encoding the at least one gRNA, wherein the at least one gRNA is complementary to genomic sequence of the endogenous *AS* gene or homolog thereof and/or the endogenous *HMT* gene or homolog thereof, and wherein the at least one gRNA is capable of binding the at least one RNA-guided nuclease to form a ribonucleoprotein (RNP) complex.

In some embodiments of the above method aspect, the introducing comprises introducing the editing reagents into the plant cell, and culturing the plant cell to regenerate a plant or plant part comprising the genetic mutation.

In some embodiments of the above method aspect, Trp and/or Met content in the seed is 1.1-fold to 10-fold greater than that of the control.

In some embodiments of the above method aspect, the control comprises a corresponding plant, plant part, plant cell, or seed without the genetic mutation.

In some embodiments of the above method aspect, the at least one RNA-guided nuclease comprises a CRISPR (Clustered Regularly Interspaced Palindromic Repeats) nuclease. In some embodiments of the above method aspect, a proto-adjacent motif (PAM) recognized by the CRISPR nuclease comprises a nucleic acid sequence of TTTA or TTTG. In some embodiments, the CRISPR nuclease comprises a Type II CRISPR system nuclease or a Type V CRISPR system nuclease. In some embodiments, the Type II CRISPR system nuclease comprises a Cas9 nuclease. In some embodiments, the Type V CRISPR system nuclease comprises a Cas12a (Cpf1) nuclease or a Cms1 nuclease. In some embodiments, the Type V CRISPR nuclease comprises the Cas12a (Cpf1) nuclease or an ortholog thereof.

In some embodiments of the above method aspect, the Type V CRISPR nuclease is encoded by a nucleotide sequence set forth as SEQ ID NO: 25. In some embodiments of the above method aspect, the CRISPR nuclease has an amino acid sequence set forth as SEQ ID NO: 26.

In some embodiments of the above method aspect, the at least one gRNA comprises: (a) a nucleic acid sequence having at least 80% sequence identity to the nucleic acid sequence set forth as any one of SEQ ID NOs: 19-21; or (b) a nucleic acid sequence having the nucleic acid sequence set forth as any one of SEQ ID NOs: 19-21.

In some embodiments of the above method aspect, the at least one target site comprises a nucleic acid sequence set forth as any one of SEQ ID NOs: 22-24.

In some embodiments of the above method aspect, the genetic mutation comprises one or more insertions, substitutions, or deletions in the endogenous *AS* gene or homolog thereof and/or in the endogenous *HMT* gene or homolog thereof.

In some embodiments of the above method aspect, the endogenous *AS* gene or homolog thereof is an endogenous *ASA* gene or homolog thereof. In some embodiments, the genetic mutation comprises an in-frame deletion in the *ASA* gene or homolog thereof. In some embodiments, the in-frame deletion comprises a deletion of 3 to 30 nucleotides.

In some embodiments of the above method aspect, the genetic mutation is located in an enzyme regulatory region of the encoded ASA protein. In some embodiments, the enzyme regulatory region comprises a region that binds Trp. In some embodiments, the *AS* gene or homolog thereof comprising the genetic mutation encodes an anthranilate synthase that is insensitive to or has reduced sensitivity to Trp feedback inhibition.

In some embodiments of the above method aspect, the genetic mutation is located in a *ASA1* gene or homolog thereof: (i) comprising a nucleic acid sequence having at least 80% sequence identity to the nucleic acid sequence set forth as SEQ ID NO: 1 and encoding an active anthranilate synthase; (ii) comprising the nucleic acid sequence set forth as SEQ ID NO: 1; (iii) encoding an anthranilate synthase alpha subunit comprising an amino acid sequence having at least 80% sequence identity to an amino acid sequence set forth as SEQ ID NO: 2, wherein the anthranilate synthase alpha subunit is active; and/or (iv) encoding an anthranilate synthase alpha subunit comprising an amino acid sequence set forth as SEQ ID NO: 2.

In some embodiments of the above method aspect, the *ASA1* gene or homolog thereof comprising the genetic mutation encodes an anthranilate synthase alpha subunit having an amino acid sequence set forth as any one of SEQ ID NOs: 6-8.

In some embodiments of the above method aspect, the genetic mutation comprises a deletion in the *HMT* gene or homolog thereof. In some embodiments, the deletion comprises a deletion of 1 to 70 nucleotides.

In some embodiments of the above method aspect, the genetic mutation is located in an *HMT1* gene or homolog thereof: (i) comprising a nucleic acid sequence having at least 80% sequence identity to the nucleic acid sequence set forth as SEQ ID NO: 9 and encoding an active homocysteine *S*-methyltransferase; (ii) comprising the nucleic acid sequence set forth as SEQ ID NO: 9; (iii) encoding a homocysteine *S*-methyltransferase comprising an amino acid sequence having at least 80% sequence identity to an amino acid sequence set forth as SEQ ID NO: 10, wherein the homocysteine *S*-methyltransferase is active; and/or (iv) encoding a homocysteine *S*-methyltransferase comprising an amino acid sequence set forth as SEQ ID NO: 10.

In some embodiments of the above method aspect, the genetic mutation is located in an *HMT3* gene or homolog thereof: (i) comprising a nucleic acid sequence having at least 80% sequence identity to the nucleic acid sequence set forth as SEQ ID NO: 13 and encodes an active homocysteine *S*-methyltransferase; (ii) comprising the nucleic acid sequence set forth as SEQ ID NO: 13; (iii) encoding a homocysteine *S*-methyltransferase comprising an amino acid sequence having at least 80% sequence identity to an amino acid sequence set forth as SEQ ID NO: 14, wherein the homocysteine *S*-methyltransferase is active; and/or (iv) encoding a homocysteine *S*-methyltransferase comprising an amino acid sequence set forth as SEQ ID NO: 14.

In some embodiments of the above method aspect, the plant, plant part, or plant cell comprising the genetic mutation comprises: (i) an in-frame deletion of nucleotides 952 to 957 of *ASA1* gene having the nucleic acid sequence set forth as SEQ ID NO: 1, or a nucleic acid sequence comprising SEQ ID NO: 3 when the deletion of (i) is introduced; (ii) an in-frame deletion of nucleotides 948 to 959 of *ASA1* gene having the nucleic acid sequence set forth as SEQ ID NO: 1, or a nucleic acid sequence comprising SEQ ID NO: 4 when the deletion of (ii) is introduced; (iii) an in-frame deletion of nucleotides 938 to 961 of *ASA1* gene having the nucleic acid sequence set forth as SEQ ID NO: 1, or a nucleic acid sequence comprising SEQ ID NO: 5 when the deletion of (iii) is introduced; (iv) a deletion of nucleotides 712 to 714 of *HMT1* gene having the nucleic acid sequence set forth as SEQ ID NO: 9, or a nucleic acid sequence comprising SEQ ID NO: 11 when the deletion of (iv) is introduced; (v) a deletion of nucleotides 695 to 760 of *HMT1* gene having the nucleic acid sequence set forth as SEQ ID NO: 9, or a nucleic acid sequence comprising SEQ ID NO: 12 when the deletion of (v) is introduced; (vi) a deletion of nucleotides 2783 to 2784 of *HMT3* gene having the nucleic acid sequence set forth as SEQ ID NO: 13, or a nucleic acid sequence comprising SEQ ID NO: 15 when the deletion of (vi) is introduced; or (vii) a deletion of nucleotides 2778 to 2790 of *HMT3* gene having the nucleic acid sequence set forth as SEQ ID NO: 13, or a nucleic acid sequence comprising SEQ ID NO: 16 when the deletion of (vii) is introduced.

In some embodiments of the above method aspect, the plant, plant part, or plant cell is from a legume. In some embodiments, the legume is soybean (*Glycine max*) or pea (*Pisum sativum*)*.*

In yet another aspect, the present disclosure provides a plant, plant part, plant cell, or population of plants produced by the method described hereinabove. In some embodiments, seed of plants generated from the plant, plant part, plant cell, or population of plants comprises increased Trp and/or Met content as compared to a control.

In still another aspect, the present disclosure provides a seed composition produced from a plant, plant part, plant cell, or population of plants described hereinabove, wherein the seed comprise increased Trp and/or Met content compared to a control. In some embodiments, the increased Trp and/or Met content is 1.1-fold to 10-fold greater than that of the control.

In some embodiments of the above plant, plant part, plant cell, population of plants, or seed composition aspects, the control comprises a corresponding plant, plant part, plant cell, population of plants, or seed composition without the genetic mutation.

In another aspect, the present disclosure provides a protein composition produced from a plant, plant part, plant cell, population of plants, or seed composition described hereinabove. In some embodiments, the protein composition is present in soybean meal. In some embodiments, the protein composition is present in animal feed.

In a further aspect, the present disclosure provides a nucleic acid molecule comprising a nucleic acid sequence of a mutated anthranilate synthase alpha subunit (*ASA*) gene, wherein the mutated *ASA* gene comprises one or more insertions, substitutions, or deletions as compared to a corresponding *ASA* gene without the mutations, wherein the nucleic acid sequence: (i) comprises at least 80% sequence identity to a nucleic acid sequence set forth as SEQ ID NO: 1 and encodes an active ASA; (ii) comprises the nucleic acid sequence set forth as SEQ ID NO: 1; (iii) encodes an ASA comprising an amino acid sequence having at least 80% sequence identity to an amino acid sequence set forth as SEQ ID NO: 2, wherein the ASA is active; and/or (iv) encodes an ASA comprising an amino acid sequence set forth as SEQ ID NO: 2.

In some embodiments of the above nucleic acid molecule aspect, the mutated *ASA* gene comprises: (i) an in-frame deletion of nucleotides 952 to 957 of *ASA1* having the nucleic acid sequence set forth as SEQ ID NO: 1, or a nucleic acid sequence comprising SEQ ID NO: 3 when the deletion of (i) is introduced; (ii) an in-frame deletion of nucleotides 948 to 959 of *ASA1* having the nucleic acid sequence set forth as SEQ ID NO: 1, or a nucleic acid sequence comprising SEQ ID NO: 4 when the deletion of (ii) is introduced; or (iii) an in-frame deletion of nucleotides 938 to 961 of *ASA1* having the nucleic acid sequence set forth as SEQ ID NO: 1, or a nucleic acid sequence comprising SEQ ID NO: 5 when the deletion of (iii) is introduced.

In some embodiments of the above nucleic acid molecule aspect, the encoded active anthranilate synthase is insensitive to or has reduced sensitivity to Trp feedback inhibition.

In still another aspect, the present disclosure provides a genetic construct comprising, in operable linkage: (i) a promoter that is functional in a plant cell; and (ii) the nucleic acid molecule described hereinabove.

In yet another aspect, the present disclosure provides a cell comprising the nucleic acid molecule or the genetic construct described hereinabove. In some embodiments, the cell is a plant cell.

### BRIEF DESCRIPTION OF THE DRAWINGS

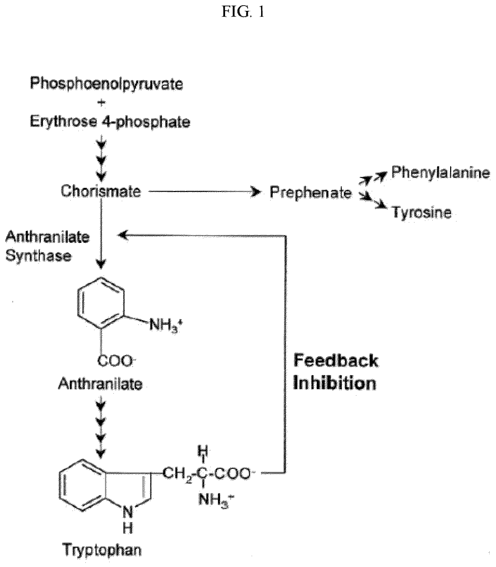

FIG. 1 shows the tryptophan (Trp) synthesis pathway. From Cho et al. (2000) Plant Physiology 123:1069-1076.

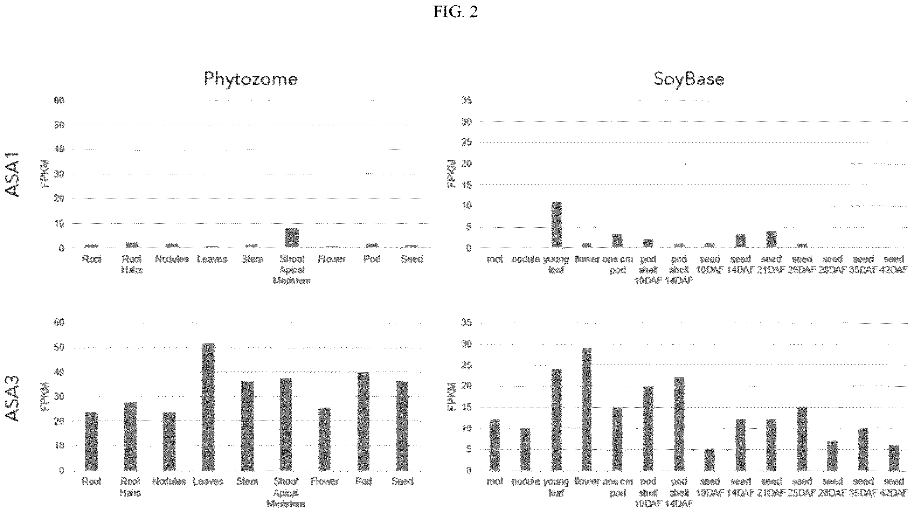

FIG. 2 shows expression of soy ASA genes. Bar graphs of the average expression values of the *Glycine max* ASA isoforms as reported by the Phytozome (left; World Wide Web at phytozome.jgi.doe.gov/pz/portal.html) and SoyBase (right; World Wide Web at soybase.org) RNA-seq datasets. FPKM= fragments per kilobase of transcript per million mapped reads.

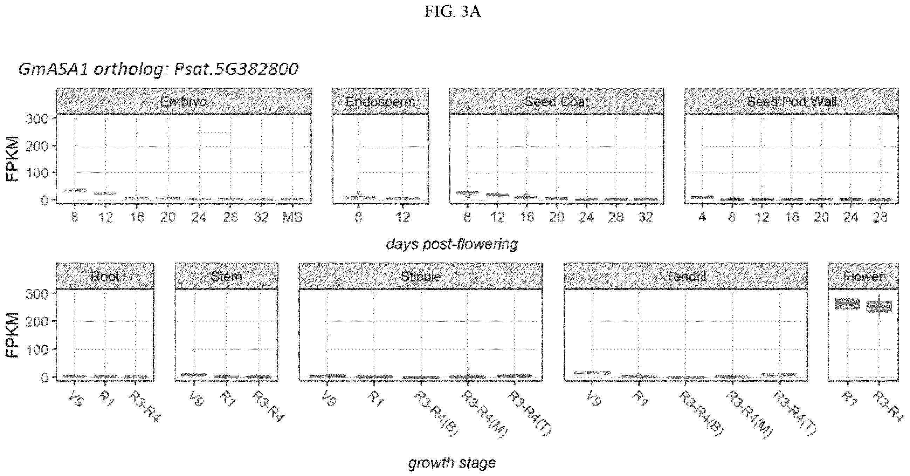

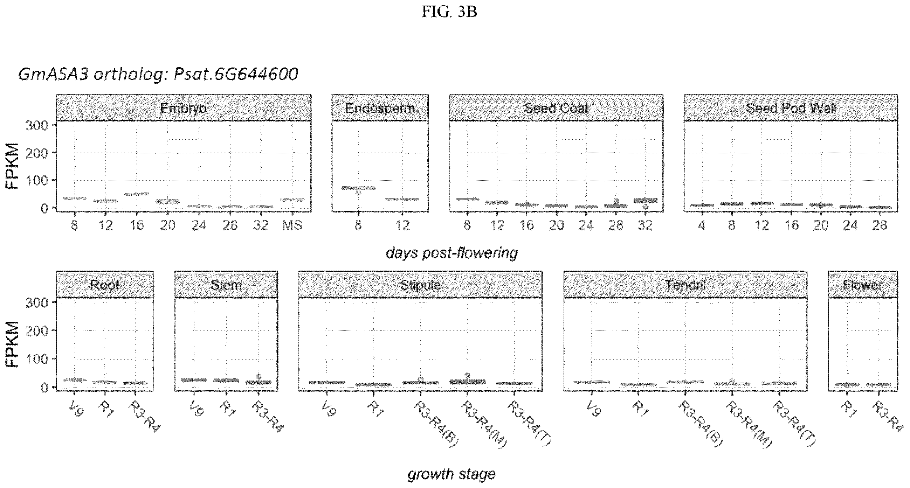

FIGs. 3A, 3B show expression of pea ASA genes: (FIG. 3A) Psat.5G382800 and (FIG. 3B) Psat.6G644600. Boxplots of the replicate-level expression values of the pea ASA isoforms in a proprietary *Pisum sativum* RNA-seq dataset. Expression levels are listed for the analyzed tissues and timepoints. Read counts were normalized to transcript length and total mapped reads, resulting in a final measure of fragments per kilobase of transcript per million mapped reads (FPKM). For reproductive tissues, timepoints are expressed as days post-flowering; for vegetative tissues, timepoints are expressed by growth stage (V9, vegetative stage 9; R1, reproductive stage 1; R3-R4, reproductive stage 3-4). For the stipule and tendril tissues at the R3-R4 timepoint, expression levels were analyzed at the bottom (B), middle (M), and top (T) of these structures.

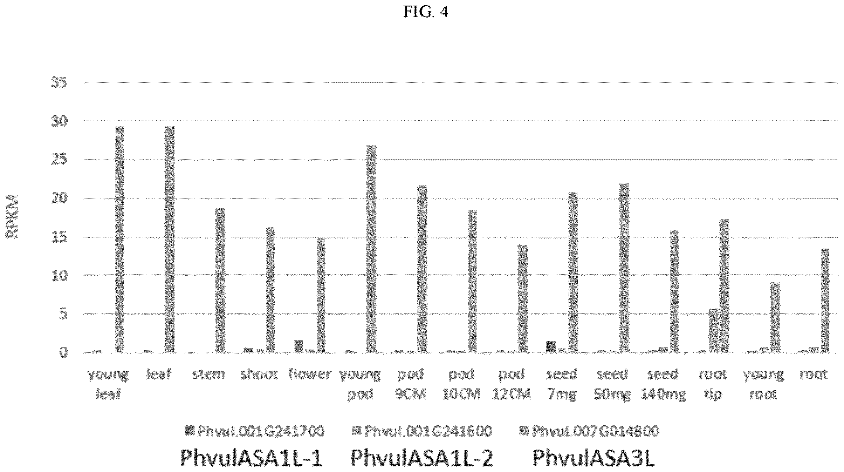

FIG. 4 shows expression of common bean ASA genes. Bar graph of the average expression values of the *Phaseolis vulgaris* ASA isoforms as reported by the PvGEA (World Wide Web at plantgrn.noble.org/PvGEA) RNA-seq datasets; isoforms are named by orthology to soy ASA isoforms.

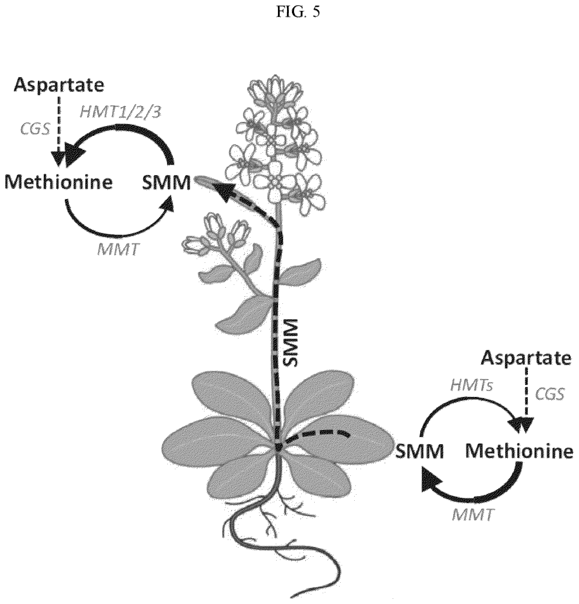

FIG. 5 shows methionine and S-methylmethionine (SMM) metabolism in vegetative tissues and seeds. From Amir et al. (2019) Journal of Experimental Botany 70(16): 4105-4114. Key enzymes are italicized. Abbreviations: CGS, cystathionine γ-synthase; HMTs, homocysteine S-methyltransferases; MMT, methionine S-methyltransferase; SMM, S-methylmethionine.

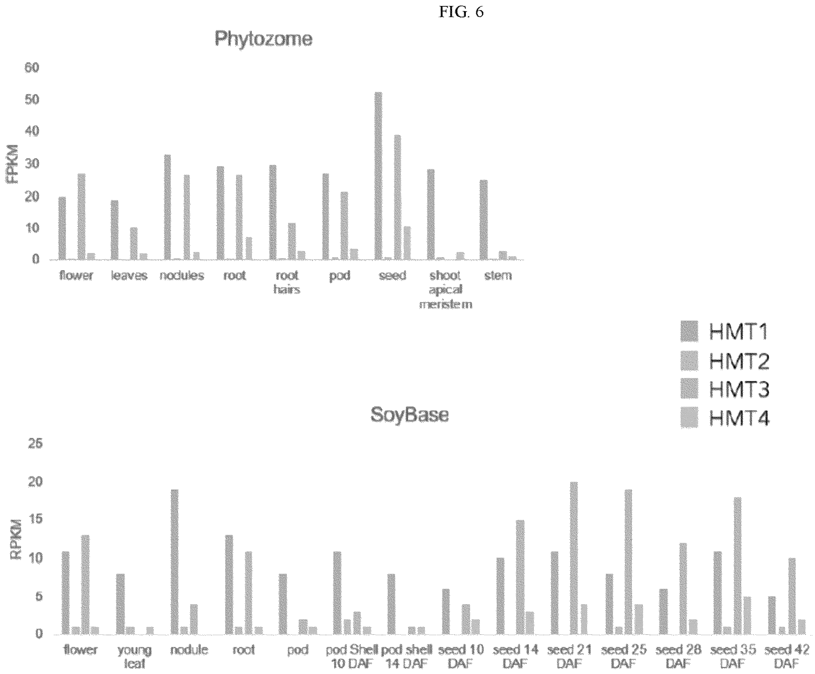

FIG. 6 shows expression of soy *HMT* genes. Bar graphs of the average expression values of the *Glycine max HMT* isoforms as reported by the Phytozome (top) and SoyBase (bottom) RNA-seq datasets.

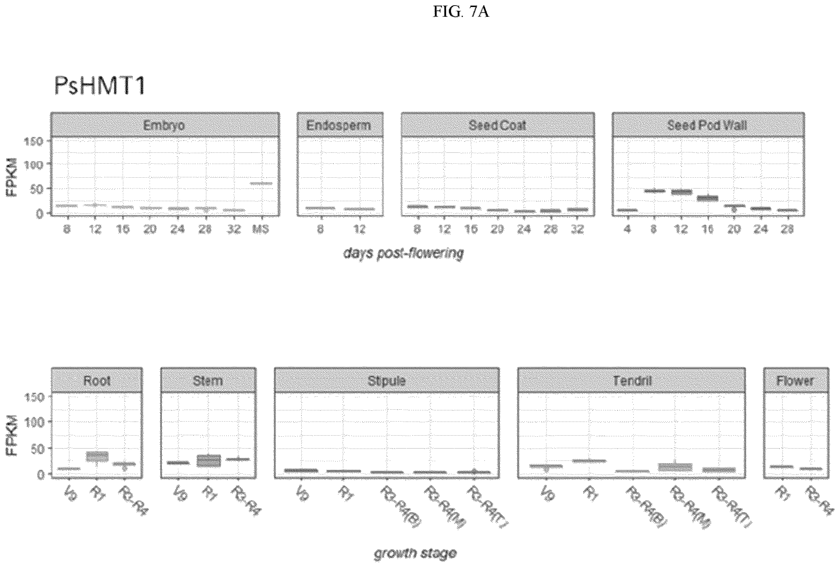

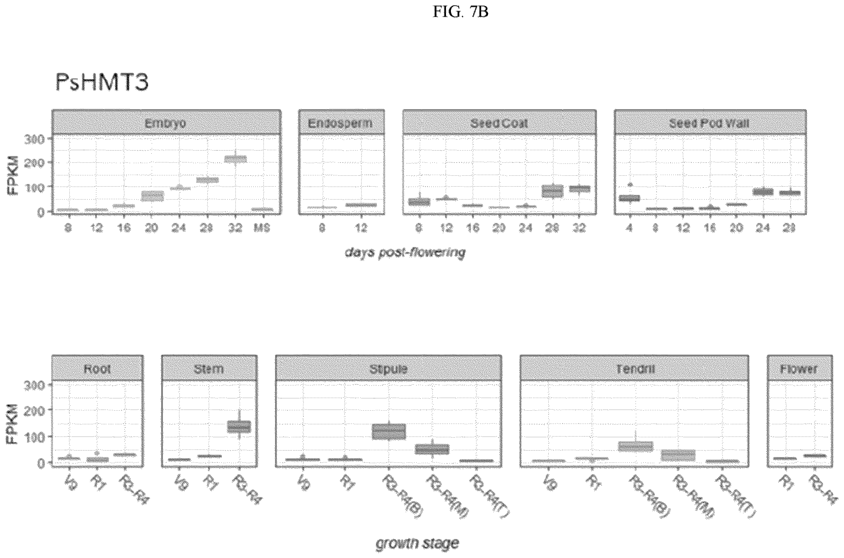

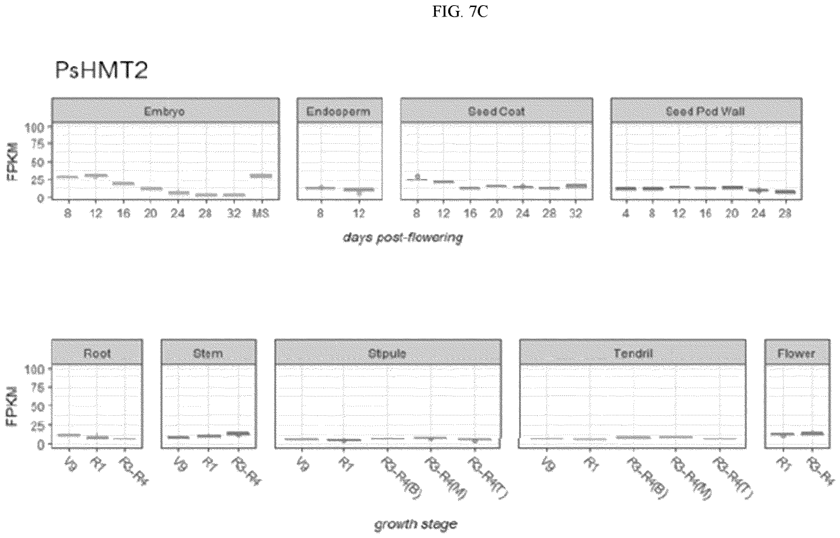

FIGs. 7A-7C show expression of pea *HMT* genes. (FIG. 7A) Ps*HMT1*; (FIG. 7B) *PsHMT3;* and (FIG. 7C) Ps*HMT2.* Boxplots of the replicate-level expression values of the pea *HMT* isoforms in a proprietary *Pisum sativum* RNA-seq dataset. Expression levels are listed for the analyzed tissues and timepoints. Read counts were normalized to transcript length and total mapped reads, resulting in a final measure of fragments per kilobase of transcript per million mapped reads (FPKM). For reproductive tissues, timepoints are expressed as days post-flowering; for vegetative tissues, timepoints are expressed by growth stage (V9, vegetative stage 9; R1, reproductive stage 1; R3-R4, reproductive stage 3-4). For the stipule and tendril tissues at the R3-R4 timepoint, expression levels were analyzed at the bottom (B), middle (M), and top (T) of these structures.

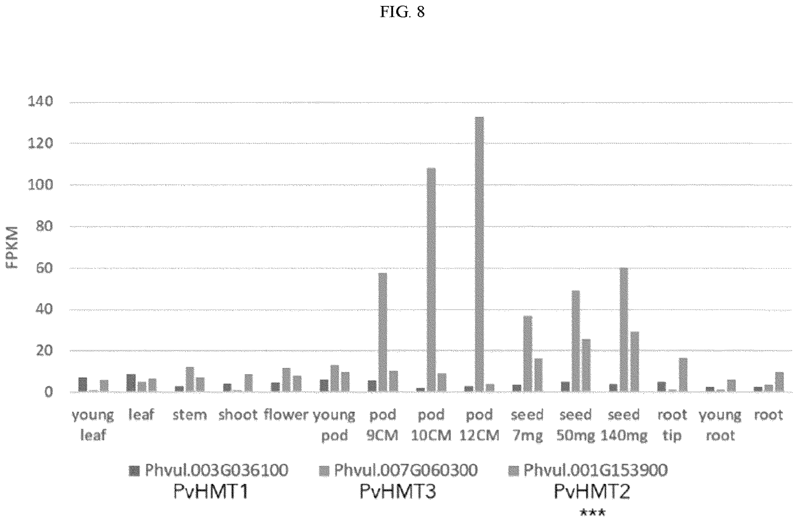

FIG. 8 shows expression of common bean *HMT* genes. Bar graph of the average expression values of the *Phaseolis vulgaris HMT* isoforms as reported by the PvGEA RNA-seq datasets.

### DETAILED DESCRIPTION OF THE INVENTION

The present disclosure now will be described more fully hereinafter. The disclosure may be embodied in many different forms and should not be construed as limited to the aspects set forth herein; rather, these aspects are provided so that this disclosure will satisfy applicable legal requirements.

### I. Definitions

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

As used herein, "a," "an," or "the" can mean one or more than one. For example, "a" cell can mean a single cell or a multiplicity of cells. Further, the term "a plant" may include a plurality of plants.

As used herein, unless specifically indicated otherwise, the word "or" is used in the inclusive sense of "and/or" and not the exclusive sense of "either/or."

The term "about" or "approximately" usually means within 5%, or more preferably within 1%, of a given value or range.

The terms "comprises", "comprising", "includes", "including", "having" and their conjugates mean "including but not limited to".

Various embodiments of this disclosure may be presented in a range format. It should be noted that whenever a value or range of values of a parameter are recited, it is intended that values and ranges intermediate to the recited values are also part of this disclosure. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1-10 should be considered to have specifically disclosed subranges such as from 1 to 3, from 1 to 4, from 1 to 5, from 1 to 6, from 1 to 7, from 1 to 8, from 1 to 9, from 2 to 4, from 2 to 6, from 2 to 8, from 2 to 10, from 3 to 6, etc., as well as individual numbers within that range, for example, 1, 2, 3, 4, 5, 6, 7, 8, 9 and 10. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between. The recitation of a numerical range for a variable is intended to convey that the present disclosure may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

A plant refers to a whole plant, any part thereof, or a cell or tissue culture derived from a plant, comprising any of: whole plants, plant components or organs (e.g., leaves, stems, roots, embryos, pollen, ovules, seeds, leaves, flowers, branches, fruit, pulp, juice, kernels, ears, cobs, husks, stalks, root tips, anthers, etc.), plant tissues, seeds, plant cells, protoplasts and/or progeny of the same. A plant part includes a plant cell. A plant cell is a biological cell of a plant, taken from a plant or derived through culture of a cell taken from a plant. Grain is intended to mean the mature seed produced by commercial growers for purposes other than growing or reproducing the species. Progeny, variants, and mutants of the regenerated plants are also included within the scope of the invention.

As used herein, a "subject plant or plant cell" is one in which genetic alteration, such as a mutation, has been effected as to a gene of interest, or is a plant or plant cell which is descended from a plant or cell so altered and which comprises the alteration. As used herein, the term "mutated" or "genetically modified" or "transgenic" or "transformed" or "edited" plants, plant cells, plant tissues, plant parts, or seeds refers to plants, plant cells, plant tissues, plant parts, or seeds that have been mutated by the methods of the present disclosure to include one or more mutations (e.g., insertions, substitutions, and/or deletions) in the genomic sequence.

As used herein, a "control plant" or "control plant part" or "control cell" or "control seed" refers to a plant, plant part, plant cell, or seed that has not been subject to the methods and compositions described herein. A "control" or "control plant" or "control plant part" or "control cell" or "control seed" provides a reference point for measuring changes in phenotype of the subject plant or plant cell. A control plant or plant cell may comprise, for example: (a) a wild-type plant or plant cell, i.e., of the same genotype as the starting material for the genetic alteration which resulted in the subject plant or plant cell; (b) a plant or plant cell of the same genotype as the starting material but which has been transformed with a null construct (i.e. with a construct which has no known effect on the trait of interest, such as a construct comprising a marker gene); (c) a plant or plant cell which is a non-transformed segregant among progeny of a subject plant or plant cell; (d) a plant or plant cell genetically identical to the subject plant or plant cell but which is not exposed to conditions or stimuli (e.g., sucrose) that would induce expression of a gene of interest; or (e) the subject plant or plant cell itself, under conditions in which a gene of interest is not expressed. In certain instances, a control plant of the present disclosure is grown under the same environmental conditions (e.g., same or similar temperature, humidity, air quality, soil quality, water quality, and/or pH conditions) as a subject plant described herein. A control plant or plant cell is grown to produce control seed. Similarly, a control composition can refer to a composition that is isolated or derived from a control plant, plant part, or seed. In some embodiments, a control plant, plant part, plant cell, or seed does not have an endogenous *AS* (anthranilate synthase) gene and/or an endogenous *HMT* (homocysteine *S*-methyltransferase) gene comprising a genetic mutation as described herein.

Plant cells possess nuclear, plastid, and mitochondrial genomes. The compositions and methods of the present invention may be used to modify the sequence of the nuclear, plastid, and/or mitochondrial genome, or may be used to modulate the expression of a gene or genes encoded by the nuclear, plastid, and/or mitochondrial genome. Accordingly, by "chromosome" or "chromosomal" is intended the nuclear, plastid, or mitochondrial genomic DNA. "Genome" as it applies to plant cells encompasses not only chromosomal DNA found within the nucleus, but organelle DNA found within subcellular components (e.g., mitochondria or plastids) of the cell.

As used herein, the term "gene" includes genomic sequences, regulatory sequences (e.g., promoters, enhancers, 5' UTR, 3' UTR, or any sequence that controls expression of a gene), cDNA sequences, and smaller engineered gene segments that express, or may be adapted to express proteins, polypeptides, domains, peptides, fusion proteins, and mutants. A gene includes a "coding sequence", which refers to a functional nucleic acid unit encoding a protein, polypeptide, or peptide. In some embodiments, a gene includes regulatory regions that control expression of a gene, including a promoter, an enhancer, a transcription factor binding site, and untranslated regions 5' and/or 3' of a coding region.

As used herein, the term "nucleic acid", used interchangeably with "nucleotide" or "polynucleotide", refers to a molecule including a nucleoside and a phosphate that serves as a component of DNA or RNA. For instance, nucleic acids include adenine, guanine, cytosine, uracil, and thymine.

As used herein, a "mutation" is any change in a nucleic acid sequence. Non-limiting examples comprise insertions, deletions, duplications, substitutions, inversions, and translocations of any nucleic acid sequence, regardless of how the mutation is brought about and regardless of how or whether the mutation alters the functions or interactions of the nucleic acid. For example and without limitation, a mutation may produce altered enzymatic activity of a ribozyme, altered base pairing between nucleic acids (e.g. RNA interference interactions, DNA-RNA binding, etc.), altered mRNA folding stability, and/or how a nucleic acid interacts with polypeptides (e.g. DNA-transcription factor interactions, RNA-ribosome interactions, gRNA-endonuclease reactions, etc.). A mutation might result in the production of proteins with altered amino acid sequences (e.g. missense mutations, nonsense mutations, frameshift mutations, etc.) and/or the production of proteins with the same amino acid sequence (e.g. silent mutations). Certain synonymous mutations may create no observed change in the plant while others that encode for an identical protein sequence nevertheless result in an altered plant phenotype (e.g. due to codon usage bias, altered secondary protein structures, etc.). Mutations may occur within coding regions (e.g., open reading frames) or outside of coding regions (e.g., within promoters, terminators, untranslated elements, or enhancers), and may affect, for example and without limitation, gene expression levels, gene expression profiles, protein sequences, and/or sequences encoding RNA elements such as tRNAs, ribozymes, ribosome components, and microRNAs.

Accordingly, "plant with a genetic mutation", "plant part with a genetic mutation", "plant cell with a genetic mutation", "plant seed with a genetic mutation", or "plant genome with a genetic mutation" refers to a plant, plant part, plant cell, plant seed, or plant genome that contains at least one genetic mutation (e.g., an insertion, a substitution, a deletion, or a combination thereof) described in the present disclosure, such as at least one genetic mutation in the nucleic acid sequence of an *AS* gene (e.g., a *Glycine max ASA1, ASA2, ASA3,* and/or *ASA4* gene encoding an anthranilate synthase alpha subunit) and/or at least one genetic mutation in the nucleic acid sequence of an *HMT* gene (e.g., a *Glycine max HMT1, HMT2, HMT3,* and/or *HMT4* gene). In some embodiments, a plant, plant part, plant cell, or plant seed having a genetic mutation may refer to a plant, plant part, plant cell, or plant seed in which, or in an ancestor of which, at least one *AS* gene (e.g., an *ASA* gene) and/or at least one *HMT* gene has been deliberately mutated such that the plant, plant part, plant cell, or plant seed expresses a mutated anthranilate synthase (AS) protein (e.g., ASA protein) and/or a mutated homocysteine *S*-methyltransferase (HMT) protein. The mutated AS (e.g., ASA) and/or HMT protein can have altered function, e.g., reduced function, loss-of-function, or gain-of-function, as compared to a wild-type, or control, AS (e.g., ASA) and/or HMT protein comprising no mutation. In some embodiments, a mutated *HMT* gene is not expressed at the mRNA and/or protein level. "Genetic mutation" and "mutation" are used interchangeably herein.

"Genome editing" or "gene editing" as used herein refers to a type of genetic engineering by which one or more mutations (e.g., insertions, substitutions, deletions, insertions and deletions) are introduced at a specific location of the genome.

As used herein, the term "recombinant DNA construct," "recombinant construct," "expression cassette," "expression construct," "chimeric construct," "construct," and "recombinant DNA fragment" are used interchangeably herein and are single or doublestranded polynucleotides. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector.

An expression construct can permit transcription of a particular nucleic acid sequence in a host cell (e.g., a bacterial cell or a plant cell). An expression cassette may be part of a plasmid, viral genome, or nucleic acid fragment. Typically, an expression cassette includes a polynucleotide to be transcribed, operably linked to a promoter. "Operably linked" is intended to mean a functional linkage between two or more elements. For example, an operable linkage between a promoter of the present disclosure and a heterologous nucleic acid molecule is a functional link that allows for expression of the heterologous nucleic acid molecule. Operably linked elements may be contiguous or non-contiguous. When used to refer to the joining of two protein coding regions, by operably linked is intended that the coding regions are in the same reading frame. The cassette may additionally contain at least one additional gene to be co-transformed into the plant. Alternatively, the additional gene(s) can be provided on multiple expression cassettes or DNA constructs. The expression cassette may additionally contain selectable marker genes. Other elements that may be present in an expression cassette include those that enhance transcription (e.g., enhancers) and terminate transcription (e.g., terminators), as well as those that confer certain binding affinity or antigenicity to the recombinant protein produced from the expression cassette.

As used herein, "function" of a gene, a peptide, a protein, or a molecule refers to activity of a gene, a peptide, a protein, or a molecule.

"Introduced" in the context of inserting a nucleic acid molecule (e.g., a recombinant DNA construct) into a cell, means "transfection" or "transformation" or "transduction" and includes reference to the incorporation of a nucleic acid fragment into a plant cell where the nucleic acid fragment may be incorporated into the genome of the cell (e.g., nuclear chromosome, plasmid, plastid chromosome or mitochondrial chromosome), converted into an autonomous replicon, or transiently expressed (e.g., transfected mRNA).

As used herein with respect to a parameter, the terms "decreased" or "decreasing" or "decrease" or "reduced" or "reducing" or "reduce" or "lower" or "loss" refers to a detectable (e.g., at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or 100%) negative change in the parameter from a comparison control, e.g., an established normal or reference level of the parameter, or an established standard control. Accordingly, the terms "decreased", "reduced", and the like encompass both a partial reduction and a complete reduction compared to a control.

As used herein with respect to a parameter, the term "increased" or "increasing" or "increase" refers to a detectable (e.g., at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, 120%, 150%, 200%, 300%, 400%, 500%, or more) positive change in the parameter from a comparison control, e.g., an established normal or reference level of the parameter, or an established standard control. Accordingly, the terms "increased", "increase", and the like encompass both a partial reduction and a significant increase compared to a control.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

As used herein, the term "polypeptide" refers to a linear organic polymer containing a large number of amino-acid residues bonded together by peptide bonds in a chain, forming part of (or the whole of) a protein molecule. The amino acid sequence of the polypeptide refers to the linear consecutive arrangement of the amino acids comprising the polypeptide, or a portion thereof.

As used herein the term "polynucleotide" refers to a single or double stranded nucleic acid sequence which is isolated and provided in the form of an RNA sequence (e.g., an mRNA sequence), a complementary polynucleic acid sequence (cDNA), a genomic polynucleic acid sequence and/or a composite polynucleic acid sequences (e.g., a combination of the above).

The term "isolated" refers to at least partially separated from the natural environment e.g., from a plant cell.

As used herein, the term "expression" or "expressing" refers to the transcription and/or translation of a particular nucleic acid sequence driven by a promoter.

As used herein, the terms "exogenous" or "heterologous" in reference to a nucleic acid sequence or amino acid sequence are intended to mean a sequence that is purely synthetic, that originates from a foreign species, or, if from the same species, is substantially modified from its native form in composition and/or genomic locus by deliberate human intervention. Thus, a heterologous nucleic acid sequence may not be naturally expressed within the plant (e.g., a nucleic acid sequence from a different species) or may have altered expression when compared to the corresponding wild type plant. An exogenous polynucleotide may be introduced into the plant in a stable or transient manner, so as to produce a ribonucleic acid (RNA) molecule and/or a polypeptide molecule. It should be noted that the exogenous polynucleotide may comprise a nucleic acid sequence which is identical or partially homologous to an endogenous nucleic acid sequence of the plant.

As used herein, by "endogenous" in reference to a gene or nucleic acid sequence or protein is intended a gene or nucleic acid sequence or protein that is naturally comprised within or expressed by a cell. Endogenous genes can include genes that naturally occur in the cell of a plant, but that have been modified in the genome of the cell without insertion or replacement of a heterologous gene that is from another plant species or another location within the genome of the modified cell.

As used herein, "fertilization" and/or "crossing" broadly includes bringing the genomes of gametes together to form zygotes but also broadly may include pollination, syngamy, fecundation and other processes related to sexual reproduction. Typically, a cross and/or fertilization occurs after pollen is transferred from one flower to another, but those of ordinary skill in the art will understand that plant breeders can leverage their understanding of fertilization and the overlapping steps of crossing, pollination, syngamy, and fecundation to circumvent certain steps of the plant life cycle and yet achieve equivalent outcomes, for example, a plant or cell of a soybean cultivar described herein. In certain embodiments, a user of this innovation can generate a plant of the claimed invention by removing a genome from its host gamete cell before syngamy and inserting it into the nucleus of another cell. While this variation avoids the unnecessary steps of pollination and syngamy and produces a cell that may not satisfy certain definitions of a zygote, the process falls within the definition of fertilization and/or crossing as used herein when performed in conjunction with these teachings. In certain embodiments, the gametes are not different cell types (i.e. egg vs. sperm), but rather the same type and techniques are used to effect the combination of their genomes into a regenerable cell. Other embodiments of fertilization and/or crossing include circumstances where the gametes originate from the same parent plant, i.e. a "self" or "self-fertilization". While selfing a plant does not require the transfer of pollen from one plant to another, those of skill in the art will recognize that it nevertheless serves as an example of a cross, just as it serves as a type of fertilization. Thus, methods and compositions taught herein are not limited to certain techniques or steps that must be performed to create a plant or an offspring plant of the claimed invention, but rather include broadly any method that is substantially the same and/or results in compositions of the claimed invention.

"Homolog" or "homologous sequence" may refer to both orthologous and paralogous sequences. Paralogous sequence relates to gene-duplications within the genome of a species. Orthologous sequence relates to homologous genes in different organisms due to ancestral relationship. Thus, orthologs are evolutionary counterparts derived from a single ancestral gene in the last common ancestor of given two species and therefore have great likelihood of having the same function. One option to identify homologs (e.g., orthologs) in monocot plant species is by performing a reciprocal BLAST search. This may be done by a first blast involving blasting the sequence-of-interest against any sequence database, such as the publicly available NCBI database which may be found at: ncbi.nlm.nih.gov. If orthologs in rice were sought, the sequence-of-interest would be blasted against, for example, the 28,469 full-length cDNA clones from *Oryza sativa* Nipponbare available at NCBI. The blast results may be filtered. The full-length sequences of either the filtered results or the non-filtered results are then blasted back (second blast) against the sequences of the organism from which the sequence-of-interest is derived. The results of the first and second blasts are then compared. An ortholog is identified when the sequence resulting in the highest score (best hit) in the first blast identifies in the second blast the query sequence (the original sequence-of-interest) as the best hit. Using the same rationale, a paralog (homolog to a gene in the same organism) is found. In case of large sequence families, the ClustalW program may be used [ebi.ac.uk/Tools/clustalw2/index.html], followed by a neighbor-joining tree (wikipedia.org/wiki/Neighbor-joining) which helps visualizing the clustering.

In some embodiments, the term "homolog" as used herein, refers to functional homologs of genes. A functional homolog is a gene encoding a polypeptide that has sequence similarity to a polypeptide encoded by a reference gene, and the polypeptide encoded by the homolog carries out one or more of the biochemical or physiological function(s) of the polypeptide encoded by the reference gene. In general, it is preferred that functional homologs and/or polypeptides encoded by functional homologs share at least some degree of sequence identity with the reference gene or polypeptide encoded by the reference gene.

Homology (e.g., percent homology, sequence identity + sequence similarity) can be determined using any homology comparison software computing a pairwise sequence alignment.

As used herein, "sequence identity," "identity," "percent identity," "percentage similarity," "sequence similarity" and the like refer to a measure of the degree of similarity of two sequences based upon an alignment of the sequences that maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov), and are described in, for example, Altschul et al. (1990), J. Mol. Biol. 215:403-410; Gish and States (1993), Nature Genet. 3:266-272; Madden et al. (1996), Meth. Enzymol.266:131-141; Altschul et al. (1997), Nucleic Acids Res. 25:3389-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3. When percentage of sequence identity is used in reference to proteins it is recognized that residue positions which are not identical often differ by conservative amino acid substitutions, where amino acid residues are substituted for other amino acid residues with similar chemical properties (e.g. charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are considered to have "sequence similarity" or "similarity". Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., according to the algorithm of Henikoff S and Henikoff J G. (Proc Natl Acad Sci 89:10915-9 (1992)). Identity (e.g., percent homology) can be determined using any homology comparison software, including for example, the BlastN software of the National Center of Biotechnology Information (NCBI) such as by using default parameters.

According to some embodiments, the identity is a global identity, i.e., an identity over the entire amino acid or nucleic acid sequences of the invention and not over portions thereof.

According to some embodiments, the term "homology" or "homologous" refers to identity of two or more nucleic acid sequences; or identity of two or more amino acid sequences; or the identity of an amino acid sequence to one or more nucleic acid sequence. According to some embodiments, the homology is a global homology, e.g., a homology over the entire amino acid or nucleic acid sequences of the invention and not over portions thereof. The degree of homology or identity between two or more sequences can be determined using various known sequence comparison tools which are described in WO2014/102774.

As used herein, the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "population" refers to a set comprising any number, including one, of individuals, objects, or data from which samples are taken for evaluation, e.g., estimating quantitative trait locus (QTL) effects and/or disease tolerance. Most commonly, the terms relate to a breeding population of plants from which members are selected and crossed to produce progeny in a breeding program. A population of plants can include the progeny of a single breeding cross or a plurality of breeding crosses and can be either actual plants or plant derived material, or *in silico* representations of plants. The members of a population need not be identical to the population members selected for use in subsequent cycles of analyses, nor does it need to be identical to those population members ultimately selected to obtain a final progeny of plants. Often, a plant population is derived from a single bi-parental cross but can also derive from two or more crosses between the same or different parents. Although a population of plants can comprise any number of individuals, those of skill in the art will recognize that plant breeders commonly use population sizes ranging from one or two hundred individuals to several thousand, and that the highest performing 5-20% of a population is what is commonly selected to be used in subsequent crosses in order to improve the performance of subsequent generations of the population in a plant breeding program.

As used herein, the term "crop performance" is used synonymously with "plant performance" and refers to of how well a plant grows under a set of environmental conditions and cultivation practices. Crop performance can be measured by any metric a user associates with a crop's productivity (e.g., yield), appearance and/or robustness (e.g., color, morphology, height, biomass, maturation rate, etc.), product quality (e.g., fiber lint percent, fiber quality, seed protein content, seed carbohydrate content, etc.), cost of goods sold (e.g., the cost of creating a seed, plant, or plant product in a commercial, research, or industrial setting) and/or a plant's tolerance to disease (e.g., a response associated with deliberate or spontaneous infection by a pathogen) and/or environmental stress (e.g., drought, flooding, low nitrogen or other soil nutrients, wind, hail, temperature, day length, etc.). Crop performance can also be measured by determining a crop's commercial value and/or by determining the likelihood that a particular inbred, hybrid, or variety will become a commercial product, and/or by determining the likelihood that the offspring of an inbred, hybrid, or variety will become a commercial product. Crop performance can be a quantity (e.g., the volume or weight of seed or other plant product measured in liters or grams) or some other metric assigned to some aspect of a plant that can be represented on a scale (e.g., assigning a 1-10 value to a plant based on its disease tolerance).

A plant, or its environment, can be contacted with a wide variety of "agriculture treatment agents." As used herein, an "agriculture treatment agent", or "treatment agent", or "agent" can refer to any exogenously provided compound that can be brought into contact with a plant tissue (e.g. a seed) or its environment that affects a plant's growth, development and/or performance, including agents that affect other organisms in the plant's environment when those effects subsequently alter a plant's performance, growth, and/or development (e.g. an insecticide that kills plant pathogens in the plant's environment, thereby improving the ability of the plant to tolerate the pathogen's presence). Agriculture treatment agents also include a broad range of chemicals and/or biological substances that are applied to seeds, in which case they are commonly referred to as seed treatments and/or seed dressings. Seed treatments are commonly applied as either a dry formulation or a wet slurry or liquid formulation prior to planting and, as used herein, generally include any agriculture treatment agent including growth regulators, micronutrients, nitrogen-fixing microbes, and/or inoculants.

Agriculture treatment agents include pesticides (e.g. fungicides, insecticides, bactericides, etc.) hormones (abscisic acids, auxins, cytokinins, gibberellins, etc.) herbicides (e.g. glyphosate, atrazine, 2,4-D, dicamba, etc.), nutrients (e.g. a plant fertilizer), and/or a broad range of biological agents, for example a seed treatment inoculant comprising a microbe that improves crop performance, e.g. by promoting germination and/or root development.

In certain embodiments, the agriculture treatment agent acts extracellularly within the plant tissue, such as interacting with receptors on the outer cell surface. In some embodiments, the agriculture treatment agent enters cells within the plant tissue. In certain embodiments, the agriculture treatment agent remains on the surface of the plant and/or the soil near the plant.

In certain embodiments, the agriculture treatment agent is contained within a liquid. Such liquids include, but are not limited to, solutions, suspensions, emulsions, and colloidal dispersions. In some embodiments, liquids described herein will be of an aqueous nature. However, in various embodiments, such aqueous liquids that comprise water can also comprise water insoluble components, can comprise an insoluble component that is made soluble in water by addition of a surfactant, or can comprise any combination of soluble components and surfactants.

In certain embodiments, the application of the agriculture treatment agent is controlled by encapsulating the agent within a coating, or capsule (e.g. microencapsulation). In certain embodiments, the agriculture treatment agent comprises a nanoparticle and/or the application of the agriculture treatment agent comprises the use of nanotechnology. In some embodiments, the plants described herein can grow in the presence of one or more agricultural treatment agents.

As used herein, "the corresponding gene or homolog thereof" refers to a same gene (e.g., *ASA1, ASA2, ASA3, ASA4, HMT1, HMT2, HMT3, HMT4*) except that the corresponding gene in a control plant or plant part does not have the genetic mutation. For example, an *ASA1* gene or homolog thereof of the present disclosure can include a genetic mutation as described herein and its corresponding gene or homolog thereof in a control plant or plant part is an *ASA1* gene or homolog thereof without the genetic mutation. As another example, an *HMT3* gene or homolog thereof of the present disclosure can include a genetic mutation as described herein and its corresponding gene or homolog thereof in a control plant or plant part is an *HMT3* gene or homolog thereof without the genetic mutation.

The patent and scientific literature referred to herein establishes knowledge that is available to those of skill in the art. The issued US patents, allowed applications, published foreign applications, and references, including GenBank database sequences, which are cited herein are hereby incorporated by reference to the same extent as if each was specifically and individually indicated to be incorporated by reference.

All publications, patent applications, patents, and other references mentioned herein are incorporated by reference herein in their entirety.

### II. Overview of the Invention

The present disclosure provides plants and plant parts having increased levels of tryptophan (Trp) and/or methionine (Met). This can be achieved by introducing specific changes to key amino acid synthesis and transport enzymes via gene editing. For example, Trp can be increased by making an in-frame deletion in a regulatory region of anthranilate synthase 1 (AS), a key enzyme in Trp biosynthesis that is regulated via feedback inhibition. Likewise, Met can be increased by creating an insertion and deletion (indel) in at least one *HMT* gene. In some embodiments, compositions and methods of the disclosure allow generation of plants and plant parts having Trp and/or Met levels in seed that are higher than levels existing in current plant varieties. Other traits (e.g., herbicide resistance, pesticide resistance, enhanced seed oil content) can be introduced into these high amino acid plant lines to create plant lines with stacked traits including high Trp and/or Met seed content.

Soybean is used for animal feed but lacks sufficient levels of several amino acids, requiring supplementation. Using soybeans with increased levels of these amino acids will reduce or eliminate this need for supplementation, lowering costs for producing animal feed and other products where augmented content of amino acids in soybean seed would be valuable. These high amino acid soybean lines are useful for the swine (Trp) and poultry (Met) industries.

It is currently unclear what factors are critical to produce plants having increased Trp and/or Met content in seed, including what genes are important in regulation of Trp and Met biosynthesis in plants, types of genetic mutations that would allow an increase in Trp and/or Met content in seed, locations of effective genetic mutations in these genes, and isoforms of these genes that would be important to target.

Disclosed herein are plants or plant parts comprising a genetic mutation in at least one endogenous *AS* gene or homolog thereof encoding an anthranilate synthase and/or *HMT* gene or homolog thereof encoding a homocysteine S-methyltransferase, wherein the mutated plant or plant part comprises increased tryptophan (Trp) and/or methionine (Met) amino acid content as compared to a control, as well as methods for making the plants or plant parts. Such plants or plant parts can have one or more insertions, substitutions, or deletions in at least one endogenous *AS* gene or homolog thereof and/or at least one endogenous *HMT* gene or homolog thereof.

In some embodiments, the *AS* gene or homolog thereof comprises an *ASA* gene or homolog thereof encoding an anthranilate synthase alpha subunit. The plants or plant parts can have a genetic mutation in an *ASA* gene or homolog thereof encoding an ASA protein that is insensitive to or has reduced sensitivity to Trp feedback inhibition as compared to an ASA protein encoded by the corresponding *ASA* gene or homolog thereof without the genetic mutation. In some embodiments, the plants or plant parts have a genetic mutation in an *ASA* gene or homolog thereof encoding an ASA protein that has increased activity as compared to an ASA protein encoded by the corresponding ASA gene or homolog thereof without the genetic mutation. The plants or plant parts can have a genetic mutation in an *ASA* gene or homolog thereof has increased seed Trp content as compared to a control plant or plant part without the genetic mutation in the corresponding *ASA* gene or homolog thereof.

The plants or plant parts can have a genetic mutation in an *HMT* gene or homolog thereof such that the *HMT* gene or homolog thereof has a reduced expression level as compared to the corresponding *HMT* gene or homolog thereof without the genetic mutation. In some embodiments, the plants or plant parts have a genetic mutation in an *HMT* gene encoding an HMT protein that has a reduced level or activity of the HMT protein encoded by the *HMT* gene or homolog thereof as compared to an HMT protein encoded by the corresponding *HMT* gene or homolog thereof without the genetic mutation. The plants or plant parts having a genetic mutation in an *HMT* gene or homolog thereof can have increased seed Met content as compared to a control plant or plant part without the genetic mutation in the corresponding *HMT* gene or homolog thereof.

The present disclosure also provides compositions and methods for producing plants or plant parts with increased Trp and/or Met amino acid content by introducing a genetic mutation that alters activity and/or expression levels of AS and/or HMT proteins. The methods disclosed herein can include introducing one or more insertions, substitutions, and/or deletions in at least one endogenous *AS* gene or homolog thereof and/or at least one endogenous *HMT* gene or homolog thereof in the genome of a plant, plant part, or plant cell, such that the AS protein encoded by the *AS* gene or homolog thereof is insensitive to or has reduced sensitivity to Trp feedback inhibition, the AS protein encoded by the *AS* gene or homolog thereof has increased activity, the *HMT* gene or homolog thereof has a reduced expression level, the HMT protein encoded by the *HMT* gene or homolog thereof has reduced expression and/or activity, and/or Trp and/or Met content is increased in the plant, plant part, or plant cell as compared to a plant, plant part, or plant cell without the genetic mutation. The methods of the present disclosure can include introducing editing reagents (e.g., nuclease, guide RNA) into the plants or plant parts to introduce a mutation in at least one endogenous *AS* gene or homolog thereof and/or at least one endogenous *HMT* gene or homolog thereof.

The plants, plant parts, or plant products, such as animal feed comprising a composition of the present disclosure, including those produced using the methods disclosed herein, can have increased Trp and/or Met seed content.

The plants or plant parts can include seed. In some embodiments, the plant, plant part, or plant cell is from a legume. In certain embodiments, the legume is soybean (*Glycine max*) or a pea variety (e.g., *Pisum sativum*)*.*

The present disclosure also provides nucleic acid molecules comprising a mutated *AS* gene or homolog thereof, a genetic construct comprising such nucleic acid molecule operably linked to a promoter, and cells comprising the nucleic acid molecule or the genetic construct of the present disclosure.

### III. Plants with Increased Amino Acid content

Plants and plant parts having a genetic mutation in at least one endogenous *AS* gene or homolog thereof encoding an anthranilate synthase and/or in at least one endogenous *HMT* gene or homolog thereof encoding a homocysteine S-methyltransferase, are provided herein wherein the mutated plants or plant parts comprise increased tryptophan (Trp) and/or methionine (Met) amino acid content as compared to a control. In some embodiments, the disclosure provides seed comprising increased Trp and/or Met content. In some embodiments, the disclosure provides a plant or plant part comprising increased Trp, Met, and/or *S*-methylmethionine (SMM) content in vegetative tissues.

The *AS* gene or homolog thereof can include an *ASA* gene or homolog thereof encoding an anthranilate synthase alpha subunit. In some embodiments, the genetic mutation in an *ASA* gene or homolog thereof alters activity of the ASA protein encoded by the *ASA* gene as compared to an ASA protein encoded by the corresponding *ASA* gene without the genetic mutation in a control plant or plant part. In some embodiments, the ASA protein encoded by the *ASA* gene having the genetic mutation is insensitive to or has reduced sensitivity to Trp feedback inhibition and/or has increased activity.

In some embodiments, the genetic mutation in an *HMT* gene or homolog thereof leads to a decrease in expression level or no expression of the *HMT* gene or homolog thereof, a decrease in expression level or no expression of the HMT protein encoded by the mutated *HMT* gene or homolog thereof, and/or a decrease in activity or lack of activity of the HMT protein encoded by the mutated *HMT* gene or homolog thereof. In some embodiments, the plants and plant parts are from a legume. In certain embodiments, the legume is soybean *(Glycine max*) or pea *(Pisum sativum).*

### A. Anthranilate synthase (AS) and homocysteine-S-methyltransferase (HMT)

### Anthranilate synthase

Anthranilate synthase (AS; EC 4.1.3.27) performs the first committed step of tryptophan (Trp) biosynthesis and is regulated by end-product inhibition (FIG. 1). AS is composed of two subunits, alpha and beta (TrpE and TrpG in prokaryotes, respectively), which together catalyze the synthesis of anthranilate from chorismate and glutamate: chorismate + L-glutamine ↔ anthranilate + pyruvate + L-glutamate. The AS holoenzyme is usually a tetramer including two large alpha subunits and two smaller beta subunits. The alpha subunit of AS (ASA; component I; encoded by an *ASA* gene) contributes both the catalytic and regulatory activities of the holoenzyme, whereas the beta subunit (ASB; component II; encoded by an *ASB* gene) has glutamine amido-transferase activity to provide an amino donor from glutamine to ASA. ASA is also able to catalyze the formation of anthranilate without ASB in the presence of high ammonium concentrations using ammonia as an amino donor. *ASA* and *ASB* genes in *Glycine max,* along with *ASA* orthologs in *Arabidopsis, Pisum sativum,* and *Phaseolis vulgaris,* are listed in Table 1. Expression of the Gm*ASA* genes are also described in Table 1.

**Table 1. Anthranilate synthase (AS) genes and their expression in Glycine max (Glyma), and AS gene orthologs in Arabidopsis thaliana (At), Pisum sativum (Psat), and Phaseolis vulgaris (Phvul).**

| **Gene Name** | **Glyma accession** | **At ortholog** | **Ps_Nette20 10.v1 ortholog** | **Phvul ortholog** | **Gm expression** | **Notes** |
|---|---|---|---|---|---|---|
| ASA1 | Glyma.18G 028900 | NA | Psat.5G382 800 | Phvul.001G 241600, Phvul.001G 241700 | Very lowly expressed | |
| ASA2 | Glyma.18G 028800 | NA | Psat.5G382 800 | Phvul.001G 241600, Phvul.001G 241700 | Not detected | Potential pseudogene |
| ASA3 | Glyma.20G 103200 | ASA2, AT2G2969 0 | Psat.6G644 600 | Phvul.007G 014800 | Broadly expressed at low levels | |
| ASA4 | Glyma. 10G 286100 | ASA2, AT2G2969 0 | Psat.6G644 600 | Phvul.007G 014800 | Not detected | Truncated |
| ASB | Glyma. 16G 058800 | NA | NA | NA | NA | NA |
| ASB | Glyma.07G 175000 | NA | NA | NA | NA | NA |

| | | | | | | |
|---|---|---|---|---|---|---|
| ASA = anthranilate synthase alpha subunit. ASB = anthranilate synthase beta subunit. | | | | | | |

The AS enzyme is feedback inhibited by the end product, Trp, by binding of Trp to an allosteric site on the alpha subunit of AS. *ASA* mutants insensitive to Trp feedback inhibition have been described in a number of plant species (Caligiuri and Bauerle (1991) J. Biol Chem 266(13):8328-8335; Matsui et al. (1987) J. Bacteriology 169(11):5330-5332; Graf et al. (1993) J. Bacteriology 175(4):1061-1068; Kreps et al. (1996) Plant Physiol. 110:1159-1165; Li and Last (1996) Plant Physiol. 110:51-59; Tozawa et al. (2001) Plant Physiol. 126:1493-1506; Song et al. (1998) Plant Physiol. 117:533-543; and Bohlmann et al. (1996) The Plant Journal 7(3):491-501). These mutants have mainly been identified through chemical mutagenesis screens, but naturally feedback insensitive mutants have been described in the plant species *Nicotiana tabacum* and *Ruta graveolens.* Mutations in *ASA* that confer Trp-insensitivity-chemically induced or naturally occurring-are located in two enzyme regulatory regions that encompass the Trp binding pocket, as mapped based on homology to bacterial TrpE structures. Two consensus motifs have been identified that are important for Trp feedback inhibition: Leu-Leu-Glu-Phe-X₁₀-Ser (SEQ ID NO: 17) and Asn-Pro-Ser-Pro-Tyr-Met (SEQ ID NO: 18) (Song et al. (1998) Plant Physiol. 117:533-543). Trp-insensitive *ASA* variants have been expressed in several plant species, increasing soluble Trp levels (Cho et al. (2000) Plant Physiol. 123(3):1069-1076; Inaba et al. (2007) Plant Cell Reports 26(10):1763-1771; Tsai et al. (2005) Plant Cell Reports 23(8):548-556; Tozawa et al. (2001) Plant Physiol. 126:1493-1506; Ishihara et al. (2006) Phytochemistry 67(21):2349-2362; Dubouzet et al. (2007) Journal of Experimental Botany 58(12):3309-3321; Yamada et al. (2005) Molecular Breeding 14(4):363-373; and Hanafy et al. (2006) Plant Science 171(6):670-676).

Soybean has four ASA isoforms, one of which is truncated (*GmASA4*) and one of which is a potential pseudogene (*GmASA2*)*.* These ASAs show diversity in the enzyme regulatory regions involved in Trp binding. Gm*ASA4* and Gm*ASA2* have large deletions in these respective regions, whereas Gm*ASA3* has a single amino acid deletion in the first enzyme regulatory region, mimicking the deletion seen in the naturally feedback insensitive *R. graveolens ASA* (*RgASA1*)*.* In other plant species, ASA isoforms have been shown to be non-allelic, exhibiting different expression patterns in response to stimuli such as wounding and bacterial infection.

*ASA* genes are found in legumes, including *Cajanus cajan, Glycine max, Phaseolus vulgaris, Vigna radiata, Pisum sativum, Medicago truncatula,* and *Arachis duranensis.* The Arabidopsis (*Arabidopsis thaliana*) and tobacco (*Nicotiana benthamiana*) *ASA* isoforms are named as in the literature, and the soy *ASA* isoforms are labelled as described herein and in Table 1. Expression of soy *ASA* genes as reported from Phytozome (World Wide Web at phytozome.jgi.doe.gov/pz/portal.html) and SoyBase (World Wide Web at soybase.org) RNA-seq datasets is shown in FIG. 2. Expression of pea *ASA* genes Psat.5G382800 and Psat.6G644600 is shown in FIGs. 3A and 3B. Expression of common bean *ASA* genes as reported by the PvGEA (World Wide Web at plantgrn.noble.org/PvGEA) RNA-seq datasets is shown in FIG. 4. The Pv *ASA* isoforms are named by orthology to soy ASA isoforms.

### Homocysteine S-methyltransferase

Methionine is synthesized through the aspartate family pathway in the endosperm and seed coat up to the mid-stages of seed filling (Gallardo et al. (2007) Molecular & Cellular Proteomics 6:2165-2179). In the model plant *Arabidopsis,* at later developmental stages methionine is also synthesized from the pool of *S*-methylmethionine (SMM) accumulated within the seed coat, which is the ostensible end-goal of the ` SMM cycle' (FIG. 5) (Bourgis et al. (1999) The Plant Cell 11:1485-1497; Gallardo et al. (2007) Molecular & Cellular Proteomics 6:2165-2179; Cohen et al. (2017) Plant Cell Reports 36(5):731-743). In this cycle, SMM is formed from methionine by a methionine *S*-methyltransferase (MMT) in vegetative tissue, transported through the phloem, and then converted back to methionine by homocysteine *S*-methyltransferase (HMT) in seeds (Bourgis et al. (1999) The Plant Cell 11:1485-1497; Gallardo et al. (2007) Molecular & Cellular Proteomics 6:2165-2179; Cohen et al. (2017) Plant Cell Reports 36(5):731-743). As such, the SMM cycle is proposed to play an important role in sulfur transport through plant tissues and provides a potential avenue by which to increase seed methionine content.

Most studies of the SMM cycle, and the relationship between HMT activity and methionine synthesis, have taken place in *Arabidopsis,* with only one study that has investigated the prevalence of SMM in other species (Bourgis et al. (1999) The Plant Cell 11:1485-1497). *Arabidopsis* has three *HMT* genes (At*HMT1,* At3g25900; At*HMT2,* At3g63250; and At*HMT3,* At3g22740). The Arabidopsis HMTs show differential feedback inhibition: AtHMT1 is sensitive to inhibition by physiological methionine concentrations, whereas both AtHMT2 and AtHMT3 are not (Ranocha et al. (2000) Journal of Biological Chemistry 275(21):15962-15968; Ranocha et al. (2001) The Plant Journal 25(5):575-584). The Arabidopsis HMTs also exhibit spatial and temporal expression patterns: At*HMT1* and At*HMT3* are more highly expressed in developing seeds, especially in the maturation stage, whereas At*HMT2* shows similar expression levels across tissues or life stages (Zhao et al. (2018) International journal of molecular sciences 19(4):1248; Zhao et al. (2018) Peer J 6:e6023). There also appears to be a compensatory relationship between the expression of cystathionine gamma-synthase (*CGS*)-the aspartate family pathway enzyme that performs the first committed step in methionine synthesis-and the *HMT* genes. Seed-specific overexpression of a feedback-insensitive AtCGS leads to an increase in At*HMT1* and At*HMT3* expression (Cohen et al. (2014) Plant Physiology 174:1322-1333). Interestingly, in an RNAi AtCGS knockdown line, At*HMT1* and At*HMT3* are also expressed higher than in wild-type plants, suggesting complex feedback mechanisms involving these genes (Cohen et al. (2017) Plant Cell Reports 36(5):731-743).

Lee et al. (Lee et al. (2008) The Plant Journal 54:310-320) found that *Arabidopsis hmt2 mutants-hmt2-1,* an alternative splice mutant, and *hmt2-2,* which contains a T-DNA insertion upstream of the start codon-exhibit increased SMM levels in vegetative tissue and increased free methionine in seeds. Cohen et al. (Cohen et al. (2017) Plant Cell Reports 36(5):731-743) obtained similar results with an additional Arabidopsis *hmt2* mutant, as well as investigated the roles of the *Arabidopsis HMT*s in methionine accumulation using a combination of RNAi, knockout, and overexpression lines. The results of this study point to consistent disagreement between At*HMT* RNAi and knockout phenotypes. For example, seed-specific knockdown of At*HMT2* had no effect on soluble seed methionine accumulation or SMM levels compared to a wild-type control; but, similar to the findings in Lee et al. 2008, seeds from an *hmt2* knockout line accumulated 22- and 6.5-fold more methionine and SMM, respectively, compared to a wild-type control. Moreover, seed-specific knockdown of At*HMT3* via RNAi led to 92% more soluble seed methionine and similar SMM levels compared to a WT control, whereas an *hmt3* knockout contained similar seed methionine levels and increased SMM levels compared to a WT control. Seed-specific overexpression of At*HMT3* led to only minor changes in methionine and SMM levels.

Compared to feedback insensitive *CGS* mutant lines or overexpression lines of *CGS* variants, the gains in soluble seed methionine reported in *Arabidopsis hmt2* mutants are striking. However, these studies have not been as widely reproduced, nor do they report any plant growth phenotypes.

Table 2 lists *Glycine max HMT* genes, along with *HMT* gene orthologs in *Arabidopsis thaliana, Pisum sativum, Phaseolis vulgaris,* and *Medicago truncatula.* Table 2 also describes expression of *Glycine max HMT* genes.

**Table 2. HMT genes and their expression in Glycine max; HMT gene orthologs in Arabidopsis thaliana, Pisum sativum, Phaseolis vulgaris, and Medicago truncatula.**

| **Gene Name** | **Glyma accession** | **At Ortholog** | **Ps_Nette20 10.v1 ortholog** | **Phvul ortholog** | **Medtr ortholog** | **Gm expression** |
|---|---|---|---|---|---|---|
| HMT1 | Glyma. 19G 158800 | AtHMT2, AT3G6325 0 | PsHMT2, Psat.3G236 900 | PvHMT2, Phvul.001G 153900 | Medtr7g09 5280 | Broadly expressed at low levels |
| HMT2 | Glyma.03G 156500 | NA | NA | NA | NA | Not detected |
| HMT3 | Glyma.20G 148900 | AtHMT3, AT3G2274 0 | PsHMT3, Psat.6G563 800 | PvHMT3, Phvul.007G 060300 | Medtr1g10 3290 | Broadly expressed at low levels |
| HMT4 | Glyma.08G 261200 | AtHMT1, AT3G2590 0 | PsHMT1, Psat.2G280 300 | PvHMT1, Phvul.003G 036100 | Medtr5g05 6640 | Very lowly expressed |

| | | | | | | |
|---|---|---|---|---|---|---|
| Glyma or *Gm=Glycine max.* At*=Arabidopsis thaliana.* Psat or Ps*=Pisum sativum.* Phvul*=Phaseolis vulgaris.* Medtr=*Medicago truncatula.* | | | | | | |

*HMT* isoforms are found across plant lineages, including *Arabidopsis thaliana, Solanum tuberosum, Medicago truncatula, Pisum sativum, Phaseolis vulgaris, Glycine max, Oryza sativa,* and *Marchantia polymorpha.* The *Arabidopsis HMT* isoforms (At*HMT*s) are labelled as in the literature, and the soybean *HMT* isoforms (GmHMTs) are labelled as described herein and in Table 2 (*HMT1,* Glyma.19G158800; *HMT2,* Glyma.03G156500; *HMT3,* Glyma.20G148900; *HMT4,* Glyma.08G261200). Expression of soy *HMT* genes as reported by the Phytozome and SoyBase RNA-seq datasets is shown in FIG. 6. Expression of pea *HMT* genes (Ps*HMT1*; Ps*HMT*3; and Ps*HMT2*) is shown in FIGs. 7A-7C. Expression of common bean (*Phaseolis vulgaris*) HMT genes as reported by the PvGEA RNA-seq datasets is shown in FIG. 8.

### B. Plants with one or more mutations in at least one endogenous AS gene and/or in at least one endogenous HMT gene

In some aspects, the plants and plant parts of the present disclosure comprise a genetic mutation in an endogenous *AS* gene or homolog thereof that alters AS activity and/or in an endogenous *HMT* gene or homolog thereof that decreases or eliminates HMT activity.

In some embodiments, the endogenous *AS* gene or homolog thereof includes an endogenous *ASA* gene or homolog thereof. In some embodiments, the endogenous *ASA* gene or homolog thereof comprising a genetic mutation encodes an ASA enzyme that is insensitive to or has reduced sensitivity to Trp feedback inhibition as compared to an ASA enzyme encoded by the corresponding *ASA* gene or homolog thereof without the genetic mutation. In some embodiments, the *ASA* gene comprising a genetic mutation encodes an ASA enzyme that has increased activity as compared to an ASA enzyme encoded by the corresponding *ASA* gene or homolog thereof without the genetic mutation.

In some embodiments, the endogenous *HMT* gene or homolog thereof comprising a genetic mutation encodes an HMT enzyme that has decreased or no activity as compared to an HMT enzyme encoded by the corresponding *HMT* gene or homolog thereof without the genetic mutation.

The genetic mutation that alters AS activity, *HMT* gene expression, HMT protein expression, and/or HMT activity in the plants and plant parts provided herein can comprise one or more insertions, substitutions, and/or deletions in at least one endogenous *AS* gene or homolog thereof and/or in at least one endogenous *HMT* gene or homolog thereof. A genetic mutation that alters AS activity can be located in at least one endogenous *AS* gene or homolog thereof (e.g., an *ASA* gene or homolog thereof); in a regulatory region of the endogenous *AS* gene or homolog thereof (e.g., an *ASA* gene or homolog thereof); a coding region, a non-coding region, or a regulatory region of any other gene; or at any other site in the genome of the plant or plant part. A genetic mutation that reduces or eliminates *HMT* gene expression, HMT protein expression, and/or HMT activity can be located in at least one endogenous *HMT* gene or homolog thereof (e.g., an *HMT1* or *HMT3* gene or homolog thereof); in a regulatory region of the endogenous *HMT* gene or homolog thereof (e.g., an *HMT1* or *HMT3* gene or homolog thereof); a coding region, a non-coding region, or a regulatory region of any other gene; or at any other site in the genome of the plant or plant part.

A "native" gene, as used herein, refers to any gene having a wild-type nucleic acid sequence, e.g., a nucleic acid sequence that can be found in the genome of a plant existing in nature, and need not naturally occur within the plant, plant part, or plant cell comprising such native gene. For example, a transgenic *ASA* gene located at a genomic site or in a plant in a non-naturally occurring matter is a "native" *ASA* gene if its nucleic acid sequence can be found in a plant existing in nature. A "regulatory region" of a gene, as used herein, refers to the region of a genome that controls expression of the gene. A regulatory region of a gene can include a genomic site where a RNA polymerase, a transcription factor, or other transcription modulators bind and interact to control mRNA synthesis of the gene, such as promoter regions, binding sites for transcription modulator proteins, and other genomic regions that contribute to regulation of transcription of the gene. A regulatory region of the gene can be located in the 5' untranslated region of the gene.

A control plant or plant part can be a plant or plant part to which a genetic mutation provided herein has not been introduced, e.g., by methods of the present disclosure. Thus, a control plant or plant part (e.g., seeds, fruit) may express a native (e.g., wild-type) *AS* gene or *HMT* gene endogenously or transgenically. A control plant of the present disclosure may be grown under the same environmental conditions (e.g., same or similar temperature, humidity, air quality, soil quality, water quality, and/or pH conditions) as a plant with a genetic mutation described herein. A plant or plant part (e.g., seeds, fruit) of the present disclosure may have altered AS activity, decreased or no expression of an *HMT* gene or homolog thereof, decreased or no expression of an HMT protein, decreased or no HMT activity, and/or increased seed Trp and/or Met content as compared to a control plant or plant part, when the plant or plant part of the present disclosure is grown under the same environmental conditions as the control plant or plant part.

A genetic mutation can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in at least one endogenous *ASA* gene or homolog thereof and/or in at least one endogenous *HMT* gene or homolog thereof in a genome of a plant or plant part.

A plant or plant part described herein can comprise at least 2 *ASA* genes, at least 3 *ASA* genes, or at least 4 *ASA* genes, each gene encoding an ASA protein and having less than 100% (e.g., less than 100%, 99%, 98%, 97%, 96%, 95%, 94%, 93%, 92%, 91%, 90%, 89%, 88%, 87%, 86%, or 85%) sequence identity to one another. A plant or plant part described herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions: in one *ASA* gene or homolog thereof; in a regulatory region of one *ASA* gene or homolog thereof; in more than one (e.g., 2 or 3), but not all *ASA* genes or homologs thereof; in regulatory regions of more than one (e.g., 2 or 3), but not all *ASA* genes or homologs thereof; in all *ASA* genes or homologs thereof; and/or in regulatory regions of all *ASA* genes or homologs thereof in the plant or plant part.

In some embodiments, the plant or plant part is from a legume. In certain embodiments, the legume is soybean (*Glycine max*) or pea (*Pisum sativum*)*.* In some embodiments, a soybean plant or plant part described herein can include 1, 2, 3, or 4 *ASA* genes, e.g., *ASA1* (Glyma.18G028900), *ASA2* (Glyma.18G028800), *ASA3* (Glyma.20G103200), and *ASA4* (Glyma.10G286100) genes, each encoding an ASA protein. A genetic mutation that alters AS activity can be located in one or more (e.g., one, more than one but not all, or all) *Glycine max ASA* genes, including a *Glycine max ASA1* gene, a *Glycine max ASA2* gene, a *Glycine max ASA3* gene, and a *Glycine max ASA4* gene. In some embodiments, the mutation is located in a *Glycine max ASA1* gene. In some embodiments, the mutation that alters AS activity can be located in an *ASA1* gene or homolog thereof comprising a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence set forth as SEQ ID NO: 1 and encoding an active ASA1, for example the nucleic acid sequence of SEQ ID NO: 1. In some embodiments, the mutation that alters AS activity can be located in an *ASA1* gene or homolog thereof encoding an ASA1 comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence set forth as SEQ ID NO: 2, wherein the ASA1 is active, for example an ASA1 comprising the amino acid sequence of SEQ ID NO: 2.

The genetic mutation can be located in an enzyme regulatory region of the encoded ASA. For example, an enzyme regulatory region comprises a region that binds Trp. As used herein, where an insertion, a substitution, or a deletion is "at least partially" in a certain nucleic acid region, the whole part of the insertion, substitution, or deletion can be within the certain nucleic acid region, or alternatively, can span across the certain nucleic acid region and a region outside the nucleic acid region. For instance, where an insertion, a substitution, or a deletion is at least partially in an exon, the whole part of the insertion, the substitution, or the deletion can be within the exon, or can span across the exon and a region (e.g., an intron, a regulatory region) upstream (5') or downstream (3') of the exon. In a plant or plant part provided herein comprising a genetic mutation that alters AS activity, the genetic mutation comprises at least one (e.g., about 1, 2, 3, 4, 5, 6, 7, 8, 9, 10, 11, 12, 13, 14, 15, 16, 17, 18, 19, 20, or more) in-frame deletion in an *ASA* gene. A plant or plant part of the present disclosure can comprise a deletion of about 3-30 nucleotides in an *ASA1* gene or homolog thereof. In some embodiments, a plant or plant part of the present disclosure can comprise a deletion of about 6-24 nucleotides in an *ASA1* gene or homolog thereof. In some embodiments, a plant or plant part of the present disclosure can comprise a deletion of about 6 nucleotides in an *ASA1* gene or homolog thereof. In some embodiments, a plant or plant part of the present disclosure can comprise a deletion of about 12 nucleotides in an *ASA1* gene or homolog thereof. In some embodiments, a plant or plant part of the present disclosure can comprise a deletion of about 24 nucleotides in an *ASA1* gene or homolog thereof. In some embodiments, the *ASA1* gene comprising the genetic mutation encodes an ASA1 protein having an amino acid sequence set forth as any one of SEQ ID NOs: 6, 7, and 8. In some embodiments, an ASA1 protein having an amino acid sequence set forth as any one of SEQ ID NOs: 6, 7, and 8 is insensitive to or has reduced sensitivity to Trp feedback inhibition as compared to an ASA1 protein encoded by the corresponding *ASA1* gene or homolog thereof without the genetic mutation. In some embodiments, the plant or plant part is soybean.

A plant or plant part described herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions: in one *HMT* gene or homolog thereof; in a regulatory region of one *HMT* gene or homolog thereof; in more than one (e.g., 2 or 3), but not all *HMT* genes or homologs thereof; or in regulatory regions of more than one (e.g., 2 or 3), but not all *HMT* genes or homologs thereof.

A genetic mutation that decreases or eliminates HMT activity can be located in one or more (e.g., one, more than one but not all, or all) *Glycine max HMT* genes, including a *Glycine max HMT1* gene, a *Glycine max HMT2* gene, a *Glycine max HMT3* gene, and a *Glycine max HMT4* gene. In some embodiments, the mutation is located in a *Glycine max HMT1* gene. In some embodiments, the mutation is located in a *Glycine max HMT3* gene. In some embodiments, a soybean plant or plant part described herein can include 1, 2, 3, or 4 *HMT* genes, e.g., *HMT1* (Glyma.19G158800), *HMT2* (Glyma.03G156500), *HMT3* (Glyma.20G148900), and *HMT4* (Glyma.08G261200) genes, each encoding an HMT protein. In some embodiments, a soybean plant or plant part described herein can comprise at least 2 *HMT* genes or at least 3 *HMT* genes, each gene encoding an HMT protein and having less than 100% (e.g., less than 100%, 99%, 98%, 97%, 96%, 95%, 94%, 93%, 92%, 91%, 90%, 89%, 88%, 87%, 86%, or 85%) sequence identity to one another.

The mutation that decreases or eliminates HMT activity can be located in an *HMT1* gene or homolog thereof comprising a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence set forth as SEQ ID NO: 9 and encoding an active HMT1, for example the nucleic acid sequence set forth as SEQ ID NO: 9. In some embodiments, the mutation that decreases or eliminates HMT activity can be located in an *HMT1* gene or homolog thereof encoding an HMT1 comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence set forth as SEQ ID NO: 10, wherein the HMT1 is active, for example an HMT1 comprising the amino acid sequence set forth as SEQ ID NO: 10. In some embodiments, the mutation that decreases or eliminates HMT activity is located in an *HMT3* gene or homolog thereof comprising a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence set forth as SEQ ID NO: 13 and encoding an active HMT3, for example the nucleic acid sequence set forth as SEQ ID NO: 13. In some embodiments, the mutation can be located in an *HMT3* gene or homolog thereof encoding an HMT3 comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to an amino acid sequence set forth as SEQ ID NO: 14, wherein the HMT3 is active, for example an HMT3 comprising an amino acid sequence set forth as SEQ ID NO: 14.

In the plant or plant part provided herein comprising a genetic mutation that decreases or eliminates HMT activity, the genetic mutation comprises at least one (e.g., about 1, 2, 3, 4, 5, 6, 7, 8, 9, 10, 11, 12, 13, 14, 15, 16, 17, 18, 19, 20, or more) deletion in an *HMT* gene. A plant or plant part of the present disclosure can comprise a deletion of about 1-70 nucleotides in an *HMT* gene or homolog thereof. In some embodiments, a plant or plant part of the present disclosure can comprise a deletion of 3-66 nucleotides in an *HMT1* gene or homolog thereof. In some embodiments, a plant or plant part of the present disclosure can comprise a deletion of 3 nucleotides in an *HMT1* gene or homolog thereof. In some embodiments, a plant or plant part of the present disclosure can comprise a deletion of 66 nucleotides in an *HMT1* gene or homolog thereof. In some embodiments, a plant or plant part of the present disclosure can comprise a deletion of 1-30 nucleotides in an *HMT3* gene or homolog thereof. In some embodiments, a plant or plant part of the present disclosure can comprise a deletion of 2-13 nucleotides in an *HMT3* gene or homolog thereof. In some embodiments, a plant or plant part of the present disclosure can comprise a deletion of 2 nucleotides in an *HMT3* gene or homolog thereof. In some embodiments, a plant or plant part of the present disclosure can comprise a deletion of 13 nucleotides in an *HMT3* gene or homolog thereof.

In some embodiments, a soybean plant or plant part of the present disclosure comprises: (i) an in-frame deletion of nucleotides 952 to 957 of *ASA1* having the nucleic acid sequence set forth as SEQ ID NO: 1, or a nucleic acid sequence comprising SEQ ID NO: 3 when the deletion of (i) is introduced; (ii) an in-frame deletion of nucleotides 948 to 959 of *ASA1* having the nucleic acid sequence set forth as SEQ ID NO: 1, or a nucleic acid sequence comprising SEQ ID NO: 4 when the deletion of (ii) is introduced; (iii) an in-frame deletion of nucleotides 938 to 961 of *ASA1* having the nucleic acid sequence set forth as SEQ ID NO: 1, or a nucleic acid sequence comprising SEQ ID NO: 5 when the deletion of (iii) is introduced; (iv) a deletion of nucleotides 712 to 714 of *HMT1* having the nucleic acid sequence set forth as SEQ ID NO: 9, or a nucleic acid sequence comprising SEQ ID NO: 11 when the deletion of (iv) is introduced; (v) a deletion of nucleotides 695 to 760 of *HMT1* having the nucleic acid sequence set forth as SEQ ID NO: 9, or a nucleic acid sequence comprising SEQ ID NO: 12 when the deletion of (v) is introduced; (vi) a deletion of nucleotides 2783 to 2784 of *HMT3* having the nucleic acid sequence set forth as SEQ ID NO: 13, or a nucleic acid sequence comprising SEQ ID NO: 15 when the deletion of (vi) is introduced; or (vii) a deletion of nucleotides 2778 to 2790 of *HMT3* having the nucleic acid sequence set forth as SEQ ID NO: 13, or a nucleic acid sequence comprising SEQ ID NO: 16 when the deletion of (vii) is introduced.

The plants or plant parts described herein can comprise a genetic mutation that alters AS and/or HMT activity, e.g., one or more (e.g., about 1, 2, 3, 4, 5, 6, 7, 8, 9, 10, 11, 12, 13, 14, 15, 16, 17, 18, 19, 20, or more) insertions, substitutions, and/or deletions in a regulatory region of at least one (e.g., one, more than one but not all, or all) *AS* gene (e.g., *ASA* gene) or homolog thereof and/or *HMT* gene or homolog thereof. The regulatory region having the mutation can comprise a promoter region, a binding site (e.g., an enhancer sequence) for a transcription modulator protein (e.g., transcription factor), or other genomic regions that contribute to regulation of transcription of an *AS* gene (e.g., *ASA* gene) or homolog thereof and/or *HMT* gene or homolog thereof. One or more insertions, substitutions, and/or deletions can be introduced into a promoter region, a transcription modulator protein (e.g., transcription factor) binding site, or other regulatory regions of at least one (e.g., one, more than one but not all, or all) *AS* gene (e.g., *ASA* gene) or homolog thereof and/or *HMT* gene or homolog thereof to confer to the plant or plant part an altered (e.g., increased, decreased) transcription activity of an *AS* gene (e.g., *ASA* gene) or homolog thereof and/or *HMT* gene or homolog thereof.

In some embodiments, the mutation is in a promoter region of at least one (e.g., one, more than one but not all, or all) *AS* gene (e.g., *ASA* gene) or homolog thereof and/or *HMT* gene or homolog thereof. As used herein, a "promoter" refers to an upstream regulatory region of DNA prior to the ATG of a native gene, having a transcription initiation activity (e.g., function) for the gene and other downstream genes. "Transcription initiation" as used herein refers to a phase or a process during which the first nucleotides in the RNA chain are synthesized. It is a multistep process that starts with formation of a complex between a RNA polymerase holoenzyme and a DNA template at the promoter, and ends with dissociation of the core polymerase from the promoter after the synthesis of approximately the first nine nucleotides. A promoter sequence can include a 5' untranslated region (5' UTR), including intronic sequences, in addition to a core promoter that contains a TATA box capable of directing RNA polymerase II (pol II) to initiate RNA synthesis at the appropriate transcription initiation site for a particular polynucleotide sequence of interest. A promoter may additionally comprise other recognition sequences positioned upstream of the TATA box, and well as within the 5' UTR intron, which influence the transcription initiation rate. The one or more insertions, substitutions, and/or deletions in the promoter region of an *AS* gene (e.g., *ASA* gene) or homolog thereof and/or *HMT* gene or homolog thereof can alter the transcription initiation activity of the promoter. For example, the modified promoter can enhance or reduce transcription of the operably linked nucleic acid molecule (e.g., an *ASA* and/or *HMT* gene), initiate transcription in a developmentally-regulated or temporally-regulated manner, initiate transcription in a cell-specific, cell-preferred, tissue-specific, or tissue-preferred manner, or initiate transcription in an inducible manner. A deletion, a substitution, or an insertion, e.g., introduction of a heterologous promoter sequence, a cis-acting factor, a motif or a partial sequence from any promoter, including those described elsewhere in the present disclosure, can be introduced into the promoter region of an *AS* gene (e.g., *ASA* gene) or homolog thereof and/or *HMT* gene or homolog thereof to confer an altered (e.g., increased, decreased) transcription initiation function according to the present disclosure. The insertion, substitution, or deletion can comprise insertion, substitution, or deletion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides. The substitution can be a cisgenic substitution, a transgenic substitution, or both. In some embodiments, the mutation of a promoter results in an enhanced activity of the promoter sequence as compared to a control promoter that is not mutated, or results in a reduced activity of the promoter as compared to a control promoter that is not mutated. In some embodiments, the mutation is in the upstream region of a promoter of at least one (e.g., one, more than one but not all, or all) *AS* gene (e.g., *ASA* gene) or homolog thereof and/or *HMT* gene or homolog thereof.

In some embodiments, a mutation is located in the gene encoding (or regulating expression of) one or more transcription factors that regulates expression of an *AS* gene (e.g., *ASA* gene) or homolog thereof and/or *HMT* gene or homolog thereof. A "transcription factor" as used herein refers to a protein (other than an RNA polymerase) that regulates transcription of a target gene. A transcription factor has DNA-binding domains to bind to specific genomic sequences such as an enhancer sequence or a promoter sequence. In some instances, a transcription factor binds to a promoter sequence near the transcription initiation site and regulate formation of the transcription initiation complex. A transcription factor can also bind to regulatory sequences, such as enhancer sequences, and modulate transcription of the target gene. The mutation in the gene encoding (or regulating expression of) a transcription factor can modulate expression or function of the transcription factor. In some embodiments, the mutation in the gene encoding (or regulating expression of) a transcription factor increases expression levels of an *AS* gene, e.g., by enhancing transcription initiation activity of an *ASA* gene promoter. In some embodiments, the mutation in the gene encoding (or regulating expression of) a transcription factor reduces expression levels of an *HMT* gene, e.g., by inhibiting transcription initiation activity of the *HMT* gene promoter. In some embodiments, the mutation modifies or inserts transcription factor binding sites or enhancer elements that regulates *AS* and/or *HMT* gene expression into the regulatory region of the *AS* and/or *HMT* gene.

In some embodiments, the mutation inserts a part or whole of one or more negative regulatory sequences of an *HMT* gene or homolog thereof into the genome of a plant cell or plant part. The negative regulatory sequence of the *HMT* gene or homolog thereof can be in a cis location or in a trans location. Negative regulatory sequences of an *HMT* gene or homolog thereof can also include upstream open reading frames (uORFs). In some instances, a negative regulatory sequence can be inserted in a region upstream of an *HMT* gene or homolog thereof to inhibit expression and/or function of the *HMT* gene or homolog thereof. In some embodiments, the mutation inserts a part or whole of one or more positive regulatory sequences of an *AS* gene or homolog thereof into the genome of a plant cell or plant part. The positive regulatory sequence of the *AS* gene or homolog thereof can be in a cis location or in a trans location. Positive regulatory sequences of an *AS* gene or homolog thereof can also include upstream open reading frames (uORFs). In some instances, a positive regulatory sequence can be inserted in a region upstream of an *AS* gene or homolog thereof to enhance expression and/or function of the *AS* gene or homolog thereof.

A plant or plant part of the present disclosure can have a genetic mutation that alters AS and/or HMT activity in a homolog or variant of an *AS* gene (e.g., *ASA* gene) and/or an *HMT* gene disclosed herein and encodes an active AS protein and/or an active HMT protein, or in a regulatory region of such homolog or variant of an *AS* gene and/or an *HMT* gene.

Variant sequences (e.g., homologs) can be isolated by PCR. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Variant sequences (e.g., homologs) may also be identified by analysis of existing databases of sequenced genomes. In this manner, variant sequences encoding AS and/or HMT can be identified and used in the methods of the present disclosure.

In certain instances, mutations in an *AS* gene (e.g., *ASA* gene) or homolog thereof and/or *HMT* gene or homolog thereof in a plant, plant part, or plant product (e.g., plant protein composition) can be identified by a diagnostic method described herein. Such diagnostic methods include use of primers for detecting a genetic mutation in an *AS* gene or homolog thereof and/or *HMT* gene or homolog thereof. In certain instances, a kit comprising a set of primers can be used for detecting a genetic mutation in an *AS* gene or homolog thereof and/or *HMT* gene or homolog thereof in soybean plants, plant parts, or plant products (e.g., plant protein composition).

In some embodiments, the mutations, e.g., one or more insertions, substitutions, and/or deletions, are integrated into the plant genome, and the plant or the plant part is stably transformed. In other embodiments, the one or more mutations are not integrated into the plant genome, and wherein the plant or the plant part is transiently transformed.

One or more mutations, e.g., insertions, substitutions, and/or deletions, located in at least one endogenous *AS* gene (e.g., *ASA* gene) or homolog thereof and/or in at least one endogenous *HMT* gene or homolog thereof in the genome of the plant or plant part can alter AS activity, decrease or eliminate expression of an *HMT* gene or homolog thereof, decrease or eliminate expression of an HMT protein, decrease or eliminate HMT activity, and/or increase seed Trp and/or Met content relative to a control plant or plant part, e.g., when grown under the same environmental conditions, as further described in the present disclosure.

### C. Plants with altered activity of anthranilate synthase (AS) and/or decreased or no activity of homocysteine-S-methyltransferase (HMT)

Provided herein are plants and plant parts (e.g., seeds, fruits) comprising altered AS activity and/or decreased or no HMT activity as compared to a control plant or plant part. As used herein, "anthranilate synthase (AS) activity" or "anthranilate synthase alpha subunit (ASA) activity" or "active anthranilate synthase (AS)" or "active anthranilate synthase alpha subunit (ASA)" refers to the ability of an AS enzyme to catalyze production of anthranilate from chorismate using an amino donor such as glutamine or ammonia. In some embodiments, the AS is ASA. In some embodiments, the mutated ASA enzyme is insensitive to or has reduced sensitivity to Trp feedback inhibition as compared to an ASA enzyme encoded by the corresponding *ASA* gene that is not mutated. In some embodiments, the mutated ASA enzyme has increased activity as compared to an ASA enzyme encoded by the corresponding *ASA* gene that is not mutated. In some embodiments, the plants and plant parts are from a legume. In certain embodiments, the legume is soybean *(Glycine max*) or pea *(Pisum sativum).*

As used herein, "homocysteine S-methyltransferase (HMT) activity" or "active homocysteine S-methyltransferase (HMT)" refers to the ability of an HMT enzyme to catalyze production of Met from the substrates SMM and homocysteine (or other methyl acceptor). In some embodiments, the HMT activity occurs in seed. In some embodiments, the mutated HMT enzyme has decreased or no activity as compared to an HMT enzyme encoded by the corresponding *HMT* gene that is not mutated.

Plants, plant parts (e.g., seeds, fruit), or plant products (e.g., plant protein composition) of the present disclosure can comprise: (i) altered AS activity, and/or (ii) reduced or no HMT activity as compared to AS and/or HMT activity in a control plant, plant part, or plant product. In some embodiments, the plants, plant parts, or plant products are from a legume. In certain embodiments, the legume is soybean (*Glycine max*) or pea (*Pisum sativum*)*.* Kinetics of enzyme activity including Vmax, the maximum rate of reaction (i.e. the rate of reaction when the enzyme is saturated with substrate) and Km (Michaelis constant), an inverse measure of enzyme affinity of an enzyme for its substrate, may be assessed. In some embodiments, Km is the concentration of substrate which permits the enzyme to achieve half Vmax. An enzyme with a high Km has a low affinity for its substrate, and requires a greater concentration of substrate to achieve Vmax. Km and Vmax may be determined by incubating an enzyme with varying concentrations of substrate, and the results may be plotted as a graph of rate of reaction (v) against concentration of substrate ([S]), and will normally yield a hyperbolic curve. The relationship among v, Vmax, Km, and [S] is defined by the Michaelis-Menten equation: v = Vmax / (1 + (Km/[S])). One of skill in the art knows that Vmax and Km may also be assessed by re-arranging the Michaelis-Menten equation to other plots, including the Lineweaver-Burk double reciprocal plot, the Eadie-Hofstee plot, and the Hanes plot.

In some embodiments, the Km for a mutated AS enzyme of the disclosure is 1.5- to 10-fold less, or 2- to 8-fold less, or 2- to 4-fold less as compared to the Km for a control AS enzyme. In some embodiments, the Km for a mutated AS enzyme of the disclosure is 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.5-fold, 4-fold, 4.5-fold, 5-fold, 6-fold, 7-fold, 8-fold, 9-fold, 10-fold less, or lesser, as compared to the Km for a control AS enzyme.

In some embodiments, the Vmax for a mutated AS enzyme of the disclosure is 1.5- to 10-fold greater, or 2- to 8-fold greater, or 2- to 4-fold greater as compared to the Vmax for a control AS enzyme. In some embodiments, the Vmax for a mutated AS enzyme of the disclosure is 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.5-fold, 4-fold, 4.5-fold, 5-fold, 6-fold, 7-fold, 8-fold, 9-fold, 10-fold greater, or greater, as compared to the Vmax for a control AS enzyme.

In some embodiments, a plant, plant part, plant cell, or plant product comprises a genetic mutation in at least one endogenous *ASA* gene or homolog thereof, wherein the mutated endogenous *ASA* gene or homolog thereof encodes an ASA protein that is insensitive to or has reduced sensitivity to Trp feedback inhibition as compared to an ASA protein encoded by the corresponding *ASA* gene or homolog thereof without the genetic mutation. Trp feedback inhibition refers to the inhibition of AS activity by the end product Trp. The inhibition is effected by Trp binding to an allosteric site on the alpha subunit of AS (ASA).

Sensitivity of an ASA enzyme to Trp feedback inhibition can be assessed by measuring ASA enzyme activity in the presence of increasing concentrations of Trp as described in e.g., Cho et al. (2000) Plant Physiology 123:1069-1076; Bernasconi et al. (1994) Plant Physiol 106: 353-358; Song et al. (1998) 117:533-543; Widholm JM (1971) Physiol Plant 5: 75-79; and Li and Last (1996) Plant Physiol 110: 51-59. For example, an AS enzyme solution can be prepared by grinding of plant tissue, (NH4)₂SO₄ precipitation, and resuspension of the precipitate in extraction buffer. The enzyme solution may be further desalted with Sephadex-25. The assay buffer to measure AS activity may include, for example: a buffer such as 50 mm Hepes, pH 7.5 or Tris-HCl, pH 8.0; chorismate as the first substrate (e.g., concentrations of 0 to 500 µM); Gln (e.g., 10-20 mM) or NH4+ (e.g., 100 mM) as the second substrate; MgCh (e.g., 2-5 mM); 0.05 mM Na₂EDTA; DTT (e.g., 0.05-2.0 mM); and glycerol (e.g., 5-20%). Trp concentrations may include a range of 0 to 1000 µM. Chorismate may be produced using the fermentation method of Gibson (1970) Methods Enzymol 17: 362-364. The anthranilate product may be extracted with ethylacetate, centrifuged, and the upper phase quantified by fluorescence emission at 400 nm (excitation at 340 nm) with a fluorescence spectrophotometer. AS enzyme activity may be expressed as concentration of anthranilate per time per amount of protein (e.g., nmol anthranilate formed min⁻¹mg⁻¹ protein). The enzyme activity assays can include a control AS enzyme. A control AS enzyme includes an AS enzyme that does not have the genetic mutation and/or is sensitive to Trp feedback inhibition.

The Kᵢ for Trp, the concentration of Trp where AS activity is inhibited by 50% as compared to activity of a control AS enzyme, may be determined for an AS enzyme having a genetic mutation that confers reduced sensitivity to Trp feedback inhibition. In some embodiments, the Kᵢ Trp value for a mutated ASA enzyme of the disclosure is 2- to 200-fold greater, or 2- to 100-fold greater, or 2- to 50-fold greater as compared to the Kᵢ Trp value for a control ASA enzyme. In some embodiments, the Kᵢ Trp value for a mutated ASA enzyme of the disclosure is 2-fold, 3-fold, 4-fold, 5-fold, 6-fold, 7-fold, 8-fold, 9-fold, 10-fold, 15-fold, 20-fold, 25-fold, 30-fold, 35-fold, 40-fold, 45-fold, 50-fold, 55-fold, 60-fold, 65-fold, 70-fold, 75-fold, 80-fold, 85-fold, 90-fold, 95-fold, 100-fold, 110-fold, 120-fold, 130-fold, 140-fold, 150-fold, 160-fold, 170-fold, 180-fold, 190-fold, 200-fold, or greater, as compared to the Kᵢ Trp value for a control ASA enzyme.

A plant, plant part, plant cell, or plant product can comprise a genetic mutation in at least one endogenous *ASA* gene or homolog thereof, wherein the mutated endogenous *ASA* gene or homolog thereof encodes an ASA protein having increased activity as compared to activity of an ASA protein encoded by the corresponding *ASA* gene or homolog thereof without the genetic mutation in a control plant, plant part, plant cell, or plant product. In some embodiments, a plant, plant part, plant cell, or plant product comprises a genetic mutation in at least one endogenous *ASA* gene or homolog thereof, wherein the mutated endogenous *ASA* gene or homolog thereof encodes an ASA protein having 5-100%, 10-100%, 20-100%, 30-100%, 40-100%, 50-100%, 60-100%, 70-100%, 80-100%, 20-90%, 30-90%, 40-90%, 50-90%, 60-90%, or 70-90% increase in activity as compared to activity of an ASA protein encoded by the corresponding *ASA* gene or homolog thereof without the genetic mutation in a control plant, plant part, plant cell, or plant product. In some embodiments, a soybean plant, plant part, plant cell, or plant product comprises a genetic mutation in at least one endogenous *ASA* gene or homolog thereof, wherein the mutated endogenous *ASA* gene encodes an ASA protein having 10-20%, 20-30%, 30-40%, 40-50%, 50-60%, 60-70%, 70-80%, 80-90%, or 90-100% increase in activity, or more as compared to activity of an ASA protein encoded by the corresponding *ASA* gene or homolog thereof without the genetic mutation in a control plant, plant part, plant cell, or plant product. In some embodiments, a plant, plant part, plant cell, or plant product comprises a genetic mutation in at least one endogenous *ASA* gene or homolog thereof, wherein the mutated endogenous *ASA* gene encodes an ASA protein having 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100% increase in activity, or more as compared to activity of an ASA protein encoded by the corresponding *ASA* gene or homolog thereof without the genetic mutation in a control plant, plant part, plant cell, or plant product. In some embodiments, the plant, plant part, plant cell, or plant product is from a legume. In certain embodiments, the legume is soybean (*Glycine max*) or pea (*Pisum sativum*)*.* In some embodiments, the *ASA* gene or homolog thereof includes a *Glycine max ASA1* gene (Glyma.18G028900), a *Glycine max ASA2* gene (Glyma.18G028800), a *Glycine max ASA3* gene (Glyma.20G103200), or a *Glycine max ASA4* gene (Glyma.10G286100). In some embodiments, the *ASA* gene or homolog thereof includes a *Glycine max ASA1* gene (Glyma.18G028900).

A mutated AS enzyme that is insensitive to or has reduced sensitivity to Trp feedback inhibition can be resistant to toxic anthranilate analogs including: 3-methyl-anthranilate (3MA), 5-methyl-anthranilate (5MA), 6-methyl-anthranilate (6MA), 5-fluoro-anthranilate (5FA), and 6-fluoro-anthranilate (6FA); and/or toxic Trp analogs including: 4-methyl-Trp (4MT), 5-methyl-Trp (5MT), 6-methyl-Trp (6MT), 7-methyl-Trp (7MT), and alpha-methyl-Trp (αMT). Resistance to toxic anthranilate and/or Trp analogs can be ascertained by growing plants in petri dishes containing agar supplemented with a toxic analog and monitoring growth of plant seedlings.

HMT enzyme activity may be assessed as described in e.g., Lee et al. (2008) The Plant Journal 54:310-320, Ranocha et al. (2000) Journal of Biological Chemistry 275(21):15962-15968, and Mudd and Datko (1990) Plant Physiol. 93, 623-630. Soluble protein may be extracted from plant tissue in HEPES-KOH buffer, pH 7.5. Protein concentration may be determined using Bradford reagent (Bio-Rad, Hercules, CA) by measuring absorbance at 595 nm with a spectrophotometer and using bovine serum albumin as the standard. An HMT activity assay may be performed at 30°C for 30 min in a volume of 50-100 µl. In some embodiments, the assay mixture contains 20 mM HEPES-KOH, pH 7.5, 2 mM DTT, 2 mM homocysteine (or other methyl acceptor), 1 mM SMM, and 70 µg of total protein. The reaction may be initiated by adding plant extracts and terminated by adding an equal volume of 40 mM HCl containing 20 µM Nor-Leu as an HPLC (high performance liquid chromatography) injection standard. The reaction may be analyzed by HPLC. In some embodiments, assays may be performed under conditions in which substrates are saturating and product formation is proportional to enzyme level and time. In some embodiments, the SMM substrate may be radiolabeled (e.g., with [³⁵S] SMM), and the amount of formed [³⁵S] Met counted in a scintillation counter (Ranocha et al. (2000) Journal of Biological Chemistry 275(21):15962-15968). The enzyme activity assays can include a control HMT enzyme. A control HMT enzyme includes an HMT enzyme that does not have the genetic mutation.

A plant, plant part, plant cell, or plant product can comprise a genetic mutation in at least one endogenous *HMT* gene or homolog thereof, wherein the mutated endogenous *HMT* gene or homolog thereof encodes an HMT protein having reduced or no activity as compared to activity of an HMT protein encoded by the corresponding *HMT* gene or homolog thereof without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having 5-100%, 10-100%, 20-100%, 30-100%, 40-100%, 50-100%, 60-100%, 70-100%, 80-100%, 20-90%, 30-90%, 40-90%, 50-90%, 60-90%, or 70-90% reduction in activity as compared to activity of an HMT protein encoded by the corresponding *HMT* gene or homolog thereof without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having 10-20%, 20-30%, 30-40%, 40-50%, 50-60%, 60-70%, 70-80%, 80-90%, or 90-100% reduction in activity as compared to activity of an HMT protein encoded by the corresponding *HMT* gene without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% reduction in activity as compared to activity of an HMT protein encoded by the corresponding *HMT* gene or homolog thereof without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having no activity. In some embodiments, the plant, plant part, plant cell, or plant product is from a legume. In certain embodiments, the legume is soybean *(Glycine max*) or pea *(Pisum sativum).* In some embodiments, the *HMT* gene or homolog thereof includes a *Glycine max HMT1* gene (Glyma.19G158800), a *Glycine max HMT2* gene (Glyma.03G156500), a *Glycine max HMT3* gene (Glyma.20G148900), or a *Glycine max HMT4* gene (Glyma.08G261200). In some embodiments, the *HMT* gene or homolog thereof includes a *Glycine max HMT1* gene (Glyma.19G158800). In some embodiments, the *HMT* gene or homolog thereof includes a *Glycine max HMT3* gene (Glyma.20G148900).

### D. Plants with reduced expression level of HMT gene and/or HMT protein

Embodiments of the present disclosure provide for a plant, plant part (e.g., seeds, fruit), plant cell, or plant product (e.g., plant protein composition) comprising a genetic mutation (e.g., one or more insertions, substitutions, and/or deletions) in at least one endogenous *HMT* gene or homolog thereof having a reduced expression level or no expression of the endogenous mutated *HMT* gene or homolog thereof as compared to the expression level of the corresponding *HMT* gene or homolog thereof without such mutation in a control plant, plant part, plant cell, or plant product. In some embodiments, a plant, plant part, plant cell, or plant product (e.g., plant protein composition) comprises a genetic mutation in at least one endogenous *HMT* gene or homolog thereof, wherein the expression level of the mutated endogenous *HMT* gene or homolog thereof is reduced by 10-100%, 20-100%, 30-100%, 40-100%, 50-100%, 60-100%, 70-100%, 80-100%, 20-90%, 30-90%, 40-90%, 50-90%, 60-90%, or 70-90% as compared to the expression level of the corresponding *HMT* gene or homolog thereof without the genetic mutation in a control plant, plant part, plant cell, or plant product. In some embodiments, a plant, plant part, plant cell, or plant product (e.g., plant protein composition) comprises a genetic mutation in at least one endogenous *HMT* gene or homolog thereof, wherein the expression level of the mutated endogenous *HMT* gene or homolog thereof is reduced by 10-20%, 20-30%, 30-40%, 40-50%, 50-60%, 60-70%, 70-80%, 80-90%, or 90-100% as compared to the expression level of the corresponding *HMT* gene or homolog thereof without the genetic mutation in a control plant, plant part, plant cell, or plant product. In some embodiments, a plant, plant part, plant cell, or plant product (e.g., plant protein composition) comprises a genetic mutation in at least one endogenous *HMT* gene or homolog thereof, wherein the expression level of the mutated endogenous *HMT* gene or homolog thereof is reduced by 10%, 15%, 20%, 25%, 30%, 35%, 40%, 45%, 50%, 55%, 60%, 65%, 70%, 75%, 80%, 85%, 90%, 95%, or 100%, or at least 10%, 15%, 20%, 25%, 30%, 35%, 40%, 45%, 50%, 55%, 60%, 65%, 70%, 75%, 80%, 85%, 90%, 95%, or 100%, as compared to the expression level of the corresponding *HMT* gene or homolog thereof without the genetic mutation in a control plant, plant part, plant cell, or plant product. In some embodiments, a plant, plant part, plant cell, or plant product (e.g., plant protein composition) comprises a genetic mutation in at least one endogenous *HMT* gene or homolog thereof, wherein there is no expression of the mutated endogenous *HMT* gene or homolog thereof. In some embodiments, the plant, plant part, plant cell, or plant product is from a legume. In certain embodiments, the legume is soybean (*Glycine max*) or pea (*Pisum sativum*)*.* In some embodiments, the *HMT* gene or homolog thereof includes a *Glycine max HMT1* gene (Glyma.19G158800), a *Glycine max HMT2* gene (Glyma.03G156500), a *Glycine max HMT3* gene (Glyma.20G148900), or a *Glycine max HMT4* gene (Glyma.08G261200). In some embodiments, the *HMT* gene or homolog thereof includes a *Glycine max HMT1* gene (Glyma.19G158800). In some embodiments, the *HMT* gene or homolog thereof includes a *Glycine max HMT3* gene (Glyma.20G148900).

Expression levels of an *HMT* gene or homolog thereof can be measured by any standard methods for measuring mRNA levels of a gene, including quantitative RT-PCR, northern blot, and serial analysis of gene expression (SAGE).

Embodiments of the present disclosure provide for a plant, plant part (e.g., seeds, fruit), plant cell, or plant product (e.g., plant protein composition) comprising a genetic mutation (e.g., one or more insertions, substitutions, and/or deletions) in at least one endogenous *HMT* gene or homolog thereof, wherein the plant, plant part, plant cell, or plant product has reduced or no expression of the HMT protein encoded by the mutated endogenous *HMT* gene or homolog thereof, as compared to the expression level of an HMT protein encoded by the corresponding *HMT* gene or homolog thereof without the genetic mutation in a control plant, plant part, plant cell, or plant product. In some embodiments, a plant, plant part, plant cell, or plant product comprises a mutated endogenous *HMT* gene or homolog thereof, wherein the mutated endogenous *HMT* gene or homolog thereof encodes a full-length HMT protein that has reduced expression as compared to expression of a full-length HMT protein encoded by the corresponding *HMT* gene or homolog thereof without the genetic mutation in a control plant, plant part, or plant product. A "full-length" HMT protein, as used herein, refers to an HMT protein comprising the complete amino acid sequence of a wild-type HMT protein, e.g., encoded by a native *HMT* gene. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having an expression level that is reduced by 10-100%, 20-100%, 30-100%, 40-100%, 50-100%, 60-100%, 70-100%, 80-100%, 20-90%, 30-90%, 40-90%, 50-90%, 60-90%, or 70-90%, as compared to the expression level of an HMT protein encoded by the corresponding *HMT* gene or homolog thereof without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having an expression level that is reduced by 10-20%, 20-30%, 30-40%, 40-50%, 50-60%, 60-70%, 70-80%, 80-90%, or 90-100%, as compared to the expression level of an HMT protein encoded by the corresponding *HMT* gene or homolog thereof without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein having an expression level that is reduced by 10%, 15%, 20%, 25%, 30%, 35%, 40%, 45%, 50%, 55%, 60%, 65%, 70%, 75%, 80%, 85%, 90%, 95%, or 100%, or at least 10%, 15%, 20%, 25%, 30%, 35%, 40%, 45%, 50%, 55%, 60%, 65%, 70%, 75%, 80%, 85%, 90%, 95%, or 100%, as compared to the expression level of an HMT protein encoded by the corresponding *HMT* gene or homolog thereof without the genetic mutation in a control plant, plant part, plant cell, or plant product. In some embodiments, a plant, plant part, plant cell, or plant product comprises a genetic mutation in at least one endogenous *HMT* gene or homolog thereof, wherein the mutated endogenous *HMT* gene or homolog thereof encodes an HMT protein that is not expressed.

In some embodiments, the plant, plant part, plant cell, or plant product is from a legume. In certain embodiments, the legume is soybean (*Glycine max*) or pea (*Pisum sativum*)*.* In some embodiments, the HMT protein is encoded by an *HMT* gene or homolog thereof including a *Glycine max HMT1* gene (Glyma.19G158800), a *Glycine max HMT2* gene (Glyma.03G156500), a *Glycine max HMT3* gene (Glyma.20G148900), and a *Glycine max HMT4* gene (Glyma.08G261200). In some embodiments, the *HMT* gene or homolog thereof includes a *Glycine max HMT1* gene (Glyma. 19G158800). In some embodiments, the HMT protein is encoded by an *HMT* gene or homolog thereof including a *Glycine max HMT3* gene (Glyma.20G148900).

Expression levels of an HMT protein encoded by an *HMT* gene or homolog thereof in a plant, plant part, plant cell, or plant product can be measured by any standard methods for measuring protein levels, including western blot analysis, ELISA, or dot blot analysis of a protein sample obtained from a plant, plant part, plant cell, or plant product using an antibody directed to the HMT protein encoded by an *HMT* gene or homolog thereof.

### E. Plants with increased seed Trp and/or Met content

Embodiments of the present disclosure provide a plant, plant part (e.g., seeds, fruit), or plant product (e.g., plant protein composition) comprising a genetic mutation (e.g., one or more insertions, substitutions, and/or deletions) in an endogenous *AS* gene or homolog thereof and/or in an endogenous *HMT* gene or homolog thereof, wherein the plant, plant part (e.g., seeds, fruit), or plant product has an increased level of Trp and/or Met content as compared to a control plant, plant part, or plant product without the genetic mutation. In some embodiments, the *AS* gene or homolog thereof is an *ASA* gene or homolog thereof. In some embodiments, the increased level of Trp and/or Met content is in seed produced from the plant, plant part, or plant product comprising the genetic mutation. In some embodiments, the disclosure provides a plant or plant part comprising increased tryptophan (Trp), methionine (Met), and/or SMM content in vegetative tissues.

In some embodiments, seed of a plant or a plant product comprising a genetic mutation in an endogenous *AS* gene or homolog thereof and/or in an endogenous *HMT* gene or homolog thereof has an increase in Trp and/or Met content of 10-500%, 20-450%, 30-400%, 40-300%, 50-200%, 60-200%, 70-200%, 80-200%, 20-100%, 30-100%, 40-100%, 50-100%, 60-100%, or 70-100%, as compared to Trp and/or Met content in seed of a control plant or in a control plant product without the genetic mutation. In some embodiments, seed of a plant or a plant product comprising a genetic mutation in an endogenous *AS* gene or homolog thereof and/or in an endogenous *HMT* gene or homolog thereof has an increase in Trp and/or Met content of 10-20%, 20-30%, 30-40%, 40-50%, 50-60%, 60-70%, 70-80%, 80-90%, 90-100%, 100-120%, 120-140%, 140-160%, 160-180%, 180-200%, 200-220%, 220-240%, 240-260%, 260-280%, 280-300%, 300-320%, 320-340%, 340-360%, 360-380%, 380-400%, 400-420%, 420-440%, 440-460%, 460-480%, or 480-500%, as compared to Trp and/or Met content in seed of a control plant or in a control plant product without the genetic mutation. In some embodiments, seed of a plant or a plant product comprising a genetic mutation in an endogenous *AS* gene or homolog thereof and/or in an endogenous *HMT* gene or homolog thereof has an increase in Trp and/or Met content of 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, or at least 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, or 500%, or more as compared to Trp and/or Met content in seed of a control plant or in a control plant product without the genetic mutation.

In some embodiments, seed of a plant or a plant product comprising a genetic mutation in an endogenous *AS* gene or homolog thereof and/or in an endogenous *HMT* gene or homolog thereof has an increase in Trp and/or Met content of 1.1- to 10-fold, or 1.1- to 8-fold, or 1.1- to 5-fold, or 1.1- to 2-fold, as compared to Trp and/or Met content in seed of a control plant or in a control plant product without the genetic mutation. In some embodiments, seed of a plant or a plant product comprising a genetic mutation in an endogenous *AS* gene or homolog thereof and/or in an endogenous *HMT* gene or homolog thereof has an increase in Trp and/or Met content of 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, or more as compared to Trp and/or Met content in seed of a control plant or in a control plant product without the genetic mutation.

In some embodiments, the plant, plant part, or plant product is from a legume. In certain embodiments, the legume is soybean (*Glycine max*) or pea (*Pisum sativum*)*.*

Trp and/or Met levels in seed or in a plant product can be measured by any standard methods of measuring or estimating the amount of Trp and/or Met amino acid content in a sample. For example, amino acid levels may be measured in seed by homogenizing seed in HCl (e.g., 20 mM or 0.1N), centrifuging to sediment debris, deproteinating the supernatant with a filter unit, and the filtrate diluted with HCl for analysis by HPLC. Measurement of free Trp is described in, e.g., Li and Last (1996) Plant Physiol 110: 51-59, Cho et al. (2000) Plant Physiology 123:1069-1076, Berardino et al. (1990) J Nutr Biochem 1: 220-222, and Heinrikson and Meredith (1984) Anal Biochem 136:65-74. Measurement of amino acid levels is described in, e.g., Lee et al. (2008) The Plant Journal 54:310-320. In some embodiments, plant tissue may be collected and frozen in liquid nitrogen and stored at -80°C until analysis. Amino acid concentrations may be calculated by comparing peak areas to those of standard curves prepared with commercially available pure amino acid compounds. Amino acid concentration may be expressed as concentration of an amino acid (e.g., Trp, Met) per weight of fresh or dry tissue (e.g., nmol g⁻¹ fresh wt).

### F. Plant parts and plant products

The present disclosure provides plant parts and plant products obtained from the plant of the present disclosure. A "plant part", as used herein, refers to any part of a plant, including plant cells, embryos, pollen, ovules, seeds, grains, leaves, flowers, branches, stalks, roots, root tips, anthers, plant protoplasts, plant cell tissue cultures from which plants can be regenerated, plant calli, plant clumps, stems, and branches. Vegetative tissue of a plant include any tissue of a plant that does not function in sexual reproduction of the plant. In some embodiments, vegetative tissue of a plant functions in asexual reproduction of the plant. In some embodiments, vegetative tissue of a plant includes root, stem, shoot buds, and leaves. A "plant product", as used herein, refers to any composition derived from the plant or plant part, including any oil product, fiber products, protein products (such as protein concentrate, protein isolate, flake, or other protein product), seed hulls, meal, flour, or sugar product, for a food, feed, aqua, or industrial product, plant extract (e.g., sweetener, antioxidants, alkaloids, etc.), plant concentrate (e.g., whole plant concentrate or plant part concentrate), plant powder (e.g., formulated powder, such as formulated plant part powder (e.g., seed flour)), plant biomass (e.g., dried biomass, such as crushed and/or powdered biomass), plant protein composition (e.g., soybean meal), and food and beverage products containing plant compositions (e.g., plant parts, plant extract, plant concentrate, plant powder, plant protein, and plant biomass) described herein.

Plant protein compositions of the present disclosure include a concentrated protein solution (e.g., soybean protein concentrate solution) in which the protein is in a higher concentration than the protein in the plant from which the protein composition is derived. The protein composition can comprise multiple proteins as a result of the extraction or isolation process. In some embodiments, the protein composition can further comprise stabilizers, excipients, drying agents, desiccating agents, anti-caking agents, or any other ingredient to make the protein fit for the intended purpose. The protein composition can be a solid, liquid, gel, or aerosol and can be formulated as a powder. The protein composition can be extracted in a powder form from a plant and can be processed and produced in different ways, such as: *(i) as an isolate* - through the process of wet fractionation, which has the highest protein concentration; *(ii) as a concentrate* - through the process of dry fractionation, which are lower in protein concentration; and/or *(iii) in textured form* - when it is used in food products as a substitute for other products, such as meat substitution (e.g. a "meat" patty). In some embodiments, a composition of the present disclosure includes a protein isolate prepared from grain by removing the majority of non-protein components and containing not less than 90% protein on a moisture-free basis. In some embodiments, a composition of the present disclosure includes a protein concentrate prepared from grain by removing most of the oil and water soluble non-protein constituents and containing not less than about 65% protein on a moisture-free basis. In some embodiments, the protein isolate or protein concentrate is from a legume. In certain embodiments, the legume is soybean *(Glycine max)* or pea *(Pisum sativum).*

Food and/or beverage products of the present disclosure can include plant compositions, e.g., plant protein compositions of the present disclosure. Food and/or beverage products of the present disclosure can include shakes (e.g., protein shakes), health drinks, alternative meat products (e.g., meatless burger patties, meatless sausages), alternative egg products (e.g., eggless mayo), non-dairy products (e.g., non-dairy whipped toppings, non-dairy milk, non-dairy creamer, non-dairy milk shakes, non-diary ice cream), energy bars (e.g., protein energy bars), infant formula, baby foods, cereals, baked goods, edamame, tofu, and tempeh.

Moreover, food and/or beverage products can be meant for human or animal consumption. For example, food and/or beverage products as disclosed herein can refer to products meant for consumption by agricultural animals (e.g., in animal feed). In some embodiments, food and/or beverage products include animal feed intended for consumption by bovine, swine, poultry, lambs, goats, or any other agricultural animal. Food and/or beverage products as disclosed herein can refer to feed for any type of fish or aquatic animal in a farmed or wild environment including, without limitation, trout, carp, catfish, salmon, tilapia, crab, lobster, shrimp, oysters, clams, mussels, and scallops.

Seeds of the present disclosure include a representative sample of seeds, from a plant of the present disclosure. A plant or plant part of the present disclosure can be a crop plant or part of a crop plant. In some embodiments, seeds of the present disclosure includes seeds from a legume. In certain embodiments, the legume is soybean *(Glycine max)* or pea *(Pisum sativum).*

As provided herein, the plant parts and plant products, including plant protein compositions and plant-based food/beverage products of the present disclosure includes a genetic mutation (e.g., one or more insertions, substitutions, and/or deletions) in at least one endogenous *AS* gene (e.g., *ASA* gene) or homolog thereof that alters AS activity and/or in at least one endogenous *HMT* gene or homolog thereof that decreases or eliminates HMT activity. In some embodiments, the plants and plant products are from a legume. In certain embodiments, the legume is soybean *(Glycine max)* or pea *(Pisum sativum).*

The genetic mutation can comprise at least one in-frame deletion in a *Glycine max ASA* gene or homolog thereof. In some embodiments, the genetic mutation comprises a deletion of about 3-30 nucleotides in the *Glycine max ASA1* gene or homolog thereof. In some embodiments, the genetic mutation comprises a deletion of about 6-24 nucleotides in the *Glycine max ASA1* gene or homolog thereof. In some embodiments, the genetic mutation comprises a deletion of about 6 nucleotides in the *Glycine max ASA1* gene or homolog thereof. In some embodiments, the genetic mutation comprises a deletion of about 12 nucleotides in the *Glycine max ASA1* gene or homolog thereof. In some embodiments, the genetic mutation comprises a deletion of about 24 nucleotides in the *Glycine max ASA1* gene or homolog thereof.

The genetic mutation can comprise at least one deletion in a *Glycine max HMT* gene. In some embodiments, the genetic mutation comprises a deletion of about 1-70 nucleotides in a *Glycine max HMT* gene or homolog thereof. In some embodiments, the genetic mutation comprises a deletion of about 3-66 nucleotides in a *Glycine max HMT1* gene or homolog thereof. In some embodiments, the genetic mutation comprises a deletion of about 3 nucleotides in a *Glycine max HMT1* gene or homolog thereof. In some embodiments, the genetic mutation comprises a deletion of about 66 nucleotides in a *Glycine max HMT1* gene or homolog thereof. In some embodiments, the genetic mutation comprises a deletion of about 1-30 nucleotides in a *Glycine max HMT3* gene or homolog thereof. In some embodiments, the genetic mutation comprises a deletion of about 2-13 nucleotides in a *Glycine max HMT3* gene or homolog thereof. In some embodiments, the genetic mutation comprises a deletion of about 2 nucleotides in a *Glycine max HMT3* gene or homolog thereof. In some embodiments, the genetic mutation comprises a deletion of about 13 nucleotides in a *Glycine max HMT3* gene or homolog thereof. The plant parts and plant products of the present disclosure can have altered AS activity, reduced or no expression of an *HMT* gene or homolog thereof, reduced or no expression of an HMT protein, reduced or no HMT activity, and/or increased seed Trp and/or Met content as compared to a control plant part or plant product, e.g., without the genetic mutation in an endogenous *AS* gene or homolog thereof and/or in an endogenous *HMT* gene or homolog thereof.

In some embodiments, the plant protein compositions of the present disclosure include meal. Meal refers to a feed ingredient that is a product of processing grain. Meal includes a defatted, desolventized, toasted, and ground material as described herein. In some embodiments, a composition comprising meal produced from a plant or plant part comprising a genetic mutation in at least one endogenous *AS* gene (e.g., *ASA* gene) or homolog thereof and/or in at least one endogenous *HMT* gene or homolog thereof has increased seed Trp and/or Met content as compared to a composition comprising meal produced from a plant or plant part without the genetic mutation.

In some embodiments, the meal is from a legume. In certain embodiments, the legume is soybean *(Glycine max*) or pea *(Pisum sativum).* Illustrative processes for soybean meal preparation include those taught in U.S. Patent Nos. 4,992,294; 5,225,230; 5,773,051; and 5,866,192. Typically, commercial soybean processes start with the step of receiving the soybeans from the field by any conventional transport means. The soybeans are typically received in a dirty and often wet condition and may be cleaned with a vibrating screen. In this step the soybeans are separated from non-soybean material, for example, rocks, sticks, leaves, stems, dirt, weed seeds, and unwanted fragments of soybeans. The cleaned soybeans, in combination with the loose hulls that are not removed by the vibrating screen, are transferred to an aspirator in which most of the remaining loose hulls are removed by air. The soybeans are then transferred to storage, and the removed loose hulls are collected as a by-product for further processing.

At this point in the processing, the soybeans typically contain about 12% water, but the actual water content of the soybeans may vary based on a host of different factors. If the water content of the soybeans is in excess of about 12%, then the soybeans may be subjected to a drying step to reduce the water content below about 12% prior to placing in storage. The control of the water content is essential to prevent mold and microbial contamination during storage.

The processing procedures from this point forward may vary depending upon the desired end products. For example, the soybeans may be first de-hulled using such conventional equipment as cracking rolls or hammer mills in combination with a conventional aspiration system. Alternatively, the hulls may not be removed prior to further processing. See, for example, U.S. Patent No. 5,225,230. In order to deactivate anti-nutritional factors, such as trypsin inhibitors, the soybeans may be subjected to heat for a set period of time prior to cracking, grinding, or crushing.

For cracking processes, clean, dry, whole soybeans are fed to coarsely corrugated roller mills or "crackers." These crackers can have one or more sets of rolls. Soybean pieces, called "cracks," are formed. The goal of the cracking step is to maximize the pieces that are l/4^{th} to l/8^{th} the size of the starting soybean, and to minimize the formation of fines, which are pieces less than 1 mm in diameter.

From the cracking mills, particles of whole soybeans (cracks) are conveyed to multistage aspiration de-hulling systems, which typically employ 1 to 3 stages. Each stage consists of an aspirator and a size screening system. At each stage, the fiber-rich hulls are first removed by means of a countercurrent air stream and a cyclone. The heavier, fiber-lean, meats fraction is conveyed to a screening system that removes at least one additional fraction by size, and yields one stream for further aspiration. Alternatively, screening can be employed prior to aspiration. The "hulls" stream is typically combined with other soybean byproducts and used as an animal feed ingredient. The once de-hulled meats are then de-hulled a second time to bring them to less than about 3% crude fiber (4.28% crude fiber on a defatted, dry basis) using a 2 stage commercial pre-extraction process. However, the single stage systems can also be employed to yield meats. The resulting meats are then heat conditioned, such as in a rotary or stack cooker. The residence times of the cracks are typically between about 20 and about 40 minutes. Discharge temperatures typically are in the range of about 120 to 180°F. Lower conditioning temperatures may be employed if a greater fines production in the flaker is tolerable. The conditioned meats are then fed to smooth roller mills called flakers. A force of greater than about 500 kPa-gauge (72.5 psig) is typically applied to the rolls. Flake thicknesses of less than about 0.75 mm (0.030 in.) are preferably produced in order to obtain maximum oil recovery in the subsequent oil extraction step. Optionally, the cracking and de-hulling steps are eliminated, or done subsequent to the conditioning step. An additional option would be to expand a percentage of the flaked soybeans to form "collets" prior to oil extraction. Other process variations include conditioning prior to the cracking step, and eliminating the de-hulling step prior to oil extraction.

The next step in the process of generating soybean meal is the extraction of oil. This extraction step is typically done using a lipophilic solvent, but may also be done by mechanical extraction. In this process, the soybean meal is contacted with a suitable solvent (e.g., hexane) to remove the oil to a content of typically less than about 1% by weight. One example of a conventional solvent extraction procedure is described in U.S. Patent No. 3,721,569. However, if a "full fat" soybean meal is desired, then the oil bearing meal is not subjected to oil (also known as fat or lipid) extraction.

At this stage, the solvent extracted, defatted soybean meal typically contains about 30% solvent by weight. Prior to being used as an animal feed, the meal is typically processed through a desolventizer-toaster (DT) to remove the residual solvent and to heat the protein fraction sufficient to inactivate trypsin inhibitors and other naturally occurring toxicants (anti-feedants). Typically, steam contacts the soybean meal and the heat of vaporization released from the condensing steam vaporizes the solvent, which is subsequently recovered and recycled.

As an alternative to solvent extraction, the soybean meal is defatted mechanically using, for example, a screw press. This mechanically extracted or "expeller" soybean meal typically contains between about 4 and about 8% residual oil. If the intended use of the meal is as a feed supplement for ruminants, then the meal may first be heated and dried in a specified manner, such as that taught in U.S. Patent No. 5,225,230, before oil is extracted mechanically. The defatted soybean meal is then dried and typically ground or pelletized, and then milled into a physical state suitable for use as a food supplement or as an animal feed.

Further processing of the soybean or the meal may optionally be done to make the resulting feed more palatable, available, and/or digestible in animals. These processes include addition of enzymes or nutrients, and heat treatment of the meal. Additionally, further processing may be done to the meal, such as pelleting, to make it more compact and dense in distribution. Further processing of the soybean meal can produce soybean flour, soybean protein concentrates, and soybean protein isolates that have food, feed, and industrial uses.

Soybean flours are produced simply by grinding and screening the defatted soybean meal. Soybean flours are often used in the manufacturing of meat extenders and analogs, pet foods, baking ingredients, and other food products. Food products made from soybean flour and isolate include baby food, candy products, cereals, food drinks, noodles, yeast, beer, ale, and the like.

Soybean protein concentrates, having at least about 65 wt.% protein, are made by removing soluble carbohydrate material from defatted soybean meal. Aqueous alcohol extraction (60-80% ethanol in water) or acid leaching at the isoelectric pH 4.5 of the protein are the most common methods of removing the soluble carbohydrate fraction. A myriad of applications has been developed for soybean protein concentrates and texturized concentrates in processed foods, meat, poultry, fish, cereal, and dairy systems.

Soybean protein isolates are preferably produced through standard chemical isolation, drawing the protein out of the defatted soybean flake through solubilization (alkali extraction at pH 7-10) and separation followed by isoelectric precipitation. As a result, isolates are at least about 90 wt.% protein. They are sometimes high in sodium and minerals (ash content). Their major applications have been in dairy substitution, as in infant formulas and milk replacers.

The high Trp and/or Met content meal of the present disclosure may be used in various feed formulations. In some embodiments, the high Trp and/or Met content soybean meal of the present disclosure is used in feed formulations for simple stomach animals, such as swine and poultry. In some embodiments, use of the meal of the present disclosure in feed formulations will reduce or eliminate the need to add exogenous sources of Trp and/or Met. In some embodiments, the high Trp and/or Met content meal of the present disclosure allows a formulator to use less expensive ingredients in animal feeds which lowers the feed cost for animal producers.

### IV. Increasing Seed Trp and/or Met Content in Plants

Methods are provided herein for increasing seed Trp and/or Met content in a plant or plant part. In some embodiments, the methods comprise introducing a genetic mutation in a plant or plant part to alter endogenous AS activity, reduce or eliminate endogenous HMT activity, reduce or eliminate expression of an endogenous *HMT* gene or homolog thereof, and/or reduce or eliminate expression of an HMT protein encoded by an endogenous *HMT* gene or homolog thereof. AS activity, HMT activity, and/or expression levels of *HMT* gene or its encoded protein in a plant or plant part may be modified (e.g., increased, decreased) by any methods known in the art, including the methods described herein.

Embodiments of the present disclosure provide for methods comprising introducing a genetic mutation into a plant or plant part that alters AS activity. In some embodiments, the AS activity is ASA activity. In some embodiments, the method includes introducing the genetic mutation that alters AS activity into a plant cell, and regenerating a plant or plant part from the plant cell. In some embodiments, ASA activity is altered such that the ASA protein is insensitive to or has reduced sensitivity to Trp feedback inhibition as compared to activity of an ASA protein encoded by an *ASA* gene or homolog thereof in a control plant or plant part without the genetic mutation. In some embodiments, ASA activity is increased as compared to activity of an ASA protein encoded by *an ASA* gene or homolog thereof in a control plant or plant part without the genetic mutation. The methods described herein provide for a plant or plant part comprising a genetic mutation in at least one endogenous *ASA* gene or homolog thereof that has: an encoded ASA protein that is insensitive or has reduced sensitivity to Trp feedback inhibition, an encoded ASA protein with increased activity, and/or increased seed Trp content, as compared to a control plant or plant part without the genetic mutation.

Embodiments of the present disclosure provide for methods comprising introducing a genetic mutation into a plant or plant part that reduces or eliminates HMT activity. In some embodiments, the method includes introducing the genetic mutation that reduces or eliminates HMT activity into a plant cell, and regenerating a plant or plant part from the plant cell. The methods described herein provide for a plant or plant part comprising a genetic mutation in at least one endogenous *HMT* gene or homolog thereof that has: an encoded HMT protein with reduced or no HMT activity, decreased expression level or no expression of at least one *HMT* gene or homolog thereof, decreased expression level or no expression of an HMT protein encoded by an *HMT* gene or homolog thereof, and/or increased seed Met content in the mutated plant or plant part as compared to a control plant or plant part without the genetic mutation.

In some embodiments, the plant or plant parts used in the methods of the disclosure are from a legume. In certain embodiments, the legume is soybean *(Glycine max*) or pea *(Pisum sativum).* In some embodiments, the methods described herein provide for a plant or plant part comprising increased tryptophan (Trp), methionine (Met), and/or SMM content in vegetative tissues.

### A. Introducing mutations into the genome of a plant, plant part, or plant cell

Introducing a genetic mutation (i.e. one or more genetic mutations) into a plant or plant part refers to introducing a genetic mutation into the plant's genome, e.g., into at least one endogenous *AS* gene or homolog thereof and/or at least one endogenous *HMT* gene or homolog thereof. In some embodiments, the *AS* gene or homolog thereof includes an *ASA* gene or homolog thereof. In some embodiments, the genetic mutation is introduced into a regulatory region of an endogenous *AS* gene or homolog thereof and/or an endogenous *HMT* gene or homolog thereof. Introducing a genetic mutation into a plant genome may employ precise genome-editing technologies that allow insertions, substitutions, and/or deletions of nucleotides in a nucleic acid sequence targeted for mutation in a plant, e.g., an endogenous *ASA* gene or homolog thereof and/or an endogenous *HMT* gene or homolog thereof. Such methods include, but are not limited to, use of a nuclease and/or nucleic acid guides designed to target plant target genomic sequences of interest for editing, including: a Type II CRISPR (Clustered Regularly Interspaced Short Palindromic Repeats) system (e.g., a CRISPR-Cas9 system)/; a Type V CRISPR system (e.g., a CRISPR-Cas12a (Cpfl) system); a type I, III, or IV CRISPR system (Makarova et al. (2020) Nat Rev Microbiol 18:67-83); a transcription activator-like effector nuclease (TALEN) system; and a zinc finger nuclease (ZFN) system. See, for example, D'Halluin et al. (2013) Plant Biotechnol J 11:933-941; Feng et al. (2013) Cell Research 23:1229-1232; Podevin et al. (2013) Trends Biotechnology 31: 375-383; Wei et al. (2013) J Gen Genomics 40:281-289; WO 2013/026740; Zetsche et al. (2015) Cell 163:759-771; Wright et al. (2005) Plant J 44:693-705; and Cai et al. (2009) Plant Mol Biol 69:699-709. Other genome editing technologies include: *Natronobacterium gregoryi* Argonaute-mediated DNA-guided system (Gao et al. (2016) Nat Biotechnol 34:768-773); Cre-lox site-specific recombination (Dale et al. (1995) Plant J 7:649-659; Lyznik et al. (2007) Transgenic Plant J 1:1-9); FLP-FRT recombination (Li et al. (2009) Plant Physiol 151:1087-1095); Bxb1-mediated integration (Yau et al. (2011) Plant J 701:147-166); and homologous recombination (Lieberman-Lazarovich and Levy (2011) Methods Mol Biol 701: 51-65; Puchta (2002) Plant Mol Biol 48:173-182). For example, an editing system can introduce insertions, substitutions, and/or deletions of genetic elements at a predefined genomic locus by causing a double-strand break at the predefined genomic locus and, optionally, providing an appropriate DNA template for insertion. This strategy is well-understood and has been demonstrated previously for insertion of a transgene at a predefined location in the cotton genome using a meganuclease (D'Halluin et al. (2013) Plant Biotechnol. J. 11: 933-941). Reagents and compositions that can be used for introducing one or more mutations into plants or plant parts according to the methods of the present disclosure are herein described.

### Editing systems, reagents, and genetic constructs comprising or encoding the same

Introducing genetic mutations at a target site of interest in a genome of a plant, plant part, or plant cell may be achieved by introducing into the plant, plant part, or plant cell a system (e.g., a gene editing system), reagents (e.g., editing reagents), or a genetic construct comprising or encoding such system or reagents. In some embodiments, introducing a genetic mutation includes inserting, substituting, and/or deleting one or more nucleotides at a precise location, also called a target site, in at least one endogenous *AS* gene or homolog thereof and/or in at least one endogenous *HMT* gene or homolog thereof in a plant, plant part, or plant cell. A "gene editing system", "editing system", "gene editing reagent", and "editing reagent" as used herein, refers to a set of one or more molecules or genetic constructs comprising or encoding the one or more molecules for introducing a genetic mutation in the genome. An exemplary gene editing system or editing reagents comprise a nuclease and/or a nucleic acid guide (e.g., guide RNA). Also disclosed herein is a genetic construct (e.g., a DNA construct, a recombinant DNA construct) for introducing a genetic mutation in a plant, plant part, or plant cell. In some embodiments, a genetic construct includes an editing system or polynucleotides encoding reagents of an editing system (e.g., nuclease, guide RNA, base editor), each operably linked to a promoter.

As used herein, the terms "nuclease" or "endonuclease" refers to naturally-occurring or engineered enzymes, which cleave a phosphodiester bond within a polynucleotide chain. Nucleases that can be used in precise genome-editing technologies to edit an endogenous gene (e.g., at least one endogenous *ASA* gene or homolog thereof and/or an *HMT* gene or homolog thereof) include, but are not limited to, meganucleases designed against a plant genomic sequence of interest; a CRISPR nuclease (e.g., Cas9 endonuclease, Cas12a (Cpfl) endonuclease, Cms1 endonuclease); an ortholog of a CRISPR nuclease; transcription activator-like effector nucleases (TALENs); zinc finger nucleases (ZFNs); and a deactivated CRISPR nuclease (e.g., a deactivated Cas9, Cas12a, or Cms1 endonuclease) fused to a transcriptional regulatory element (Piatek et al. (2015) Plant Biotechnol J 13:578-589). In some embodiments, the editing system or the editing reagents comprise a ZFN, a TALEN, or a CRISPR nuclease. A "TALEN" nuclease is an endonuclease comprising a DNA-binding domain comprising a plurality of TAL domain repeats fused to a nuclease domain or an active portion thereof from an endonuclease or exonuclease, including but not limited to a restriction endonuclease, homing endonuclease, and yeast HO endonuclease. A "zinc finger nuclease" or "ZFN" refers to a chimeric protein comprising a zinc finger DNA-binding domain fused to a nuclease domain from an endonuclease or exonuclease, including but not limited to a restriction endonuclease, homing endonuclease, and yeast HO endonuclease.

In some embodiments, the editing reagents comprise an RNA-guided nuclease (e.g., a CRISPR nuclease) where binding of a guide RNA to the RNA-guided nuclease forms a ribonucleoprotein (RNP) complex. The RNP complex is directed to a genomic sequence of interest for gene editing. For example, the following may occur. The RNA-guided nuclease of the RNP complex unwinds the nucleic acid duplex at the target site and optionally cleaves at least one nucleic acid strand, as mediated by recognition of a nucleic acid strand of the target site by the guide RNA. Such recognition and cutting of a target site by an RNA-guided nuclease typically occurs if a correct protospacer-adjacent motif (PAM) is located at or adjacent 5' or 3' to the target site. Alternatively, an RNA-guided nuclease herein may lack cleavage or nicking activity, but can still specifically bind to a target site when complexed with a suitable guide RNA. In some embodiments, cleavage of a target site by an RNA-guided nuclease leads to non-homologous end joining or homology-directed repair to introduce a mutation at the cleavage site. In some embodiments, the CRISPR nuclease is a Cas12a nuclease, herein used interchangeably with a Cpf1 nuclease, e.g., a McCpf1 nuclease. In some embodiments, the CRISPR nuclease is a Cas12a nuclease ortholog, e.g., *Lachnospiracea* bacterium Cas12a (Lb5Cas12a), *Candidatus Methanomethylophilus alvus* Cas12a (CMaCas12a), *Acidaminococcus* Cas12a (AsCas12a), *Butyrivibrio* sp. Cas12a (BsCas12a), *Bacteroidetes* oral taxon 274 Cas12a (BoCas12a), *Moraxella lacunata* Cas12a (MlCas12a), *Moraxella bovoculi* Cas12a (Mb2Cas12a), *Thiomicrospira* sp. Cas12a (TsCas12a), and MAD7 endonucleases (see, e.g., Zetsche et al. (2019) The Keio Journal of Medicine 69(3):59-65; Zhang et al. (2021) Nature Communications 12:1944; Teng et al. (2019) Genome 20(1):1-6). In some embodiments, an RNA-guided nuclease useful in compositions and methods of the present disclosure has a nucleotide sequence set forth as SEQ ID NO: 25. In some embodiments, an RNA-guided nuclease useful in compositions and methods of the present disclosure has an amino acid sequence set forth as SEQ ID NO: 26.

A Cas12a (Cpfl) endonuclease coupled with a guide RNA (gRNA) (i.e., a CRISPR-Cas12a system) designed to target a genomic sequence of interest (i.e., at least one endogenous *AS* gene or homolog thereof and/or at least one endogenous *HMT* gene or homolog thereof) can be used. Alternatively, a Cas9 endonuclease coupled with a gRNA (a CRISPR-Cas9 system) or a Cms1 endonuclease coupled with a gRNA (a CRISPR-Cms1 system) designed to target a genomic sequence of interest can be used. In some embodiments, a deactivated CRISPR nuclease (e.g., a deactivated Cas9, Cas12a, or Cms1 endonuclease) fused to a transcriptional regulatory element can be targeted to a regulatory region (e.g., upstream regulatory region) of at least one endogenous *AS* gene or homolog thereof and/or at least one endogenous *HMT* gene or homolog thereof, thereby modulating the transcription of the *AS* gene or homolog thereof and/or *HMT* gene or homolog thereof (Piatek et al. (2015) Plant Biotechnol J 13:578-589). Generation of site-specific genetic mutations in plant cells has been shown by biolistic introduction of a ribonucleoprotein complex comprising a nuclease and suitable guide RNA (Svitashev et al. (2016) Nat Commun 7(1):1-7).

In some embodiments, a CRISPR system includes a CRISPR nuclease (e.g., CRISPR-associated (Cas) endonuclease, variant, or ortholog thereof, such as Cas12a or Cas12a ortholog) and a guide RNA (gRNA). "Guide RNA" or "gRNA" as used herein refers to a RNA molecule that function as guides for RNA- or DNA-targeting enzymes, e.g., nucleases. A CRISPR nuclease associates with (i.e. binds to) a gRNA. In some embodiments, a guide RNA includes a CRISPR RNA (crRNA). In some embodiments, the crRNA comprises a direct repeat and a spacer. The spacer has a sequence that is fully or substantially complementary to a nucleic acid strand of a target site.

The spacer of a gRNA for use in a method described herein can be about 10-40 nucleotides long (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides long). For example, the spacer of a gRNA for use in a method described herein may be 24 nucleotides in length.

In some embodiments, a gRNA of the disclosure hybridizes to a nucleic acid region of an *ASA* gene or of an *HMT* gene. In some embodiments, a gRNA of the disclosure comprises a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to a nucleic acid sequence set forth as any one of SEQ ID NOs: 19-21. In some embodiments, the gRNA comprises a sequence having 100% sequence identity to the nucleic acid sequence set forth as any one of SEQ ID NOs: 19-21.

A gRNA of the disclosure can hybridize to a nucleic acid region of an *ASA1* gene and can comprise a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to a nucleic acid sequence set forth as SEQ ID NO: 19. In some embodiments, a gRNA of the disclosure hybridizes to a nucleic acid region of an *ASA1* gene and comprises a nucleic acid sequence having 100% sequence identity to the nucleic acid sequence set forth as SEQ ID NO: 19. In some embodiments, the *ASA1* gene has a nucleic acid sequence set forth as SEQ ID NO: 1.

A gRNA of the disclosure can hybridize to a nucleic acid region of an *HMT1* gene and can comprise a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to a nucleic acid sequence set forth as SEQ ID NO: 20. In some embodiments, a gRNA of the disclosure hybridizes to a nucleic acid region of an *HMT1* gene and comprises a nucleic acid sequence having 100% sequence identity to the nucleic acid sequence set forth as SEQ ID NO: 20. In some embodiments, the *HMT1* gene has a nucleic acid sequence set forth as SEQ ID NO: 9.

A gRNA of the disclosure can hybridize to a nucleic acid region of an *HMT3* gene and can comprise a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to a nucleic acid sequence set forth as SEQ ID NO: 21. In some embodiments, a gRNA of the disclosure hybridizes to a nucleic acid region of an *HMT3* gene and comprises a nucleic acid sequence having 100% sequence identity to the nucleic acid sequence set forth as SEQ ID NO: 21. In some embodiments, the *HMT3* gene has a nucleic acid sequence set forth as SEQ ID NO: 13.

A target site for a CRISPR system described herein refers to a nucleic acid region where a guide RNA and nuclease of a CRISPR system binds for gene editing. In some embodiments, a target site includes two nucleic acid strands, a strand having a sequence substantially complementary to the spacer, and a strand having substantial identity to the sequence of the spacer. "Substantially complementary" refers to at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.9%, or more sequence complementarity between a target site strand sequence and a guide RNA sequence. "Substantial identity" refers to at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.9%, or more sequence identity between a target site strand sequence and a guide RNA sequence. In some embodiments, a target site includes the nucleic acid strand substantially complementary to the guide RNA and/or the nucleic acid strand substantially identical to the guide RNA. In some embodiments, a target site can be about 100-300 nucleotides long.

A target site of the present disclosure can be located in an *ASA* gene or homolog thereof comprising a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to the nucleic acid sequence set forth as SEQ ID NO: 1.

A target site of the present disclosure can be located in an *HMT* gene or homolog thereof comprising a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to the nucleic acid sequence set forth as SEQ ID NO: 9.

A target site of the present disclosure can be located in an *HMT* gene or homolog thereof comprising a nucleic acid sequence having at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to the nucleic acid sequence set forth as SEQ ID NO: 13.

In some embodiments, a target site is located in a Gm *ASA1* gene and comprises a nucleic acid sequence set forth as SEQ ID NO: 22. In some embodiments, a target site is located in a Gm *HMT1* gene and comprises a nucleic acid sequence set forth as SEQ ID NO: 23. In some embodiments, a target site is located in a Gm *HMT3* gene and comprises a nucleic acid sequence set forth as SEQ ID NO: 24.

In some embodiments, a gRNA includes a region that allows the gRNA to form a complex with a nuclease (e.g., a CRISPR nuclease) of interest.

In some embodiments, a CRISPR system of the disclosure can include a tracrRNA (trans-activating CRISPR RNA) as part of the gRNA. A tracrRNA includes sequence that is complementary (fully or partially) to the sequence of the direct repeat of a crRNA.

An editing system or editing reagent can also include base editing components. In some embodiments, cytosine base editing (CBE) reagents change a C-G base pair to a T-A base pair and include a guide RNA, a nuclease (e.g., dCas9 (inactive Cas9), Cas9 nickase), a cytosine deaminase (e.g., APOBEC1), and a uracil DNA glycosylase inhibitor (UGI). In some embodiments, adenine base editing (ABE) reagents change an A-T base pair to a G-C base pair and include a deaminase (e.g., TadA), a nuclease (e.g., dCas9 or Cas9 nickase), and a guide RNA.

The gene editing system (e.g., CRISPR-Cas12a system), editing reagents, or a genetic construct of the present disclosure can include at least one polynucleotide comprising a regulatory element operably linked to a nucleic acid sequence encoding a gRNA for targeting a target site, and at least one polynucleotide comprising a regulatory element (which may be the same as or different from the gRNA regulatory element) operably linked to a nucleic acid sequence encoding an RNA-guided nuclease (e.g., CRISPR nuclease), for generation of an editing system by which the gRNA targets a target site for editing and the RNA-guided nuclease cleaves a nucleic acid sequence at the target site, and wherein the RNA-guided nuclease, and the gRNA, do not naturally occur together. In such system, the gRNA regulatory element may include an RNA polymerase II (Pol II) promoter, a single transcript unit (STU) regulatory element, a ubiquitin promoter, a U6 promoter, and/or a U3 promoter.

In some embodiments, the methods described herein include introducing into a plant, plant part, or plant cell a non-naturally occurring heterologous CRISPR-Cas12a genome editing system, wherein the CRISPR-Cas12a genome editing system introduces a genetic mutation in at least one endogenous *ASA* gene or homolog thereof and/or in at least one endogenous *HMT* gene or homolog thereof. The gene editing system (e.g., the CRISPR-Cas12a system) can target PAM sites including TTN, TTV, TTTV, NTTV, TATV, TATG, TATA, YTTN, GTTA, and/or GTTC, wherein N is A, C, G, or T/U; V is A, G, or C, but not T or U; and Y is T/U or C. In some embodiments, an RNA-guided gene editing system recognizes a PAM site of TTTA for a target site of an endogenous Gm *ASA1* gene or homolog thereof. In some embodiments, an RNA-guided gene editing system recognizes a PAM site of TTTG for a target site of an endogenous Gm *HMT1* gene or homolog thereof. In some embodiments, an RNA-guided gene editing system recognizes a PAM site of TTTG for a target site of an endogenous Gm *HMT3* gene or homolog thereof.

Such methods of introducing mutations into plants, plant parts, or plant cells may be carried out at moderate temperatures, e.g., below 25°C and above temperatures that freeze a plant or produce frost damage to the plant. The methods provided herein may be performed on a wide variety of plants.

Methods disclosed herein are not limited to certain techniques of mutagenesis. Any method of creating a change in a nucleic acid of a plant can be used in conjunction with the disclosed methods, including the use of chemical mutagens (e.g. methanesulfonate, sodium azide, aminopurine, etc.), genome/gene editing techniques (e.g. CRISPR-like technologies, TALENs, zinc finger nucleases, and meganucleases), ionizing radiation (e.g. ultraviolet and/or gamma rays) temperature alterations, long-term seed storage, tissue culture conditions, targeting induced local lesions in a genome, sequence-targeted and/or random recombinases, etc. It is anticipated that new methods of introducing a genetic mutation in a nucleic acid of a plant will be developed and yet fall within the scope of the claimed invention when used with the teachings described herein. Any editing system or editing reagents for use in any genome-editing methods including those described herein can be expressed in a plant, plant part, or plant cell.

In some embodiments, the method includes introducing into a plant, plant part, or plant cell editing reagents comprising: at least one RNA-guided nuclease, or a polynucleotide encoding the at least one RNA-guided nuclease; and at least one guide RNA (gRNA), or a polynucleotide encoding the at least one gRNA, wherein the at least one gRNA is complementary to genomic sequence of an endogenous *AS* gene or homolog thereof and/or an endogenous *HMT* gene or homolog thereof to produce a plant, plant part, or plant cell comprising a genetic mutation in the endogenous *AS* gene or homolog thereof and/or in the endogenous *HMT* gene or homolog thereof. In some embodiments, the endogenous *AS* gene or homolog thereof is an endogenous *ASA* gene or homolog thereof. In some embodiments, the introducing comprises introducing the editing reagents into the plant cell, and culturing the plant cell to regenerate a plant or plant part comprising the genetic mutation. In some embodiments, the genetic mutation comprises one or more insertions, substitutions, or deletions in the endogenous *AS* gene or homolog thereof and/or in the endogenous *HMT* gene or homolog thereof. In some embodiments, the at least one gRNA is capable of binding the at least one RNA-guided nuclease to form a ribonucleoprotein (RNP) complex. In some embodiments, the RNP complex cleaves a target site in the endogenous *AS* gene or homolog thereof and/or in the endogenous *HMT* gene or homolog thereof, and wherein the genetic mutation is introduced at the cleaved target site.

A genetic mutation that is introduced into a plant, plant part, or plant cell according to the methods provided herein can comprise one or more (e.g., about 1, 2, 3, 4, 5, 6, 7, 8, 9, 10, 11, 12, 13, 14, 15, 16, 17, 18, 19, 20, or more) nucleotide insertions, substitutions, and/or deletions in at least one endogenous *AS* gene (e.g., *ASA1* gene) or homolog thereof and/or in at least one endogenous *HMT* gene or homolog thereof in a genome of a plant, plant part, or plant cell.

In some embodiments, a plant, plant part, or plant cell to which a genetic mutation is introduced according to the methods provided herein can comprise at least 2 *ASA* genes, at least 3 *ASA* genes, or at least 4 *ASA* genes, each gene encoding an ASA protein and having less than 100% (e.g., less than 100%, 99%, 98%, 97%, 96%, 95%, 94%, 93%, 92%, 91%, 90%, 89%, 88%, 87%, 86%, or 85%) sequence identity to one another. A plant, plant part, or plant cell to which a genetic mutation is introduced according to the methods provided herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions: in one *ASA* gene or homolog thereof; in a regulatory region of one *ASA* gene or homolog thereof; in more than one (e.g., 2 or 3), but not all *ASA* genes or homologs thereof; in regulatory regions of more than one (e.g., 2 or 3), but not all *ASA* genes or homologs thereof; in all *ASA* genes or homologs thereof; and/or in regulatory regions of all *ASA* genes or homologs thereof in the plant or plant part.

In some embodiments, a genetic mutation that alters HMT activity is introduced according to the methods provided herein in at least 2 *HMT* genes or at least 3 *HMT* genes, each gene encoding an HMT protein and having less than 100% (e.g., less than 100%, 99%, 98%, 97%, 96%, 95%, 94%, 93%, 92%, 91%, 90%, 89%, 88%, 87%, 86%, or 85%) sequence identity to one another. A plant, plant part, or plant cell to which a genetic mutation is introduced according to the methods provided herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions: in one *HMT* gene or homolog thereof; in a regulatory region of one *HMT* gene or homolog thereof; in more than one (e.g., 2 or 3), but not all *HMT* genes or homologs thereof; or in regulatory regions of more than one (e.g., 2 or 3), but not all *HMT* genes or homologs thereof; in the plant or plant part.

In some embodiments, a plant, plant part, or plant cell to which a genetic mutation is introduced includes a plant, plant part, or plant cell from a legume. In certain embodiments, the legume is soybean *(Glycine max)* or pea *(Pisum sativum).* A genetic mutation that alters AS activity is introduced according to the methods provided herein in one or more (e.g., one, more than one but not all, or all) *Glycine max ASA* genes, including a *Glycine max ASA1* gene, a *Glycine max ASA2* gene, a *Glycine max ASA3* gene, and a *Glycine max ASA4* gene. In some embodiments, the mutation is located in a *Glycine max ASA1* gene. In some embodiments, a soybean plant, plant part, or plant cell to which a genetic mutation is introduced according to the methods provided herein can include 1, 2, 3, or 4 *ASA* genes, e.g., *ASA1* (Glyma.18G028900), *ASA2* (Glyma.18G028800), *ASA3* (Glyma.20G103200), and *ASA4* (Glyma.10G286100) genes, each encoding an ASA protein.

In some embodiments, a genetic mutation that alters AS activity is introduced according to the methods provided herein in an *ASA1* gene or homolog thereof comprising a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence set forth as SEQ ID NO: 1 and encoding an active ASA1, for example the nucleic acid sequence set forth as SEQ ID NO: 1. In some embodiments, the mutation that alters AS activity is introduced according to the methods provided herein in an *ASA1* gene or homolog thereof encoding an ASA1 comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence set forth as SEQ ID NO: 2, wherein the ASA1 is active, for example an ASA1 comprising the amino acid sequence set forth as SEQ ID NO: 2.

Embodiments of the present disclosure provide for methods that introduce a genetic mutation comprising at least one (e.g., about 1, 2, 3, 4, 5, 6, 7, 8, 9, 10, 11, 12, 13, 14, 15, 16, 17, 18, 19, 20, or more) in-frame deletion in an *ASA* gene. In some embodiments, the genetic mutation is located in an enzyme regulatory region of the encoded ASA. In some embodiments, the enzyme regulatory region comprises a region that binds Trp. The methods provided herein include introducing a deletion of about 3-30 nucleotides in an *ASA* gene or homolog thereof. In some embodiments, the methods include introducing a deletion of about 6-24 nucleotides in an *ASA* gene or homolog thereof. In some embodiments, the methods include introducing a deletion of 6 nucleotides in an *ASA* gene or homolog thereof. In some embodiments, the methods include introducing a deletion of 12 nucleotides in an *ASA* gene or homolog thereof. In some embodiments, the methods include introducing a deletion of 24 nucleotides in an *ASA* gene or homolog thereof. In some embodiments, an *ASA1* gene comprising the genetic mutation encodes an ASA1 protein having an amino acid sequence set forth as any one of SEQ ID NOs: 6, 7, and 8. In some embodiments, an ASA1 protein having an amino acid sequence set forth as any one of SEQ ID NOs: 6, 7, and 8 is insensitive to or has reduced sensitivity to Trp feedback inhibition as compared to an ASA1 protein encoded by the corresponding *ASA1* gene or homolog thereof without the genetic mutation.

A genetic mutation that decreases or eliminates HMT activity is introduced according to the methods provided herein in one or more (e.g., one, more than one but not all, or all) *Glycine max HMT* genes, including a *Glycine max HMT1* gene, a *Glycine max HMT2* gene, a *Glycine max HMT3* gene, and a *Glycine max HMT4* gene. In some embodiments, the mutation is located in a *Glycine max HMT1* gene. In some embodiments, the mutation is located in a *Glycine max HMT3* gene. In some embodiments, a genetic mutation that decreases or eliminates HMT activity is introduced according to the methods provided herein in 1, 2, 3, and/or 4 *HMT* genes, e.g., *HMT1* (Glyma.19G158800), *HMT2* (Glyma.03G156500), *HMT3* (Glyma.20G148900), and/or *HMT4* (Glyma.08G261200) genes, each encoding an HMT protein.

In some embodiments, a genetic mutation that decreases or eliminates HMT activity is introduced according to the methods provided herein in an *HMT1* gene or homolog thereof comprising a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence set forth as SEQ ID NO: 9 and encoding an active HMT1, for example the nucleic acid sequence set forth as SEQ ID NO: 9. In some embodiments, a genetic mutation that decreases or eliminates HMT activity is introduced according to the methods provided herein in an *HMT1* gene or homolog thereof encoding an HMT1 comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence set forth as SEQ ID NO: 10, wherein the HMT1 is active, for example an HMT1 comprising the amino acid sequence set forth as SEQ ID NO: 10.

In some embodiments, a genetic mutation that decreases or eliminates HMT activity is introduced according to the methods provided herein in an *HMT3* gene or homolog thereof comprising a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence set forth as SEQ ID NO: 13 and encoding an active HMT3, for example the nucleic acid sequence set forth as SEQ ID NO: 13. In some embodiments, a genetic mutation that decreases or eliminates HMT activity is introduced according to the methods provided herein in an *HMT3* gene or homolog thereof encoding an HMT3 comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the amino acid sequence set forth as SEQ ID NO: 14, wherein the HMT3 is active, for example an HMT3 comprising the amino acid sequence set forth as SEQ ID NO: 14.

Embodiments of the present disclosure provide for methods that introduce a genetic mutation comprising at least one (e.g., about 1, 2, 3, 4, 5, 6, 7, 8, 9, 10, 11, 12, 13, 14, 15, 16, 17, 18, 19, 20, or more) deletion in an *HMT* gene. The methods provided herein include introducing a deletion of about 1-70 nucleotides in an *HMT* gene or homolog thereof. In some embodiments, a deletion of 3-66 nucleotides is introduced into an *HMT1* gene or homolog thereof. In some embodiments, a deletion of 3 nucleotides is introduced into an *HMT1* gene or homolog thereof. In some embodiments, a deletion of 66 nucleotides is introduced into an *HMT1* gene or homolog thereof. In some embodiments, a deletion of 1-30 nucleotides is introduced into an *HMT3* gene or homolog thereof. In some embodiments, a deletion of 2-13 nucleotides is introduced into an *HMT3* gene or homolog thereof. In some embodiments, a deletion of 2 nucleotides is introduced into an *HMT3* gene or homolog thereof. In some embodiments, a deletion of 13 nucleotides is introduced into an *HMT3* gene or homolog thereof.

The methods of the present disclosure can produce a soybean plant or plant part comprising: (i) an in-frame deletion of nucleotides 952 to 957 of *ASA1* having the nucleic acid sequence set forth as SEQ ID NO: 1, or a nucleic acid sequence comprising SEQ ID NO: 3 when the deletion of (i) is introduced; (ii) an in-frame deletion of nucleotides 948 to 959 of *ASA1* having the nucleic acid sequence set forth as SEQ ID NO: 1, or a nucleic acid sequence comprising SEQ ID NO: 4 when the deletion of (ii) is introduced; (iii) an in-frame deletion of nucleotides 938 to 961 of *ASA1* having the nucleic acid sequence set forth as SEQ ID NO: 1, or a nucleic acid sequence comprising SEQ ID NO: 5 when the deletion of (iii) is introduced; (iv) a deletion of nucleotides 712 to 714 of *HMT1* having the nucleic acid sequence set forth as SEQ ID NO: 9, or a nucleic acid sequence comprising SEQ ID NO: 11 when the deletion of (iv) is introduced; (v) a deletion of nucleotides 695 to 760 of *HMT1* having the nucleic acid sequence set forth as SEQ ID NO: 9, or a nucleic acid sequence comprising SEQ ID NO: 12 when the deletion of (v) is introduced; (vi) a deletion of nucleotides 2783 to 2784 of *HMT3* having the nucleic acid sequence set forth as SEQ ID NO: 13, or a nucleic acid sequence comprising SEQ ID NO: 15 when the deletion of (vi) is introduced; or (vii) a deletion of nucleotides 2778 to 2790 of *HMT3* having the nucleic acid sequence set forth as SEQ ID NO: 13, or a nucleic acid sequence comprising SEQ ID NO: 16 when the deletion of (vii) is introduced.

The methods described herein can comprise introducing a mutation that alters ASA and/or HMT activity, e.g., by introducing one or more (e.g., about 1, 2, 3, 4, 5, 6, 7, 8, 9, 10, 11, 12, 13, 14, 15, 16, 17, 18, 19, 20, or more) insertions, substitutions, and/or deletions in a regulatory region of at least one (e.g., one, more than one but not all, or all) *AS* gene or homolog thereof and/or at least one *HMT* gene or homolog thereof. In some embodiments, one or more insertions, substitutions, and/or deletions can be introduced into a promoter, a transcription modulator protein (e.g., transcription factor) binding site, or other regulatory regions of at least one (e.g., one, more than one but not all, or all) *AS* gene or homolog thereof and/or at least one *HMT* gene or homolog thereof to confer to the plant or plant part an altered (e.g., increased, decreased) transcription activity of an *AS* gene or homolog thereof and/or an *HMT* gene or homolog thereof.

In some embodiments, the methods provided herein include introducing a genetic mutation in a promoter of at least one (e.g., one, more than one but not all, or all) *AS* gene or homolog thereof and/or at least one *HMT* gene or homolog thereof. The one or more insertions, substitutions, and/or deletions in the promoter of an *AS* gene or homolog thereof and/or an *HMT* gene or homolog thereof can alter the transcription initiation activity of the promoter. For example, the modified promoter can enhance or reduce transcription of the operably linked nucleic acid molecule (e.g., an *ASA* and/or *HMT* gene), initiate transcription in a developmentally-regulated or temporally-regulated manner, initiate transcription in a cell-specific, cell-preferred, tissue-specific, or tissue-preferred manner, or initiate transcription in an inducible manner. A deletion, a substitution, or an insertion, e.g., introduction of a heterologous promoter sequence, a cis-acting factor, a motif or a partial sequence from any promoter, including those described elsewhere in the present disclosure, can be introduced into the promoter of an *AS* gene or homolog thereof and/or an *HMT* gene or homolog thereof to confer an altered (e.g., increased, decreased) transcription initiation function according to the present disclosure.

In some embodiments, promoter activity of an *AS* gene is enhanced by inserting, deleting, and/or substituting one or more nucleotides (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more nucleotides). In some embodiments, promoter activity of an *HMT* gene is reduced or inactivated by inserting, deleting, and/or substituting one or more nucleotides (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more nucleotides). The substitution can be a cisgenic substitution, a transgenic substitution, or both.

In some embodiments, promoter activity of an *AS* gene or homolog thereof and/or an *HMT* gene or homolog thereof is altered (e.g., enhanced, reduced) by addition, insertion, and/or engineering of cis-acting factors that interact with and modify the promoter. In some embodiments, the methods provided herein include introducing a genetic mutation in a gene encoding (or regulating expression of) a transcription factor such that expression or function of the transcription factor is modulated, leading to altered (e.g., enhanced, reduced) expression of an *AS* gene or homolog thereof and/or an *HMT* gene or homolog thereof. In some embodiments, the mutation in a gene encoding (or regulating expression of) a transcription factor increases expression levels of an *AS* gene, e.g., by enhancing transcription initiation activity of the *AS* gene promoter. In some embodiments, the mutation in a gene encoding (or regulating expression of) a transcription factor increases activity of the AS protein encoded by the *AS* gene. In some embodiments, the mutation in the gene encoding (or regulating expression of) a transcription factor reduces expression levels of an *HMT* gene, e.g., by inhibiting transcription initiation activity of the *HMT* gene promoter. In some embodiments, the mutation in a gene encoding (or regulating expression of) a transcription factor reduces activity of the HMT protein encoded by the *HMT* gene. In some embodiments, the methods provided herein include introducing a genetic mutation that modifies or inserts transcription factor binding sites or enhancer elements that regulates *AS* and/or *HMT* gene expression into the regulatory region of the *AS* and/or *HMT* gene.

In some embodiments, the methods provided herein include introducing a part or whole of one or more negative regulatory sequences of an *HMT* gene or homolog thereof into the genome of a plant cell or plant part. The negative regulatory sequence of the *HMT* gene or homolog thereof can be in a cis location or in a trans location. Negative regulatory sequences of an *HMT* gene or homolog thereof can also include upstream open reading frames (uORFs). In some embodiments, the methods provided herein include introducing a negative regulatory sequence upstream of an *HMT* gene or homolog thereof to inhibit expression and/or function of the *HMT* gene or homolog thereof. In some embodiments, the methods provided herein include introducing a part or whole of one or more positive regulatory sequences of an *AS* gene (e.g., *ASA* gene) or homolog thereof into the genome of a plant cell or plant part. The positive regulatory sequence of the *AS* gene or homolog thereof can be in a cis location or in a trans location. Positive regulatory sequences of an *AS* gene or homolog thereof can also include upstream open reading frames (uORFs). In some embodiments, the methods provided herein include introducing a positive regulatory sequence upstream of an *AS* gene or homolog thereof to enhance expression and/or function of the *AS* gene.

The methods provided herein include introducing into a plant, plant part, or plant cell a genetic mutation in a homolog or variant of an *AS* gene and/or an *HMT* gene disclosed herein and encodes an active AS protein and/or an active HMT protein, or in a regulatory region of such homolog or variant of an *AS* gene and/or an *HMT* gene. Variant sequences (e.g., homologs) can be isolated by methods known in the art and described herein. In certain instances, mutations in an *AS* gene and/or an *HMT* gene in a plant, plant part, or plant product (e.g., plant protein composition) can be identified by a diagnostic method described herein. In some embodiments, the methods are applied to a plant, plant part, or plant cell from a legume. In certain embodiments, the legume is soybean *(Glycine max)* or pea *(Pisum sativum).*

### B. Altering activity of anthranilate synthase (AS) and/or decreasing activity of homocysteine S-methyltransferase (HMT) in a plant, plant part, or plant cell

The methods of the present disclosure produce a plant, plant part, or plant cell comprising a genetic mutation that: (i) alters AS activity, and/or (ii) reduces or eliminates HMT activity as compared to AS and/or HMT activity in a control plant, plant part, or plant cell.

In some embodiments, the methods of the present disclosure comprise introducing a genetic mutation in at least one endogenous *ASA* gene or homolog thereof in a plant, plant part, or plant cell, wherein the mutated endogenous *ASA* gene or homolog thereof encodes an ASA protein that is insensitive to or has reduced sensitivity to Trp feedback inhibition as compared to an ASA protein encoded by the corresponding *ASA* gene in a control plant, plant part, or plant cell without the genetic mutation. Sensitivity of an ASA enzyme to Trp feedback inhibition can be assessed as described herein. In some embodiments, the Kᵢ Trp value for a mutated ASA enzyme of the disclosure is 2- to 200-fold greater, or 2- to 100-fold greater, or 2- to 50-fold greater as compared to the Kᵢ Trp value for a control AS enzyme. In some embodiments, the Kᵢ Trp value for a mutated ASA enzyme of the disclosure is 2-fold, 3-fold, 4-fold, 5-fold, 6-fold, 7-fold, 8-fold, 9-fold, 10-fold, 15-fold, 20-fold, 25-fold, 30-fold, 35-fold, 40-fold, 45-fold, 50-fold, 55-fold, 60-fold, 65-fold, 70-fold, 75-fold, 80-fold, 85-fold, 90-fold, 95-fold, 100-fold, 110-fold, 120-fold, 130-fold, 140-fold, 150-fold, 160-fold, 170-fold, 180-fold, 190-fold, 200-fold, or greater, as compared to the Kᵢ Trp value for a control ASA enzyme.

In some embodiments, the methods of the present disclosure comprise introducing a genetic mutation in at least one endogenous *ASA* gene or homolog thereof in a plant, plant part, or plant cell, wherein the mutated endogenous *AS* gene (e.g., *ASA* gene) or homolog thereof encodes an AS protein having 5-100%, 10-100%, 20-100%, 30-100%, 40-100%, 50-100%, 60-100%, 70-100%, 80-100%, 20-90%, 30-90%, 40-90%, 50-90%, 60-90%, 70-90%, 10-20%, 20-30%, 30-40%, 40-50%, 50-60%, 60-70%, 70-80%, 80-90%, or 90-100%, or having 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, or more increase in activity as compared to activity of an AS protein encoded by the corresponding *AS* gene or homolog thereof without the genetic mutation in a control plant, plant part, or plant cell. AS activity may be measured as described herein. In some embodiments, the *AS* gene or homolog thereof includes a *Glycine max ASA1* gene (Glyma. 18G028900), a *Glycine max ASA2* gene (Glyma.18G028800), a *Glycine max ASA3* gene (Glyma.20G103200), or a *Glycine max ASA4* gene (Glyma. 10G286100). In some embodiments, the *ASA* gene or homolog thereof includes a *Glycine max ASA1* gene (Glyma.18G028900).

In some embodiments, the methods of the present disclosure comprise introducing a genetic mutation in at least one endogenous *HMT* gene or homolog thereof, wherein the mutated endogenous *HMT* gene or homolog thereof encodes an HMT protein having 5-100%, 10-100%, 20-100%, 30-100%, 40-100%, 50-100%, 60-100%, 70-100%, 80-100%, 20-90%, 30-90%, 40-90%, 50-90%, 60-90%, 70-90%, 10-20%, 20-30%, 30-40%, 40-50%, 50-60%, 60-70%, 70-80%, 80-90%, or 90-100%, or having 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% reduced activity as compared to activity of an HMT protein encoded by the corresponding *HMT* gene or homolog thereof without the genetic mutation in a control plant, plant part, or plant cell. In some embodiments, the methods of the present disclosure comprise introducing a genetic mutation in at least one endogenous *HMT* gene or homolog thereof, wherein the mutated endogenous *HMT* gene or homolog thereof encodes an HMT protein having no activity. HMT activity may be measured as described herein. In some embodiments, the *HMT* gene or homolog thereof includes a *Glycine max HMT1* gene (Glyma.19G158800), a *Glycine max HMT2* gene (Glyma.03G156500), a *Glycine max HMT3* gene (Glyma.20G148900), or a *Glycine max HMT4* gene (Glyma.08G261200). In some embodiments, the *HMT* gene or homolog thereof includes a *Glycine max HMT1* gene (Glyma.19G158800). In some embodiments, the *HMT* gene or homolog thereof includes a *Glycine max HMT3* gene (Glyma.20G148900).

In some embodiments, the methods are applied to a plant, plant part, or plant cell from a legume. In certain embodiments, the legume is soybean *(Glycine max)* or pea *(Pisum sativum).*

### C. Reducing or eliminating expression of HMT gene and/or HMT protein

The methods of the present disclosure produce a plant, plant part, or plant cell comprising a genetic mutation in at least one endogenous *HMT* gene or homolog thereof that has reduced or no expression of the endogenous mutated *HMT* gene or homolog thereof as compared to expression of the corresponding *HMT* gene or homolog thereof without the genetic mutation in a control plant, plant part, or plant cell. In some embodiments, the methods provided herein reduce expression of the mutated endogenous *HMT* gene or homolog thereof by 10-100%, 20-100%, 30-100%, 40-100%, 50-100%, 60-100%, 70-100%, 80-100%, 20-90%, 30-90%, 40-90%, 50-90%, 60-90%, 70-90%, 10-20%, 20-30%, 30-40%, 40-50%, 50-60%, 60-70%, 70-80%, 80-90%, or 90-100%, or by 10%, 15%, 20%, 25%, 30%, 35%, 40%, 45%, 50%, 55%, 60%, 65%, 70%, 75%, 80%, 85%, 90%, 95%, or 100%, or at least 10%, 15%, 20%, 25%, 30%, 35%, 40%, 45%, 50%, 55%, 60%, 65%, 70%, 75%, 80%, 85%, 90%, 95%, or 100%, as compared to the expression of the corresponding *HMT* gene or homolog thereof without the genetic mutation in a control plant, plant part, or plant cell. In some embodiments, the methods provided herein produce a plant, plant part, or plant cell comprising a mutated endogenous *HMT* gene or homolog thereof that is not expressed. In some embodiments, the *HMT* gene or homolog thereof includes a *Glycine max HMT1* gene (Glyma.19G158800), a *Glycine max HMT2* gene (Glyma.03G156500), a *Glycine max HMT3* gene (Glyma.20G148900), or a *Glycine max HMT4* gene (Glyma.08G261200). In some embodiments, the *HMT* gene or homolog thereof includes a *Glycine max HMT1* gene (Glyma.19G158800). In some embodiments, the *HMT* gene or homolog thereof includes a *Glycine max HMT3* gene (Glyma.20G148900).

The methods of the present disclosure produce a plant, plant part, or plant cell comprising a genetic mutation in at least one endogenous *HMT* gene or homolog thereof that has reduced or no expression of the HMT protein encoded by the mutated endogenous *HMT* gene or homolog thereof, as compared to the expression level of an HMT protein encoded by the corresponding *HMT* gene or homolog thereof without the genetic mutation in a control plant, plant part, or plant cell. In some embodiments, the methods provided herein reduce expression of an HMT protein encoded by a mutated endogenous *HMT* gene or homolog thereof by 10-100%, 20-100%, 30-100%, 40-100%, 50-100%, 60-100%, 70-100%, 80-100%, 20-90%, 30-90%, 40-90%, 50-90%, 60-90%, 70-90%, 10-20%, 20-30%, 30-40%, 40-50%, 50-60%, 60-70%, 70-80%, 80-90%, or 90-100%, or by 10%, 15%, 20%, 25%, 30%, 35%, 40%, 45%, 50%, 55%, 60%, 65%, 70%, 75%, 80%, 85%, 90%, 95%, or 100%, or at least 10%, 15%, 20%, 25%, 30%, 35%, 40%, 45%, 50%, 55%, 60%, 65%, 70%, 75%, 80%, 85%, 90%, 95%, or 100%, as compared to the expression level of an HMT protein encoded by the corresponding *HMT* gene or homolog thereof without the genetic mutation in a control plant, plant part, or plant cell. In some embodiments, the methods provided herein produce plant, plant part, or plant cell comprising an HMT protein lacking expression.

In some embodiments, the HMT protein is encoded by an *HMT* gene or homolog thereof including a *Glycine max HMT1* gene (Glyma. 19G158800), a *Glycine max HMT2* gene (Glyma.03G156500), a *Glycine max HMT3* gene (Glyma.20G148900), and a *Glycine max HMT4* gene (Glyma.08G261200). In some embodiments, the HMT protein is encoded by an *HMT gene* or homolog thereof including a *Glycine max HMT1* gene (Glyma.19G158800). In some embodiments, the HMT protein is encoded by an *HMT* gene or homolog thereof including a *Glycine max HMT3* gene (Glyma.20G148900).

Expression levels of an *HMT* gene or homolog thereof or expression levels of an HMT protein can be measured by any standard methods known in the art and as described herein.

In some embodiments, the methods are applied to a plant, plant part, or plant cell from a legume. In certain embodiments, the legume is soybean *(Glycine max*) or pea *(Pisum sativum*)*.*

### D. Increasing seed Trp and/or Met content in plants

Provided herein are compositions and methods for increasing Trp and/or Met amino acid content produced by a plant by introducing a genetic mutation in an endogenous *AS* gene or homolog thereof encoding an anthranilate synthase and/or in an endogenous *HMT* gene or homolog thereof encoding a homocysteine S-methyltransferase. The plant or plant part comprising the genetic mutation can have increased seed Trp and/or Met content as compared to seed of a control plant or plant part without the genetic mutation. The methods of the present disclosure generate a plant or plant part comprising a genetic mutation wherein Trp and/or Met content in seed produced from the plant or plant part is 1.1-fold to 10-fold greater than that of control seed. In some embodiments, control seed comprises seed produced by a plant, plant part, or a population of plants without the genetic mutation.

In some embodiments, the methods provided herein produce a plant, plant part, plant cell, or population of plants comprising a genetic mutation in an endogenous *AS* gene or homolog thereof and/or in an endogenous *HMT* gene or homolog thereof, wherein seed produced from the plant, plant part, plant cell, or population of plants comprising the genetic mutation has an increase in Trp and/or Met content of 10-500%, 20-450%, 30-400%, 40-300%, 50-200%, 60-200%, 70-200%, 80-200%, 20-100%, 30-100%, 40-100%, 50-100%, 60-100%, 70-100%, 10-20%, 20-30%, 30-40%, 40-50%, 50-60%, 60-70%, 70-80%, 80-90%, 90-100%, 100-120%, 120-140%, 140-160%, 160-180%, 180-200%, 200-220%, 220-240%, 240-260%, 260-280%, 280-300%, 300-320%, 320-340%, 340-360%, 360-380%, 380-400%, 400-420%, 420-440%, 440-460%, 460-480%, or 480-500%, or of 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, or at least 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, or 500%, or more as compared to Trp and/or Met content in seed of a control plant, plant part, plant cell, or population of plants without the genetic mutation.

In some embodiments, the methods provided herein produce a plant, plant part, plant cell, or population of plants comprising a genetic mutation in an endogenous *AS* gene or homolog thereof and/or in an endogenous *HMT* gene or homolog thereof, wherein seed produced from the plant, plant part, plant cell, or population of plants comprising the genetic mutation has an increase in Trp and/or Met content of 1.1- to 10-fold, 1.1- to 8-fold, 1.1- to 5-fold, 1.1- to 2-fold, or 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, or more as compared to Trp and/or Met content in seed of a control plant, plant part, plant cell, or population of plants without the genetic mutation. Trp and/or Met levels in seed or in a plant product can be measured by any standard methods known to one of skill in the art and as described herein.

In some embodiments, the methods are applied to a plant, plant part, or plant cell from a legume. In certain embodiments, the legume is soybean *(Glycine max*) or pea *(Pisum sativum).* In some embodiments, the methods produce a plant or plant part comprising increased tryptophan (Trp), methionine (Met), and/or SMM content in vegetative tissues.

### E. Plants, plant parts, and plant products produced by methods of the disclosure

The present disclosure provides plants, plant parts, and plant products produced according to the methods provided herein. In some embodiments, such plants, plant parts, and plant products includes a genetic mutation (e.g., one or more insertions, substitutions, and/or deletions) in at least one endogenous *AS* gene (e.g., *ASA* gene) or homolog thereof and/or in at least one endogenous *HMT* gene or homolog thereof, as compared to a control plant, plant part, or plant product. In some embodiments, plants, plant parts, and plant products produced according to the methods provided herein comprise altered AS activity, reduced or no expression of an *HMT* gene or homolog thereof, reduced or no expression of an HMT protein, reduced or no HMT activity, and/or increased seed Trp and/or Met content, as compared to a control plant, plant part, or plant product. In some embodiments, plant products include any composition derived from the plant or plant part, including plant extract, plant concentrate, plant powder, plant biomass, grains, plant protein composition, and food and beverage products as described herein. In some embodiments, plant products include meal with a high content of Trp and/or Met as described herein. In some embodiments, the plants, plant parts, and plant products are from a legume. In certain embodiments, the legume is soybean *(Glycine max)* or pea *(Pisum sativum).* In some embodiments, the plants or plant parts comprise increased tryptophan (Trp), methionine (Met), and/or SMM content in vegetative tissues.

### F. Transformation of plants

Provided herein are methods for transforming plants or plant parts by introducing into the plants or plant parts a genetic mutation (e.g., one or more insertions, substitutions, and/or deletions) in at least one endogenous *AS* gene (e.g., *ASA* gene) or homolog thereof and/or in at least one endogenous *HMT* gene or homolog thereof. The methods can comprise introducing a system (e.g., a gene editing system), reagents (e.g., editing reagents), or a genetic construct including or encoding editing reagents for introducing mutations at a target site of interest.

The term "transform" or "transformation" as used herein refers to any method used to introduce genetic mutations (e.g., insertions, substitutions, and/or deletions) in a genome, or to introduce polypeptides, or polynucleotides into plant cells. For purpose of the present disclosure, the transformation can be "stable transformation", wherein the genetic mutation (e.g., in at least one endogenous *AS* gene, e.g., *ASA* gene, or homolog thereof and/or in at least one endogenous *HMT* gene or homolog thereof) or the transformation constructs (e.g., a genetic construct comprising a polynucleotide encoding a gRNA, a polynucleotide comprising a gRNA, and/or a polynucleotide encoding a nuclease for use in the methods of the present disclosure) are introduced into a host (e.g., a host plant, plant part, plant cell, etc.), integrate into the genome of the host, and are capable of being inherited by the progeny thereof; or "transient transformation", wherein the genetic mutation (e.g., in at least one endogenous *AS* gene, e.g., *ASA* gene, or homolog thereof and/or in at least one endogenous *HMT* gene or homolog thereof) or the transformation constructs (e.g., a genetic construct comprising a polynucleotide encoding a gRNA, a polynucleotide comprising a gRNA, and/or a polynucleotide encoding a nuclease for use in the methods of the present disclosure) are introduced into a host (e.g., a host plant, plant part, plant cell, etc.) and expressed temporarily. The methods disclosed herein can also be used for insertion of heterologous genes and/or modification of endogenous plant gene expression to achieve desirable plant traits, e.g., increased seed Trp and/or Met content.

Any mutation, polynucleotide of interest (e.g., encoding a nuclease and/or a guide RNA), or editing reagent or system, can be introduced into a plant cell, organelle, or plant embryo by a variety of means of transformation, including microinjection (Crossway et al. (1986) Biotechniques 4:320-334), electroporation (Riggs et al. (1986) Proc. Natl. Acad. Sci. USA 83:5602-5606, *Agrobacterium-mediated* transformation (U.S. Patent No. 5,563,055 and U.S. Patent No. 5,981,840), direct gene transfer (Paszkowski et al. (1984) EMBO J. 3:2717-2722), ballistic particle acceleration (see, for example, U.S. Patent Nos. 4,945,050; 5,879,918; 5,886,244; and 5,932,782; Tomes et al. (1995) in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); U.S. Patent Nos. 5,240,855; 5,322,783; and, 5,324,646; Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; U.S. Patent No. 5,736,369 (cereals); Bytebier et al. (1987) Proc. Natl. Acad. Sci. USA 84:5345-5349 (*Liliaceae*); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*); all of which are herein incorporated by reference.

*Agrobacterium*-and biolistic-mediated transformation remain the two predominantly employed approaches. However, transformation may be performed by infection, transfection, microinjection, electroporation, microprojection, biolistics or particle bombardment, silica/carbon fibers, ultrasound mediated, PEG mediated, calcium phosphate co-precipitation, polycation DMSO technique, DEAE dextran procedure, viral infection, *Agrobacterium* and viral mediated (Caulimoriviruses, Geminiviruses, RNA plant viruses), liposome mediated and the like. Methods disclosed herein are not limited to any size of nucleic acid sequences that are introduced, and thus one could introduce a nucleic acid comprising a single nucleotide (e.g. an insertion) into a nucleic acid of the plant and still be within the teachings described herein. Nucleic acids introduced in substantially any useful form, for example, on supernumerary chromosomes (e.g. B chromosomes), plasmids, vector constructs, additional genomic chromosomes (e.g. substitution lines), and other forms is also anticipated. It is envisioned that new methods of introducing nucleic acids into plants and new forms or structures of nucleic acids will be discovered and yet fall within the scope of the claimed invention when used with the teachings described herein.

More than one polynucleotide of interest and/or editing reagent can be introduced into a plant, plant cell, plant organelle, or plant embryo simultaneously or sequentially. For example, different editing reagents, e.g., nuclease polypeptides (or nucleic acid molecule encoding such), guide RNAs (or DNA molecules encoding the guide RNAs), donor polynucleotide(s), and/or repair templates can be introduced into a plant, a plant cell, plant organelle, or plant embryo simultaneously or sequentially. The amount or ratio of more than one polynucleotide of interest, and/or molecules encoded therein, can be adjusted by adjusting the amount or concentration of the polynucleotides and/or timing and dosage of introducing the polynucleotides into the plant, plant cell, plant organelle, or plant embryo. For example, the ratio of the nuclease (or nucleic acid molecule encoding such) to the guide RNA(s) (or DNA molecule encoding such) to be introduced into plants, plant cells, plant organelles, or plant embryos generally will be about stoichiometric such that the two components can form an RNA-protein complex that can bind a target site. In some embodiments, a nucleic acid molecule encoding a nuclease and a DNA molecule encoding a guide RNA are delivered together in a plasmid vector.

Alteration of expression and/or activity of AS and/or HMT in plants, plant parts, or plant cells may also be achieved through the use of transposable element technologies. It is well understood that transposable elements can alter the expression of nearby DNA (McGinnis et al. (1983) Cell 34:75-84). Thus, in some embodiments, alteration of AS and/or HMT gene or protein expression level or enzyme activity is achieved by inserting a transposable element into at least one endogenous *AS* gene (e.g., *ASA* gene) or homolog thereof and/or at least one endogenous *HMT* gene or homolog thereof.

The cells that have been transformed may be grown into plants (i.e., cultured) in accordance with conventional protocols. See, for example, McCormick et al. (1986) Plant Cell Reports 5:81-84. In this manner, the present disclosure provides transformed plants or plant parts, transformed seed (also referred to as "transgenic seed") or transformed plant progenies having a nucleic acid modification stably incorporated into their genome.

The present invention may be used for transformation of any plant species, e.g., both monocots and dicots (including legumes). Plants or plant parts to be transformed according to the methods disclosed herein can be a legume, i.e., a plant belonging to the family Fabaceae (or Leguminosae), or a part (e.g., fruit or seed) of such a plant. When used as a dry grain, the seed of a legume is also called a pulse. Examples of legume include, without limitation, soybean *(Glycine max),* beans (*Phaseolus* spp.), common bean (*Phaseolus vulgaris*), fava bean (*Vicia faba*), mung bean (*Vigna radiata*), cowpea (*Vigna unguiculata*), adzuki bean (*Vigna angularis*), pea *(Pisum sativum),* chickpea (*Cicer arietinum*), peanut (*Arachis hypogaea*), lentils (*Lens culinaris, Lens esculenta*), lupins *(Lupinus* spp.), white lupin (*Lupinus albus),* mesquite *(Prosopis* spp.), carob (*Ceratonia siliqua*), tamarind *(Tamarindus indica*), alfalfa (*Medicago sativa*), barrel medic (*Medicago truncatula),* birdsfood trefoil (*Lotus japonicus*), licorice (*Glycyrrhiza glabra*), and clover (*Trifolium* spp.). In some embodiments, a plant or plant part to be transformed according to the methods of the present disclosure is *Glycine max* or a part of *Glycine max.* Additionally, a plant or plant part to be transformed according to the methods of the present disclosure can be a crop plant or part of a crop plant, including legumes. Examples of crop plants include, but are not limited to, corn (*Zea mays), Brassica* sp. (e.g., *B. napus, B. rapa, B. juncea),* particularly those *Brassica* species useful as sources of seed oil, alfalfa *(Medicago sativa),* rice (*Oryza sativa*), rye (*Secale cereale*), sorghum (*Sorghum bicolor, Sorghum vulgare*), camelina (*Camelina sativa*), millet (e.g., pearl millet (*Pennisetum glaucum*), proso millet (*Panicum miliaceum),* foxtail millet (*Setaria italica*), finger millet *(Eleusine coracana*)), sunflower *(Helianthus annuus),* quinoa *(Chenopodium quinoa),* chicory *(Cichorium intybus),* lettuce *(Lactuca sativa),* safflower *(Carthamus tinctorius),* wheat *(Triticum aestivum),* soybean *(Glycine max),* tobacco *(Nicotiana* spp., e.g., *Nicotiana tabacum, Nicotiana sylvestris),* potato *(Solanum tuberosum),* tomato *(Solanum lycopersicum),* peanuts *(Arachis hypogaea*), cotton *(Gossypium barbadense, Gossypium hirsutum),* sweet potato *(Ipomoea batatus),* cassava *(Manihot esculenta*), coffee (*Coffea* spp.), coconut *(Cocos nucifera*), pineapple *(Ananas comosus),* citrus trees *(Citrus* spp.), cocoa *(Theobroma cacao),* tea *(Camellia sinensis),* banana *(Musa* spp.), avocado *(Persea americana),* fig *(Ficus casica*), guava *(Psidium guajava*), mango *(Mangifera indica),* grapes (*Vitis vinifera, Vitis riparia*), olive *(Olea europaea),* papaya (*Carica papaya*), cashew *(Anacardium occidentale),* macadamia *(Macadamia integrifolia*), almond *(Prunus amygdalus*), sugar beets *(Beta vulgaris),* sugarcane *(Saccharum* spp.), oil palm (*Elaeis guineensis),* poplar (*Populus* spp.), pea *(Pisum sativum),* eucalyptus (*Eucalyptus* spp.), oats (*Avena sativa),* barley (*Hordeum vulgare),* vegetables, ornamentals, and conifers. Additionally, a plant or plant part of the present disclosure can be an oilseed plant (e.g., canola (*Brassica napus),* cotton (*Gossypium* sp.), camelina (*Camelina sativa*) and sunflower (*Helianthus* sp.)), or other species including wheat (*Triticum* sp., such as *Triticum aestivum* L. ssp. *aestivum* (common or bread wheat), other subspecies of *Triticum aestivum, Triticum turgidum* L. ssp. durum (durum wheat, also known as macaroni or hard wheat), *Triticum monococcum* L. ssp. *monococcum* (cultivated einkorn or small spelt), *Triticum timopheevi* ssp. *timopheevi, Triticum turgigum* L. ssp. dicoccon (cultivated emmer), and other subspecies of *Triticum turgidum* (Feldman)), barley (*Hordeum vulgare*), maize (*Zea mays),* oats *(Avena sativa),* or hemp *(Cannabis sativa).*

The embodiments disclosed herein are not limited to certain methods of introducing nucleic acids into a plant and are not limited to certain forms or structures that the introduced nucleic acids take. Any method of transforming a cell of a plant described herein with mutations, polynucleotides, or polypeptides are also incorporated into the teachings of this innovation. For example, one of ordinary skill in the art will realize that the use of particle bombardment (e.g. using a gene-gun), *Agrobacterium* infection and/or infection by other bacterial species capable of transferring DNA into plants (e.g., *Ochrobactrum* sp., *Ensifer* sp., *Rhizobium* sp.), viral infection, and other techniques can be used to deliver mutations, polynucleotides, or polypeptides into a plant, plant part, or plant cell described herein.

The present disclosure provides plants and plant parts transformed according to the methods of the present disclosure. Transformed plant parts of the disclosure include plant cells, plant protoplasts, plant cell tissue cultures from which plants can be regenerated, plant calli, plant clumps, and plant cells that are intact in plants or parts of plants such as embryos, pollen, ovules, seeds, grains, leaves, flowers, branches, fruit, kernels, ears, cobs, husks, stalks, roots, root tips, anthers, and the like. Progeny, variants, and mutants of the regenerated plants are also included within the scope of the disclosure, provided that these parts comprise the introduced mutations, polynucleotides, or polypeptides.

### G. Breeding of Plants

Also disclosed herein are methods for breeding a plant, such as a plant which includes (i) a genetic mutation in at least one endogenous *AS* gene (e.g., *ASA* gene) or homolog thereof and/or in at least one endogenous *HMT* gene or homolog thereof, (ii) editing reagents, e.g., a polynucleotide encoding a guide RNA specific to at least one endogenous *AS* gene (e.g., *ASA* gene) or homolog thereof and/or in at least one endogenous *HMT* gene or homolog thereof, and/or (iii) a nucleic acid molecule comprising a mutated *ASA* gene or homolog thereof. A plant comprising a genetic mutation or a nucleic acid molecule of the present disclosure may be regenerated from a plant cell or plant part, wherein the genome of the plant cell or plant part has been modified by transformation to include a genetic mutation or a nucleic acid molecule of the present disclosure. Using conventional breeding techniques or self-pollination, one or more seeds may be produced from the plant that contains the genetic mutation or the nucleic acid molecule of the present disclosure. Such a seed, and the resulting progeny plant grown from such a seed, may contain the genetic mutation or the nucleic acid molecule of the present disclosure. In some embodiments, a plant or plant part of the disclosure may be transgenic. Progeny plants are plants comprising a genetic mutation or a nucleic acid molecule of the present disclosure, which descended from an original plant that was modified (e.g., by transformation) to have the genetic mutation or the nucleic acid molecule of the present disclosure. Seeds produced using such a plant of the disclosure can be harvested and used to grow successive generations of plants having the genetic mutation or the nucleic acid molecule of the present disclosure.

Descriptions of breeding methods that are commonly used for different crops can be found in one of several reference books, see, e.g., Allard, Principles of Plant Breeding, John Wiley & Sons, NY, U. of CA, Davis, Calif., 50-98 (1960); Simmonds, Principles of Crop Improvement, Longman, Inc., NY, 369-399 (1979); Sneep and Hendriksen, Plant breeding Perspectives, Wageningen (ed), Center for Agricultural Publishing and Documentation (1979); Fehr, Soybeans: Improvement, Production and Uses, 2nd Edition, Monograph, 16:249 (1987); Fehr, Principles of Variety Development, Theory and Technique, (Vol. 1) and Crop Species Soybean (Vol. 2), Iowa State Univ., Macmillan Pub. Co., NY, 360-376 (1987).

Methods disclosed herein include conferring desired traits (e.g., increased seed Trp and/or Met content) to plants, for example, by mutating sequences of a plant, introducing nucleic acids into plants, using plant breeding techniques and various crossing schemes, etc. These methods are not limited as to certain mechanisms of how the plant exhibits and/or expresses the desired trait. In some embodiments, the trait is conferred to the plant by introducing a nucleic acid sequence (e.g. using plant transformation methods) that encodes production of a certain protein by the plant. In some embodiments, the desired trait is conferred to a plant by causing a null mutation in the plant's genome (e.g., when the desired trait is reduced expression or no expression of a certain gene). In some embodiments, the desired trait is conferred to a plant by crossing two plants to create offspring that express the desired trait. It is expected that users of these teachings will employ a broad range of techniques and mechanisms known to bring about the expression of a desired trait in a plant. Thus, as used herein, conferring a desired trait to a plant is meant to include any process that causes a plant to exhibit a desired trait, regardless of the specific techniques employed.

In some embodiments, a user can combine the teachings herein with high-density molecular marker profiles spanning substantially the entire genome of a plant to estimate the value of selecting certain candidates in a breeding program in a process commonly known as genome selection.

### V. Nucleic Acid Molecules, Constructs, and Cells Comprising Mutated ASA gene

### A. Nucleic acid molecules

Nucleic acid molecules are provided herein comprising a mutated anthranilate synthase alpha subunit (*ASA*) gene or homolog thereof that alters AS activity in a plant or plant part. In some embodiments, the mutated endogenous *ASA* gene or homolog thereof encodes an ASA protein that is insensitive to or has reduced sensitivity to Trp feedback inhibition as compared to an ASA protein encoded by the corresponding *ASA* gene or homolog thereof without the genetic mutation. In some embodiments, the mutated endogenous *ASA* gene or homolog thereof encodes an ASA protein having increased activity as compared to activity of an ASA protein encoded by the corresponding *ASA* gene or homolog thereof without the genetic mutation. The nucleic acid molecule can comprise any nucleic acid sequence that alters ASA activity (as described herein) in a plant or plant part. Such nucleic acid molecules may be present in, or obtained from a plant cell, plant part, or plant of the present disclosure, or may be obtained by the methods described herein, e.g., by introducing a genetic mutation in a native *ASA* gene or homolog thereof and/or by introducing editing reagents targeting a target site of interest in at least one endogenous *ASA* gene or homolog thereof in a plant or plant part. The nucleic acid molecule described herein can encode an altered (e.g., containing an in-frame deletion) ASA protein that has an amino acid sequence different from that of an ASA protein encoded by an *ASA* gene or homolog thereof without the genetic mutation (e.g., an ASA protein encoded by a native *ASA* gene or homolog thereof). The nucleic acid molecule described herein can encode an ASA protein that is insensitive to or has reduced sensitivity to Trp feedback inhibition and/or has increased activity, as compared to an ASA protein encoded by an *ASA* gene or homolog thereof without the genetic mutation (e.g., an ASA protein encoded by a native *ASA* gene or homolog thereof).

The nucleic acid molecule provided herein can encode an ASA protein and comprise a genetic mutation (e.g., about 1, 2, 3, 4, 5, 6, 7, 8, 9, 10, 11, 12, 13, 14, 15, 16, 17, 18, 19, 20, or more insertions, substitutions, and/or deletions) in an *ASA* gene or homolog thereof as compared to the corresponding *ASA* gene or homolog thereof without the genetic mutation. In some embodiments, the nucleic acid molecule includes an in-frame deletion in the *ASA* gene or homolog thereof.

In some embodiments, a nucleic acid molecule comprises a mutated *ASA* gene or homolog thereof from a legume. In certain embodiments, the legume is soybean (*Glycine max*) or pea *(Pisum sativum).* A nucleic acid molecule comprising a *Glycine max ASA* gene or homolog thereof that can be mutated includes a *Glycine max ASA1* gene (Glyma.18G028900), a *Glycine max ASA2* gene (Glyma.18G028800), a *Glycine max ASA3* gene (Glyma.20G103200), and a *Glycine max ASA4* gene (Glyma.10G286100). In some embodiments, the genetic mutation is located in a *Glycine max ASA1* gene. In some embodiments, a nucleic acid molecule of the present disclosure comprises a nucleic acid sequence of a mutated *ASA* gene, wherein the mutated *ASA* gene comprises one or more insertions, substitutions, or deletions as compared to the corresponding *ASA* gene without the mutations, wherein the nucleic acid sequence: (i) has at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the nucleic acid sequence set forth as SEQ ID NO: 1 and encodes an active ASA, for example the nucleic acid sequence set forth as SEQ ID NO: 1; (ii) comprises the nucleic acid sequence set forth as SEQ ID NO: 1; (iii) encodes an ASA comprising an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence set forth as SEQ ID NO: 2, wherein the ASA is active, for example a polypeptide comprising the amino acid sequence set forth as SEQ ID NO: 2; and/or (iv) encodes an ASA comprising an amino acid sequence set forth as SEQ ID NO: 2.

The genetic mutation in a nucleic acid molecule provided herein includes one or more (e.g., about 1, 2, 3, 4, 5, 6, 7, 8, 9, 10, 11, 12, 13, 14, 15, 16, 17, 18, 19, 20, or more) insertion, substitution, and/or deletion located in an *ASA* gene. The genetic mutation in a nucleic acid molecule provided herein can comprise a deletion of about 3-30 nucleotides in an *ASA* gene or homolog thereof. In some embodiments, a nucleic acid molecule provided herein comprises a deletion of 6-24 nucleotides in an *ASA* gene or homolog thereof. In some embodiments, a nucleic acid molecule provided herein comprises a deletion of 6 nucleotides in an *ASA* gene or homolog thereof. In some embodiments, a nucleic acid molecule provided herein comprises a deletion of 12 nucleotides in an *ASA* gene or homolog thereof. In some embodiments, a nucleic acid molecule provided herein comprises a deletion of 24 nucleotides in an *ASA* gene or homolog thereof. In some embodiments, a nucleic acid molecule provided herein comprising a nucleic acid sequence of a mutated *ASA1* gene encodes an ASA1 protein having an amino acid sequence set forth as any one of SEQ ID NOs: 6, 7, and 8. In some embodiments, an ASA1 protein having an amino acid sequence set forth as any one of SEQ ID NOs: 6, 7, and 8 is insensitive to or has reduced sensitivity to Trp feedback inhibition as compared to an ASA1 protein encoded by the corresponding *ASA1* gene or homolog thereof without the genetic mutation.

In some embodiments, the nucleic acid molecule of the present disclosure can comprise: (i) an in-frame deletion of nucleotides 952 to 957 of *ASA1* having the nucleic acid sequence set forth as SEQ ID NO: 1, or a nucleic acid sequence comprising SEQ ID NO: 3 when the deletion of (i) is introduced; (ii) an in-frame deletion of nucleotides 948 to 959 of *ASA1* having the nucleic acid sequence set forth as SEQ ID NO: 1, or a nucleic acid sequence comprising SEQ ID NO: 4 when the deletion of (ii) is introduced; or (iii) an in-frame deletion of nucleotides 938 to 961 of *ASA1* having the nucleic acid sequence set forth as SEQ ID NO: 1, or a nucleic acid sequence comprising SEQ ID NO: 5 when the deletion of (iii) is introduced.

### B. Genetic constructs, vectors, and cells

The nucleic acid molecules encoding molecules of interest of the present disclosure can be assembled within a genetic construct operably linked to a promoter. When transiently or stably transformed with such genetic construct, a plant, plant part, or plant cell can express an ASA protein encoded by a mutated *ASA* gene or homolog thereof contained in a nucleic acid molecule of the genetic construct. For example, the nucleic acid molecules described herein can be provided in expression cassettes or expression constructs operably linked to a promoter of interest, typically a heterologous promoter, for expression in a plant. By "heterologous promoter" is intended a nucleic acid sequence that is not naturally operably linked with the nucleic acid molecule of interest. For instance, a 2x35s cauliflower mosaic virus (CaMV) promoter, the promoter of a native *ASA* gene or homolog thereof, or a promoter (native or heterologous) comprising an exogenous or synthetic motif sequence may be operably linked to a nucleic acid molecule comprising a mutated *ASA* gene or homolog thereof. The ASA-encoding nucleic acid molecule and the promoter may each be homologous, native, heterologous, or foreign to the plant host. It is recognized that a heterologous promoter may also drive expression of its homologous or native nucleic acid sequence. In this case, the transformed plant will have a change in phenotype.

### Promoter

As used herein, "promoter" refers to a regulatory region of DNA that is capable of driving expression of a sequence in a plant or plant cell. A number of promoters may be used in the practice of the disclosure, e.g., to express editing reagents in plants, plant parts, or plant cells. The promoter may have a constitutive expression profile. Constitutive promoters include the CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like.

In some embodiments, promoters for use in the methods of the present disclosure include seed-specific and seed-preferred promoters. Seed-specific and seed-preferred promoters include, e.g., napin (Hitz et al., Kader, J.-C. and Mazliak, P., Eds. (Kluwer, Dordecht, Netherlands), p. 534 (1995) and U.S. Patent No. 5,608,152); phaseolin (U.S. Patent No. 5,504,200 and Ahm et al. (1995) Plant Phys 109:1151-1158); and storage protein gene promoters (Chen et al. (1989) Dev. Genet., 10(2):112-122; Keddie et al. (1992) Plant Mol. Biol., 19(3):443-453; Sjodahl et al. (1995) Planta., 197(2):264-271; Reidt et al. (2000) Plant J., 21(5):401-408).

In some embodiments, promoters for use in the methods of the present disclosure include tissue-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Leaf-preferred promoters are also known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6): 1129-1138; and Matsuoka et al. (1993) Proc. Natl. Acad. Sci. USA 90(20):9586-9590.

In some embodiments, promoters for use in the methods of the present disclosure include developmentally-regulated promoters. Such promoters may show a peak in expression at a particular developmental stage. Such promoters have been described in the art, e.g., U.S. Patent No. 10,407,670; Gan and Amasino (1995) Science 270: 1986-1988; Rinehart et al. (1996) Plant Physiol 112: 1331-1341; Gray-Mitsumune et al. (1999) Plant Mol Biol 39: 657-669; Beaudoin and Rothstein (1997) Plant Mol Biol 33: 835-846; Genschik et al. (1994) Gene 148: 195-202, and the like.

In some embodiments, promoters for use in the methods of the present disclosure include promoters that are induced following the application of a particular biotic and/or abiotic stress. Such promoters have been described in the art, e.g., Yi et al. (2010) Planta 232: 743-754; Yamaguchi-Shinozaki and Shinozaki (1993) Mol Gen Genet 236: 331-340; U.S. Patent No. 7,674,952; Rerksiri et al. (2013) Sci World J 2013: Article ID 397401; Khurana et al. (2013) PLoS One 8: e54418; Tao et al. (2015) Plant Mol Biol Rep 33: 200-208, and the like.

In some embodiments, promoters for use in the methods of the present disclosure include cell-preferred promoters. Such promoters may preferentially drive the expression of a downstream gene in a particular cell type such as a mesophyll or a bundle sheath cell. Such cell-preferred promoters have been described in the art, e.g., Viret et al. (1994) Proc Natl Acad USA 91: 8577-8581; U.S. Patent No. 8,455,718; U.S. Patent No. 7,642,347; Sattarzadeh et al. (2010) Plant Biotechnol J 8: 112-125; Engelmann et al. (2008) Plant Physiol 146: 1773-1785; Matsuoka et al. (1994) Plant J 6: 311-319, and the like.

It is recognized that a specific, non-constitutive expression profile may provide an improved plant phenotype relative to constitutive expression of a gene or genes of interest. For instance, many plant genes are regulated by light conditions, the application of particular stresses, the circadian cycle, or the stage of a plant's development. These expression profiles may be important for the function of the gene or gene product *in planta.* One strategy that may be used to provide a desired expression profile is the use of synthetic promoters containing cis-regulatory elements that drive the desired expression levels at the desired time and place in the plant. Cis-regulatory elements that can be used to alter gene expression *in planta* have been described in the scientific literature (e.g., Vandepoele et al. (2009) Plant Physiol 150: 535-546; Rushton et al. (2002) Plant Cell 14: 749-762). Cis-regulatory elements may also be used to alter promoter expression profiles, as described in Venter (2007) Trends Plant Sci 12: 118-124.

### Transfer DNA

Nucleic acid molecules comprising transfer DNA (T-DNA) sequences can be used in the practice of the disclosure, e.g., to express editing reagents in plants, plant parts, or plant cells. For example, a genetic construct of the present disclosure may contain T-DNA of tumor-inducing (Ti) plasmid of *Agrobacterium tumefaciens.* Alternatively, a recombinant genetic construct of the present disclosure may contain T-DNA of tumor-inducing (Ti) plasmid of *Agrobacterium rhizogenes.* The *vir* genes of the Ti plasmid may help in transfer of T-DNA of a recombinant genetic construct into nuclear DNA genome of a host plant. For example, Ti plasmid of *Agrobacterium tumefaciens* may help in transfer of T-DNA of a recombinant genetic construct of the present disclosure into nuclear DNA genome of a host plant, thus enabling the transfer of a nucleic acid molecule encoding a gRNA of the present disclosure into nuclear DNA genome of a host plant (e.g., a soybean plant).

### Regulatory signal

A genetic construct of the disclosure may include regulatory signals, including, but not limited to, transcriptional initiation sites, operators, activators, enhancers, other regulatory elements, ribosomal binding sites, an initiation codon, termination signals, and the like. See, for example, U.S. Patent Nos. 5,039,523 and 4,853,331; EPO 0480762A2; Sambrook et al. (1992) Molecular Cloning: A Laboratory Manual, ed. Maniatis et al. (Cold Spring Harbor Laboratory Press, Cold Spring Harbor, N.Y.); Davis et al., eds. (1980) Advanced Bacterial Genetics (Cold Spring Harbor Laboratory Press), Cold Spring Harbor, N.Y., and the references cited therein.

### Reporter genes / selectable marker genes

Reporter genes or selectable marker genes may be included in genetic constructs of the present disclosure. Examples of suitable reporter genes known in the art can be found in, for example, Jefferson et al. (1991) in Plant Molecular Biology Manual, ed. Gelvin et al. (Kluwer Academic Publishers), pp. 1-33; DeWet et al. (1987) Mol. Cell. Biol. 7:725-737; Goff et al. (1990) EMBO J. 9:2517-2522; Kain et al. (1995) Bio Techniques 19:650-655; and Chiu et al. (1996) Current Biology 6:325-330, herein incorporated by reference in their entirety.

Selectable marker genes include genes encoding antibiotic resistance, such as those encoding neomycin phosphotransferase II (NEO), spectinomycin/streptinomycin resistance (SpcR, AAD), and hygromycin phosphotransferase (HPT or HGR) as well as genes conferring resistance to herbicidal compounds. Herbicide resistance genes generally code for a modified target protein insensitive to the herbicide or for an enzyme that degrades or detoxifies the herbicide in the plant before it can act. For example, resistance to glyphosate has been obtained by using genes coding for mutant target enzymes, 5-enolpyruvylshikimate-3-phosphate synthase (EPSPS). Genes and mutants for EPSPS are well known, and further described below. Resistance to glufosinate ammonium, bromoxynil, and 2,4-dichlorophenoxyacetate (2,4-D) have been obtained by using bacterial genes encoding PAT or DSM-2, a nitrilase, an AAD-1, or an AAD-12, each of which are examples of proteins that detoxify their respective herbicides.

Herbicides such as imidazolinone or sulfonylurea can inhibit the growing point or meristem, and genes that confer resistance/tolerance to these herbicides are well known, including acetohydroxyacid synthase (AHAS) and acetolactate synthase (ALS). Glyphosate resistance genes include mutant 5-enolpyruvylshikimate-3-phosphate synthase (EPSPs) and dgt-28 genes (via the introduction of recombinant nucleic acids and/or various forms of *in vivo* mutagenesis of native EPSPs genes), aroA genes and glyphosate acetyl transferase (GAT) genes, respectively). Resistance genes for other phosphono compounds include bar and pat genes from *Streptomyces* species, including *Streptomyces hygroscopicus* and *Streptomyces viridichromogenes,* and pyridinoxy or phenoxy proprionic acids and cyclohexones (ACCase inhibitor-encoding genes). Exemplary genes conferring resistance to cyclohexanediones and/or aryloxyphenoxypropanoic acid (including haloxyfop, diclofop, fenoxyprop, fluazifop, quizalofop) include genes of acetyl coenzyme A carboxylase (ACCase); Accl-S1, Accl-S2 and Accl-S3. Herbicides can also inhibit photosynthesis, including triazine (psbA and 1s+ genes) or benzonitrile (nitrilase gene). In some embodiments, selectable markers can include positive selection markers such as phosphomannose isomerase (PMI) enzyme.

Selectable marker genes can further include, but are not limited to genes encoding: 2,4-D; SpcR; neomycin phosphotransferase II; cyanamide hydratase; aspartate kinase; dihydrodipicolinate synthase; tryptophan decarboxylase; dihydrodipicolinate synthase and desensitized aspartate kinase; bar gene; tryptophan decarboxylase; neomycin phosphotransferase (NEO); hygromycin phosphotransferase (HPT or HYG); dihydrofolate reductase (DHFR); phosphinothricin acetyltransferase; 2,2-dichloropropionic acid dehalogenase; acetohydroxyacid synthase; 5-enolpyruvyl-shikimate-phosphate synthase (aroA); haloarylnitrilase; acetyl-coenzyme A carboxylase; dihydropteroate synthase (sul I); and 32 kD photosystem II polypeptide (psbA).

Examples of suitable selectable marker genes include, but are not limited to, genes encoding resistance to chloramphenicol (Herrera Estrella et al. (1983) EMBO J. 2:987-992); methotrexate (Herrera Estrella et al. (1983) Nature 303:209-213 and Meijer et al. (1991) Plant Mol. Biol. 16:807-820); hygromycin (Waldron et al. (1985) Plant Mol. Biol. 5:103-108 and Zhijian et al. (1995) Plant Science 108:219-227); streptomycin (Jones et al. (1987) Mol. Gen. Genet. 210:86-91); spectinomycin (Bretagne-Sagnard et al. (1996) Transgenic Res. 5:131-137); bleomycin (Hille et al. (1990) Plant Mol. Biol. 7:171-176); sulfonamide (Guerineau et al. (1990) Plant Mol. Biol. 15:127-36); bromoxynil (Stalker et al. (1988) Science 242:419-423); glyphosate (Shaw et al. (1986) Science 233:478-481 and US Patent Application Nos. 10/004,357 and 10/427,692); phosphinothricin (DeBlock et al. (1987) EMBO J. 6:2513-2518), herein incorporated by reference in their entirety.

Other selectable marker genes that could be used in genetic constructs disclosed herein include, but are not limited to, GUS (beta-glucuronidase; Jefferson (1987) Plant Mol. Biol. Rep. 5:387); GFP (green fluorescence protein; Chalfie et al. (1994) Science 263:802); luciferase (Riggs et al. (1987) Nucleic Acids Res. 15(19):8115 and Luehrsen et al. (1992) Methods Enzymol. 216:397-414); red fluorescent protein (DsRFP, RFP, etc); beta-galactosidase; maize genes encoding for saponin production (Ludwig et al. (1990) Science 247:449); and the like (See Sambrook et al. Molecular Cloning: A Laboratory Manual, Third Edition, Cold Spring Harbor Press, N.Y., 2001), herein incorporated by reference in their entirety. The above list of selectable marker genes is not meant to be limiting. Any reporter or selectable marker gene is encompassed by the present disclosure.

### Terminator

A transcription terminator may also be included in genetic constructs of the present disclosure. Plant terminators are known in the art and include those available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acids Res. 15:9627-9639.

### Vector

Disclosed herein are vectors containing genetic constructs (e.g., recombinant DNA constructs encoding editing reagents) of the present disclosure. As used herein, "vector" refers to a nucleic acid molecule (e.g., a plasmid, cosmid), bacterial phage, or virus for introducing a genetic construct, for example, a recombinant DNA construct, into a host cell. Cloning vectors typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance and are further described herein. In some embodiments, a genetic construct or an expression cassette comprising a nucleic acid molecule of the present disclosure is located on a vector.

In some embodiments, a vector is a plasmid containing a recombinant DNA construct of the present disclosure. For example, the present disclosure may provide a plasmid containing a recombinant DNA construct that comprises or encodes a gRNA for editing of a target site comprising an *ASA* gene or homolog thereof.

In some embodiments, a vector is a recombinant virus containing a recombinant DNA construct of the present disclosure. For example, the present disclosure may provide a recombinant virus containing a recombinant DNA construct that comprises or encodes a gRNA for editing of a target site comprising *an ASA* gene or homolog thereof. A recombinant virus described herein can be a recombinant lentivirus, a recombinant retrovirus, a recombinant cucumber mosaic virus (CMV), a recombinant tobacco mosaic virus (TMV), a recombinant cauliflower mosaic virus (CaMV), a recombinant odontoglossum ringspot virus (ORSV), a recombinant tomato mosaic virus (ToMV), a recombinant bamboo mosaic virus (BaMV), a recombinant cowpea mosaic virus (CPMV), a recombinant potato virus X (PVX), a recombinant Bean yellow dwarf virus (BeYDV), or a recombinant turnip vein-clearing virus (TVCV).

### Cells

Provided herein are cells comprising a nucleic acid molecule or a genetic construct of the present disclosure. In some embodiments, the nucleic acid molecule or the genetic construct comprises a mutated nucleic acid sequence of an *ASA* gene or homolog thereof. In some embodiments, the nucleic acid molecule or the genetic construct comprises an editing reagent (e.g., a nuclease, a gRNA), a gene editing system, and/or a genetic construct for genome editing of a plant, plant part, or plant cell. A cell of the disclosure includes a plant cell, a bacterial cell, a fungal cell, and an insect cell. In some embodiments, the plant cell is from a legume. In certain embodiments, the legume is soybean *(Glycine max)* or pea *(Pisum sativum).* In some embodiments, the cell is a bacterium, e.g., an *Agrobacterium tumefaciens,* containing a gRNA targeting at least one endogenous *ASA* gene or homolog thereof and/or at least one endogenous *HMT* gene or homolog thereof to introduce mutations at a target site of interest. The cells of the present disclosure may be grown, or may have been grown, in a cell culture.

Also provided herein is a plant cell including a genetic mutation in at least one endogenous *ASA* gene or homolog thereof and/or in at least one endogenous *HMT* gene or homolog thereof. In some embodiments, the plant cell including the genetic mutation comprises an editing reagent (e.g., a nuclease, a gRNA), a gene editing system, and/or a genetic construct of the disclosure. In some embodiments, the plant cell including the genetic mutation no longer comprises an editing reagent (e.g., a nuclease, a gRNA), a gene editing system, and/or a genetic construct of the disclosure after the genetic mutation has been introduced. In some embodiments, the plant cell is from a legume. In certain embodiments, the legume is soybean *(Glycine max*) or pea *(Pisum sativum).*

Methods to generate cells of the disclosure are described herein.

It will be readily apparent to those skilled in the art that other suitable modifications and adaptations of the methods of the invention described herein are obvious and may be made using suitable equivalents without departing from the scope of the invention or the embodiments disclosed herein. Having now described the invention in detail, the same will be more clearly understood by reference to the following examples, which are included for purposes of illustration only and are not intended to be limiting. Unless otherwise noted, all parts and percentages are by dry weight.

### EXAMPLES

### EXAMPLE 1: Generation of gene editing reagents to produce ASA genes comprising genetic mutations

Guide RNAs targeting *ASA1* were designed to eliminate a conserved regulatory region within the enzyme. Optimized gRNA design to maximize activity and minimize off-target effects of CRISPR- Cas9 and CRISPR-Cas12a has been described (Nat Biotechnol 34, 184-191, doi: 10.1038/nbt.3437 (2016)). The CRISPR-Cas12a system of the present disclosure can be employed for targeting PAM sites such as TTN, TTV, TTTV, NTTV, TATV, TATG, TATA, YTTN, GTTA, and GTTC, utilizing corresponding gRNAs. In particular, a CRISPR-Cas12a system used to edit a *Glycine max ASA1* gene recognizes a PAM site of TTTA. A guide RNA targeting *GmASA1* gene includes the nucleic acid sequence set forth as SEQ ID NO: 19, and a target genomic site having the nucleic acid sequence set forth as SEQ ID NO: 22.

### EXAMPLE 2: Generation of gene editing reagents to produce HMT genes comprising genetic mutations

Guide RNAs targeting *HMT1* and *HMT3* were designed according to standard methods of the art (Zetsche et al., Cell, Volume 163, Issue 3, Pages 759-771, 2015; Cui et al., Interdisciplinary Sciences: Computational Life Sciences, volume 10, pages 455-465, 2018). Optimized gRNA design to maximize activity and minimize off-target effects of CRISPR-Cas9 and CRISPR-Cas12a has been described (Nat Biotechnol 34, 184-191, doi: 10.1038/nbt.3437 (2016)). The CRISPR-Cas12a system of the present disclosure can be employed for targeting PAM sites such as TTN, TTV, TTTV, NTTV, TATV, TATG, TATA, YTTN, GTTA, and GTTC, utilizing corresponding gRNAs. In particular, a CRISPR-Cas12a system used to edit a *Glycine max HMT1* gene or a *Glycine max HMT3* gene recognizes a PAM site of TTTG. Guide RNAs targeting *GmHMT1* and *GmHMT3* genes include nucleic acid sequences set forth as SEQ ID NO: 20 or 21, and target genomic sites having nucleic acid sequences set forth as SEQ ID NOs: 23 or 24, respectively.

### EXAMPLE 3: Genetic modification of a GmASA and/or GmHMT gene or its regulatory region and screening for expression or function

Soybean protoplasts are transformed with constructs comprising: a guide RNA targeting a genomic site in the Glyma.18G028900 *GmASAl* gene, the Glyma.19G158800 *GmHMT1* gene, or the Glyma.20G148900 *GmHMT3* gene; and a nuclease, using *Agrobacterium* transformation. Amplicons are produced near the target sites, and are sequenced to detect mutations. A mutated read is recorded for any sequence with more than two reads containing a deletion at the predicted cleavage site. Editing efficiency is calculated based on the percentage of mutated reads to total aligned reads using next generation sequencing (NGS).

A number of mutants having mutations in: the Glyma. 18G028900 *GmASAl* gene, the Glyma. 19G158800 *GmHMT1* gene, or the Glyma.20G148900 *GmHMT3* gene, or the regulatory region (e.g., promoter, 5'UTR) of each, are generated by introducing into protoplasts the gene editing system provided herein, including one or more guide RNAs as described in Examples 1 and 2. In specific experiments, two or more guide RNAs are used.

The mutants having mutations in *GmASAl* gene, *GmHMT1* gene, or *GmHMT3* gene are screened for editing efficiency and expression levels. Expression cassettes comprising the (mutated or wild-type) Glyma. 18G028900 *GmASAl* gene, the (mutated or wild-type) Glyma. 19G158800 *GmHMT1* gene, or the (mutated or wild-type) Glyma.20G148900 *GmHMT3* gene, operably linked to a functional promoter, are generated. The cassettes with mutations, as well as no mutations (wild-type) are transiently expressed in tobacco leaves. Levels of the Glyma. 18G028900 *GmASAl* gene, the Glyma.19G158800 *GmHMT1* gene, or the Glyma.20G148900 *GmHMT3* gene, are measured by standard methods for measuring mRNA levels of a gene, including quantitative RT-PCR, northern blot, and serial analysis of gene expression (SAGE). Levels of the *Gm*ASA1, *Gm*HMT1, or *Gm*HMT3 polypeptide encoded by the Glyma. 18G028900 *GmASA1* gene, the Glyma. 19G158800 *GmHMT1* gene, or the Glyma.20G148900 *GmHMT3* gene, respectively, are also measured by standard methods for measuring protein levels, including western blot analysis, ELISA, or dot blot analysis of a plant sample using an antibody directed to the *Gm*ASA1, *Gm*HMT1, or *Gm*HMT3 polypeptide.

The mutants having mutation in the regulatory region (e.g., promoter, 5'UTR) of a *GmASA1* gene, *GmHMT1* gene, or *GmHMT3* gene, are screened for editing efficiency and effects on expression levels of a downstream gene. Expression cassettes comprising the (mutated or wild-type) regulatory region of the Glyma. 18G028900 *GmASAl* gene, the (mutated or wild-type) regulatory region of the Glyma. 19G158800 *GmHMT1* gene, or the (mutated or wild-type) regulatory region of the Glyma.20G148900 *GmHMT3* gene, operably linked to a polynucleotide encoding a reporter (e.g., GFP, luciferase), are generated. The cassettes with mutations, as well as no mutations (wild-type) are transiently expressed in tobacco leaves, and reporter (e.g., GFP, luciferase) protein levels in infiltrated leaves are quantified as a readout for expression levels of genes operably linked to the (mutated or wild-type) regulatory region of Glyma. 18G028900 *GmASAl* gene, the (mutated or wild-type) regulatory region of the Glyma.19G158800 *GmHMT1* gene, or the (mutated or wild-type) regulatory region of the Glyma.20G148900 *GmHMT3* gene.

### EXAMPLE 4: Generation of T0 and T1 soybean plants with mutations in ASA1, HMT1, and/or HMT3 gene(s)

Embryonic axes of mature seeds of soybean varieties are stably transformed with constructs comprising: one, two, or multiple guide RNAs (e.g., SEQ ID NOs: 19-21) targeting the Glyma.18G028900 *GmASA1* gene, the Glyma.19G158800 *GmHMT1* gene, or the Glyma.20G148900 *GmHMT3* gene, or their regulatory regions (e.g., promoter, 5'UTR); and a nuclease, using *Agrobacterium* transformation. Transformed plants are identified by selective marker (e.g., resistance to an herbicide). Amplicons are produced of the genomic regions near the targeted Glyma.18G028900 *GmASA1,* Glyma.19G158800 *GmHMT1,* and/or Glyma.20G148900 *GmHMT3* sites and sequenced to evaluate the presence of the mutation using pairs of primers to detect mutations introduced. Transgenic events are recorded, and the T0 plants are assigned unique plant names and are subjected to molecular characterization and propagation.

Partial nucleic acid sequences of the T0 plants with mutations introduced into *ASA1, HMT1,* or *HMT3* using the gRNAs of SEQ ID NOs: 19-21 are determined. Homozygous and/or heterozygous deletions are identified in T0 plants: 6 bp in *GmASA1*; 12 bp in *GmASA1,* 24 bp in *GmASA1,* 3 bp in *GmHMT1*; 66 bp in *GmHMT1*; 2 bp in *GmHMT3*; and/or 13 bp in *GmHMT3.* A nucleic acid sequence of *GmASAl* gene having 6 bp deletion, as compared to SEQ ID NO: 1, is set forth as SEQ ID NO: 3 and encodes a mutant ASA1 protein set forth as SEQ ID NO: 6. A nucleic acid sequence of *GmASA1* gene having 12 bp deletion, as compared to SEQ ID NO: 1, is set forth as SEQ ID NO: 4 and encodes a mutant ASA1 protein set forth as SEQ ID NO: 7. A nucleic acid sequence of *GmASA1* gene having 24 bp deletion, as compared to SEQ ID NO: 1, is set forth as SEQ ID NO: 5 and encodes a mutant ASA1 protein set forth as SEQ ID NO: 8. A nucleic acid sequence of *GmHMT1* gene having 3 bp deletion, as compared to SEQ ID NO: 9, is set forth as SEQ ID NO: 11. A nucleic acid sequence of *GmHMT1* gene having 66 bp deletion, as compared to SEQ ID NO: 9, is set forth as SEQ ID NO: 12. A nucleic acid sequence of *GmHMT3* gene having 2 bp deletion, as compared to SEQ ID NO: 13, is set forth as SEQ ID NO: 15. A nucleic acid sequence of *GmHMT3* gene having 13 bp deletion, as compared to SEQ ID NO: 13, is set forth as SEQ ID NO: 16.

T0 plants are self-pollinated and T1 plants are generated. Crosses are made to generate lines that are homozygous or heterozygous for the target mutation and lack the editing reagents. Expression and activity levels of the ASA1, HMT1, or HMT3 polypeptides, as well as seed Trp and/or Met content of transformed plants are analyzed, as described in Examples 5-7.

### EXAMPLE 5: Screening of plants with mutations in ASA1, HMT1, and/or HMT3 gene(s) by measuring gene/protein levels

Transformed plants are screened using a variety of molecular tools to identify plants and genotypes that will result in the expected phenotype. For example, expression levels of *GmASA1* gene, *GmHMT1* gene, and/or *GmHMT3* gene and levels of the polypeptides encoded by these genes are measured in mutant plants. Expression levels of *GmASA1, GmHMT1,* and/or *GmHMT3* gene(s) are measured by any standard methods for measuring mRNA levels of a gene, including quantitative RT-PCR, northern blot, and serial analysis of gene expression (SAGE). Expression levels of *Gm*ASA1, *Gm*HMT1, and/or *Gm*HMT3 polypeptides are measured by any standard methods for measuring protein levels, including western blot analysis, ELISA, or dot blot analysis of a protein sample obtained from the plant using an antibody directed to the respective polypeptide.

### EXAMPLE 6: Measuring Trp feedback inhibition of mutated ASA enzymes and/or ASA enzymes comprising genetic mutations

Sensitivity of an ASA enzyme to Trp feedback inhibition in plants and plant parts comprising at least one endogenous *ASA* gene or homolog thereof comprising a genetic mutation can be assessed by measuring ASA enzyme activity in the presence of increasing concentrations of Trp as described in e.g., Cho et al. (2000) Plant Physiology 123:1069-1076; Bernasconi et al. (1994) Plant Physiol 106: 353-358; Song et al. (1998) 117:533-543; Widholm JM (1971) Physiol Plant 5: 75-79; and Li and Last (1996) Plant Physiol 110: 51-59. For example, an AS enzyme solution is prepared by grinding of plant tissue, (NH4)₂SO₄ precipitation, and resuspension of the precipitate in extraction buffer. The enzyme solution may be further desalted with Sephadex-25. The assay buffer to measure AS activity includes, for example: a buffer such as 50 mm Hepes, pH 7.5 or Tris-HCl, pH 8.0; chorismate as the first substrate (e.g., concentrations of 0 to 500 µM); Gln (e.g., 10-20 mM) or NH4+ (e.g., 100 mM) as the second substrate; MgCh (e.g., 2-5 mM); 0.05 mM Na₂EDTA; DTT (e.g., 0.05-2.0 mM); and glycerol (e.g., 5-20%). Trp concentrations include a range of 0 to 1000 µM. Chorismate can be produced using the fermentation method of Gibson (1970) Methods Enzymol 17: 362-364. The anthranilate product is extracted with ethylacetate, centrifuged, and the upper phase quantified by fluorescence emission at 400 nm (excitation at 340 nm) with a fluorescence spectrophotometer. ASA enzyme activity can be expressed as concentration of anthranilate per time per amount of protein (e.g., nmol anthranilate formed min⁻¹mg⁻¹ protein).

A mutated ASA enzyme that is insensitive to or has reduced sensitivity to Trp feedback inhibition is resistant to toxic anthranilate analogs including: 3-methyl-anthranilate (3MA), 5-methyl-anthranilate (5MA), 6-methyl-anthranilate (6MA), 5-fluoro-anthranilate (5FA), and 6-fluoro-anthranilate (6FA); and/or toxic Trp analogs including: 4-methyl-Trp (4MT), 5-methyl-Trp (5MT), 6-methyl-Trp (6MT), 7-methyl-Trp (7MT), and alpha-methyl-Trp (αMT). Resistance to toxic anthranilate and/or Trp analogs is ascertained by growing plants comprising one or more genetic mutations in an *ASA* gene in petri dishes containing agar supplemented with a toxic analog and monitoring growth of plant seedlings.

### EXAMPLE 7: Measuring Trp and/or Met content in seed and vegetative tissues of plants having mutated ASA and/or HMT genes

Trp and/or Met levels in seed and/or vegetative tissues of plants and plant parts comprising at least one endogenous *AS* gene or homolog thereof and/or at least one endogenous *HMT* gene or homolog thereof comprising a genetic mutation are measured by any standard methods of measuring or estimating the amount of Trp and/or Met amino acid content in a sample. For example, amino acid levels are measured in seed by homogenizing seed in HCl (e.g., 20 mM or 0.1N), centrifuging to sediment debris, deproteinating the supernatant with a filter unit, and the filtrate diluted with HCl for analysis by HPLC. Measurement of free Trp is described in, e.g., Li and Last (1996) Plant Physiol 110: 51-59, Cho et al. (2000) Plant Physiology 123:1069-1076, Berardino et al. (1990) J Nutr Biochem 1: 220-222, and Heinrikson and Meredith (1984) Anal Biochem 136:65-74. Measurement of amino acid levels is described in, e.g., Lee et al. (2008) The Plant Journal 54:310-320. Plant tissue can be collected and frozen in liquid nitrogen and stored at -80°C until analysis. Amino acid concentrations are calculated by comparing peak areas to those of standard curves prepared with commercially available pure amino acid compounds. Amino acid concentration can be expressed as concentration of an amino acid (e.g., Trp, Met) per weight of fresh or dry tissue (e.g., nmol g⁻¹ fresh wt). Controls comprising plants or plant parts comprising at least one endogenous *AS* gene or homolog thereof and/or at least one endogenous *HMT* gene or homolog thereof not comprising the genetic mutation are included in the assays.

### EMBODIMENTS

The following represent specific embodiments of the invention.
1. A plant or plant part comprising a genetic mutation in at least one endogenous *AS* gene or homolog thereof encoding an anthranilate synthase and/or *HMT* gene or homolog thereof encoding a homocysteine *S*-methyltransferase, wherein the mutated plant or plant part comprises increased tryptophan (Trp) and/or methionine (Met) amino acid content as compared to a control.
2. The plant or plant part of embodiment 1, wherein the plant or plant part is a seed.
3. The plant or plant part of embodiment 1 or 2, wherein the genetic mutation comprises one or more insertions, substitutions, or deletions in the at least one endogenous *AS* gene or homolog thereof and/or *HMT* gene or homolog thereof.
4. The plant or plant part of any one of embodiments 1-3, wherein the *AS* gene or homolog thereof comprises *an ASA* gene or homolog thereof encoding an anthranilate synthase alpha subunit.
5. The plant or plant part of embodiment 4, wherein the genetic mutation comprises an in-frame deletion in the *ASA* gene or homolog thereof.
6. The plant or plant part of embodiment 5, wherein the in-frame deletion comprises a deletion of 3 to 30 nucleotides.
7. The plant or plant part of any one of embodiments 4-6, wherein the genetic mutation is located in an enzyme regulatory region of the encoded ASA protein.
8. The plant or plant part of embodiment 7, wherein the enzyme regulatory region comprises a region that binds Trp.
9. The plant or plant part of any one of embodiments 1-8, wherein the *AS* gene or homolog thereof comprising the genetic mutation encodes an anthranilate synthase that is insensitive to or has reduced sensitivity to Trp feedback inhibition.
10. The plant or plant part of any one of embodiments 4-9, wherein the genetic mutation is located in a *ASA1* gene or homolog thereof
   (i) comprising a nucleic acid sequence having at least 80% sequence identity to the nucleic acid sequence set forth as SEQ ID NO: 1 and encoding an active anthranilate synthase;
   (ii) comprising the nucleic acid sequence set forth as SEQ ID NO: 1;
   (iii) encoding an anthranilate synthase alpha subunit comprising an amino acid sequence having at least 80% sequence identity to an amino acid sequence set forth as SEQ ID NO: 2, wherein the anthranilate synthase alpha subunit is active; and/or
   (iv) encoding an anthranilate synthase alpha subunit comprising an amino acid sequence set forth as SEQ ID NO: 2.
11. The plant or plant part of any one of embodiments 1-10, wherein the genetic mutation comprises a deletion in the *HMT* gene or homolog thereof.
12. The plant or plant part of embodiment 11, wherein the deletion comprises a deletion of 1 to 70 nucleotides.
13. The plant or plant part of any one of embodiments 1-12, wherein the genetic mutation is located in a *HMT1* gene or homolog thereof
   (i) comprising a nucleic acid sequence having at least 80% sequence identity to the nucleic acid sequence set forth as SEQ ID NO: 9 and encoding an active homocysteine *S-*methyltransferase;
   (ii) comprising the nucleic acid sequence set forth as SEQ ID NO: 9;
   (iii) encoding a homocysteine S-methyltransferase comprising an amino acid sequence having at least 80% sequence identity to an amino acid sequence set forth as SEQ ID NO: 10, wherein the homocysteine S-methyltransferase is active; and/or
   (iv) encoding a homocysteine S-methyltransferase comprising an amino acid sequence set forth as SEQ ID NO: 10.
14. The plant or plant part of any one of embodiments 1-13, wherein the genetic mutation is located in a *HMT3* gene or homolog thereof
   (i) comprising a nucleic acid sequence having at least 80% sequence identity to the nucleic acid sequence set forth as SEQ ID NO: 13 and encodes an active homocysteine *S-*methyltransferase;
   (ii) comprising the nucleic acid sequence set forth as SEQ ID NO: 13;
   (iii) encoding a homocysteine *S*-methyltransferase comprising an amino acid sequence having at least 80% sequence identity to an amino acid sequence set forth as SEQ ID NO: 14, wherein the homocysteine *S*-methyltransferase is active; and/or
   (iv) encoding a homocysteine *S*-methyltransferase comprising an amino acid sequence set forth as SEQ ID NO: 14.
15. The plant or plant part of any one of embodiments 1-14, wherein the plant or plant part comprises:
   (i) an in-frame deletion of nucleotides 952 to 957 of *ASA1* gene having the nucleic acid sequence set forth as SEQ ID NO: 1, or a nucleic acid sequence comprising SEQ ID NO: 3 when the deletion of (i) is introduced;
   (ii) an in-frame deletion of nucleotides 948 to 959 of *ASA1* gene having the nucleic acid sequence set forth as SEQ ID NO: 1, or a nucleic acid sequence comprising SEQ ID NO: 4 when the deletion of (ii) is introduced;
   (iii) an in-frame deletion of nucleotides 938 to 961 of *ASA1* gene having the nucleic acid sequence set forth as SEQ ID NO: 1, or a nucleic acid sequence comprising SEQ ID NO: 5 when the deletion of (iii) is introduced;
   (iv) a deletion of nucleotides 712 to 714 of *HMT1* gene having the nucleic acid sequence set forth as SEQ ID NO: 9, or a nucleic acid sequence comprising SEQ ID NO: 11 when the deletion of (iv) is introduced;
   (v) a deletion of nucleotides 695 to 760 of *HMT1* gene having the nucleic acid sequence set forth as SEQ ID NO: 9, or a nucleic acid sequence comprising SEQ ID NO: 12 when the deletion of (v) is introduced;
   (vi) a deletion of nucleotides 2783 to 2784 *of HMT3* gene having the nucleic acid sequence set forth as SEQ ID NO: 13, or a nucleic acid sequence comprising SEQ ID NO: 15 when the deletion of (vi) is introduced; or
   (vii) a deletion of nucleotides 2778 to 2790 *of HMT3* gene having the nucleic acid sequence set forth as SEQ ID NO: 13, or a nucleic acid sequence comprising SEQ ID NO: 16 when the deletion of (vii) is introduced.
16. The plant or plant part of embodiment 15, wherein the *ASA1* gene comprising the genetic mutation encodes an anthranilate synthase alpha subunit having an amino acid sequence set forth as any one of SEQ ID NOs: 6-8.
17. The plant or plant part of any one of embodiments 1-16, wherein Trp and/or Met content in the seed is 1. 1-fold to 10-fold greater than that of the control.
18. The plant or plant part of any one of embodiments 1-17, wherein the *S-*methylmethionine (SMM) level in vegetative tissue is increased as compared to the control.
19. The plant or plant part of any one of embodiments 1-18, wherein the control comprises a corresponding plant, plant part, or seed without the genetic mutation.
20. The plant or plant part of any one of embodiments 1-19, wherein the plant or plant part is from a legume.
21. The plant or plant part of embodiment 20, wherein the legume is soybean (*Glycine max*) or pea (*Pisum sativum*)*.*
22. A population of plants or plant parts comprising the plant or plant part of any one of embodiments 1-21.
23. The population of plants or plant parts of embodiment 22, wherein the population is a population of seeds, and the plant or plant part is a seed.
24. A seed composition produced from the plant or plant part of any one of embodiments 1-21, or the population of plants or plant parts of embodiment 22 or 23.
25. A protein composition produced from the plant or plant part of any one of embodiments 1-21, the population of plants or plant parts of embodiment 22 or 23, or the seed composition of embodiment 24.
26. A method for increasing tryptophan (Trp) and/or methionine (Met) amino acid content produced by a plant, the method comprising:
   introducing editing reagents into a plant, plant part, or plant cell to produce a mutated plant, plant part, or plant cell comprising a genetic mutation at at least one target site, wherein the at least one target site comprises an endogenous *AS* gene or homolog thereof encoding an anthranilate synthase and/or an endogenous *HMT* gene or homolog thereof encoding a homocysteine *S*-methyltransferase, and wherein Trp and/or Met content in a plant or plant part generated from the mutated plant part, plant part, or plant cell, is increased as compared to a control.
27. The method of embodiment 26, wherein the generated plant or plant part is a seed.
28. The method of embodiment 26 or 27, wherein the editing reagents comprise:
   at least one RNA-guided nuclease, or a polynucleotide encoding the at least one RNA-guided nuclease; and
   at least one guide RNA (gRNA), or a polynucleotide encoding the at least one gRNA, wherein the at least one gRNA is complementary to genomic sequence of the endogenous *AS* gene or homolog thereof and/or the endogenous *HMT* gene or homolog thereof,
   and wherein the at least one gRNA is capable of binding the at least one RNA-guided nuclease to form a ribonucleoprotein (RNP) complex.
29. The method of any one of embodiments 26-28, wherein the introducing comprises introducing the editing reagents into the plant cell, and
   culturing the plant cell to regenerate a plant or plant part comprising the genetic mutation.
30. The method of any one of embodiments 27-29, wherein Trp and/or Met content in the seed is 1.1-fold to 10-fold greater than that of the control.
31. The method of any one of embodiments 26-30, wherein the control comprises a corresponding plant, plant part, plant cell, or seed without the genetic mutation.
32. The method of any one of embodiments 28-31, wherein the at least one RNA-guided nuclease comprises a CRISPR (Clustered Regularly Interspaced Palindromic Repeats) nuclease.
33. The method of embodiment 32, wherein a proto-adjacent motif (PAM) recognized by the CRISPR nuclease comprises a nucleic acid sequence of TTTA or TTTG.
34. The method of embodiment 32, wherein the CRISPR nuclease comprises a Type II CRISPR system nuclease or a Type V CRISPR system nuclease.
35. The method of embodiment 34, wherein the Type II CRISPR system nuclease comprises a Cas9 nuclease.
36. The method of embodiment 34, wherein the Type V CRISPR system nuclease comprises a Cas12a (Cpf1) nuclease or a Cms1 nuclease.
37. The method of embodiment 36, wherein the Type V CRISPR nuclease comprises the Cas12a (Cpf1) nuclease or an ortholog thereof.
38. The method of embodiment 37, wherein the Type V CRISPR nuclease is encoded by a nucleotide sequence set forth as SEQ ID NO: 25.
39. The method of embodiment 37 or 38, wherein the CRISPR nuclease has an amino acid sequence set forth as SEQ ID NO: 26.
40. The method of any one of embodiments 28-39, wherein the at least one gRNA comprises:
   (a) a nucleic acid sequence having at least 80% sequence identity to the nucleic acid sequence set forth as any one of SEQ ID NOs: 19-21; or
   (b) a nucleic acid sequence having the nucleic acid sequence set forth as any one of SEQ ID NOs: 19-21.
41. The method of any one of embodiments 26-40, wherein the at least one target site comprises a nucleic acid sequence set forth as any one of SEQ ID NOs: 22-24.
42. The method of any one of embodiments 26-41, wherein the genetic mutation comprises one or more insertions, substitutions, or deletions in the endogenous *AS* gene or homolog thereof and/or in the endogenous *HMT* gene or homolog thereof.
43. The method of any one of embodiments 26-42, wherein the endogenous *AS* gene or homolog thereof is an endogenous *ASA* gene or homolog thereof.
44. The method of embodiment 43, wherein the genetic mutation comprises an in-frame deletion in the *ASA* gene or homolog thereof.
45. The method of embodiment 44, wherein the in-frame deletion comprises a deletion of 3 to 30 nucleotides.
46. The method of any one of embodiments 43-45, wherein the genetic mutation is located in an enzyme regulatory region of the encoded ASA protein.
47. The method of embodiment 46, wherein the enzyme regulatory region comprises a region that binds Trp.
48. The method of any one of embodiments 26-47, wherein the *AS* gene or homolog thereof comprising the genetic mutation encodes an anthranilate synthase that is insensitive to or has reduced sensitivity to Trp feedback inhibition.
49. The method of any one of embodiments 43-48, wherein the genetic mutation is located in a *ASA1* gene or homolog thereof
   (i) comprising a nucleic acid sequence having at least 80% sequence identity to the nucleic acid sequence set forth as SEQ ID NO: 1 and encoding an active anthranilate synthase;
   (ii) comprising the nucleic acid sequence set forth as SEQ ID NO: 1;
   (iii) encoding an anthranilate synthase alpha subunit comprising an amino acid sequence having at least 80% sequence identity to an amino acid sequence set forth as SEQ ID NO: 2, wherein the anthranilate synthase alpha subunit is active; and/or
   (iv) encoding an anthranilate synthase alpha subunit comprising an amino acid sequence set forth as SEQ ID NO: 2.
50. The method of embodiment 49, wherein the *ASA1* gene or homolog thereof comprising the genetic mutation encodes an anthranilate synthase alpha subunit having an amino acid sequence set forth as any one of SEQ ID NOs: 6-8.
51. The method of any one of embodiments 26-50, wherein the genetic mutation comprises a deletion in the *HMT* gene or homolog thereof.
52. The method of embodiment 51, wherein the deletion comprises a deletion of 1 to 70 nucleotides.
53. The method of any one of embodiments 26-52, wherein the genetic mutation is located in an *HMT1* gene or homolog thereof
   (i) comprising a nucleic acid sequence having at least 80% sequence identity to the nucleic acid sequence set forth as SEQ ID NO: 9 and encoding an active homocysteine S-methyltransferase;
   (ii) comprising the nucleic acid sequence set forth as SEQ ID NO: 9;
   (iii) encoding a homocysteine *S*-methyltransferase comprising an amino acid sequence having at least 80% sequence identity to an amino acid sequence set forth as SEQ ID NO: 10, wherein the homocysteine *S*-methyltransferase is active; and/or
   (iv) encoding a homocysteine *S*-methyltransferase comprising an amino acid sequence set forth as SEQ ID NO: 10.
54. The method of any one of embodiments 26-53, wherein the genetic mutation is located in an *HMT3* gene or homolog thereof
   (i) comprising a nucleic acid sequence having at least 80% sequence identity to the nucleic acid sequence set forth as SEQ ID NO: 13 and encodes an active homocysteine *S-*methyltransferase;
   (ii) comprising the nucleic acid sequence set forth as SEQ ID NO: 13;
   (iii) encoding a homocysteine *S*-methyltransferase comprising an amino acid sequence having at least 80% sequence identity to an amino acid sequence set forth as SEQ ID NO: 14, wherein the homocysteine *S*-methyltransferase is active; and/or
   (iv) encoding a homocysteine *S*-methyltransferase comprising an amino acid sequence set forth as SEQ ID NO: 14.
55. The method of any one of embodiments 26-54, wherein the plant, plant part, or plant cell comprising the genetic mutation comprises:
   (i) an in-frame deletion of nucleotides 952 to 957 of *ASA1* having the nucleic acid sequence set forth as SEQ ID NO: 1, or a nucleic acid sequence comprising SEQ ID NO: 3 when the deletion of (i) is introduced;
   (ii) an in-frame deletion of nucleotides 948 to 959 of *ASA1* having the nucleic acid sequence set forth as SEQ ID NO: 1, or a nucleic acid sequence comprising SEQ ID NO: 4 when the deletion of (ii) is introduced;
   (iii) an in-frame deletion of nucleotides 938 to 961 of *ASA1* having the nucleic acid sequence set forth as SEQ ID NO: 1, or a nucleic acid sequence comprising SEQ ID NO: 5 when the deletion of (iii) is introduced;
   (iv) a deletion of nucleotides 712 to 714 of *HMT1* having the nucleic acid sequence set forth as SEQ ID NO: 9, or a nucleic acid sequence comprising SEQ ID NO: 11 when the deletion of (iv) is introduced;
   (v) a deletion of nucleotides 695 to 760 of *HMT1* having the nucleic acid sequence set forth as SEQ ID NO: 9, or a nucleic acid sequence comprising SEQ ID NO: 12 when the deletion of (v) is introduced;
   (vi) a deletion of nucleotides 2783 to 2784 *of HMT3* having the nucleic acid sequence set forth as SEQ ID NO: 13, or a nucleic acid sequence comprising SEQ ID NO: 15 when the deletion of (vi) is introduced; or
   (vii) a deletion of nucleotides 2778 to 2790 *of HMT3* having the nucleic acid sequence set forth as SEQ ID NO: 13, or a nucleic acid sequence comprising SEQ ID NO: 16 when the deletion of (vi) is introduced.
56. The method of any one of embodiments 26-55, wherein the plant, plant part, or plant cell is from a legume.
57. The method of embodiment 56, wherein the legume is soybean (*Glycine max)* or pea *(Pisum sativum).*
58. A plant, plant part, plant cell, or population of plants produced by the method of any one of embodiments 26-57.
59. The plant, plant part, plant cell, or population of plants of embodiment 58, wherein seed of plants generated from the plant, plant part, plant cell, or population of plants of embodiment 58 comprises increased Trp and/or Met content as compared to a control.
60. A seed composition produced from the plant, plant part, plant cell, or population of plants of embodiment 58, wherein the seed comprise increased Trp and/or Met content compared to a control.
61. The plant, plant part, plant cell, or population of plants of embodiment 58 or 59, or the seed composition of embodiment 60, wherein the increased Trp and/or Met content is 1.1-fold to 10-fold greater than that of the control.
62. The plant, plant part, plant cell, or population of plants of embodiment 58, 59, and 61, or the seed composition of embodiment 60 or 61, wherein the control comprises a corresponding plant, plant part, plant cell, population of plants, or seed composition without the genetic mutation.
63. A protein composition produced from the plant, plant part, plant cell, or population of plants of embodiment 58 or 59, or from the seed composition of embodiment 60.
64. The protein composition of embodiment 63, wherein the protein composition is present in soybean meal.
65. The protein composition of embodiment 63 or 64, wherein the protein composition is present in animal feed.
66. A nucleic acid molecule comprising a nucleic acid sequence of a mutated anthranilate synthase alpha subunit *(ASA)* gene, wherein the mutated *ASA* gene comprises one or more insertions, substitutions, or deletions as compared to a corresponding *ASA* gene without the mutations, wherein the nucleic acid sequence:
   (i) comprises at least 80% sequence identity to a nucleic acid sequence set forth as SEQ ID NO: 1 and encodes an active ASA;
   (ii) comprises the nucleic acid sequence set forth as SEQ ID NO: 1;
   (iii) encodes an ASA comprising an amino acid sequence having at least 80% sequence identity to an amino acid sequence set forth as SEQ ID NO: 2, wherein the ASA is active; and/or
   (iv) encodes an ASA comprising an amino acid sequence set forth as SEQ ID NO: 2.
67. The nucleic acid molecule of embodiment 66, wherein the mutated *ASA* gene comprises:
   (i) an in-frame deletion of nucleotides 952 to 957 of *ASA1* having the nucleic acid sequence set forth as SEQ ID NO: 1, or a nucleic acid sequence comprising SEQ ID NO: 3 when the deletion of (i) is introduced;
   (ii) an in-frame deletion of nucleotides 948 to 959 of *ASA1* having the nucleic acid sequence set forth as SEQ ID NO: 1, or a nucleic acid sequence comprising SEQ ID NO: 4 when the deletion of (ii) is introduced; or
   (iii) an in-frame deletion of nucleotides 938 to 961 of *ASA1* having the nucleic acid sequence set forth as SEQ ID NO: 1, or a nucleic acid sequence comprising SEQ ID NO: 5 when the deletion of (iii) is introduced.
68. The nucleic acid molecule of embodiment 66 or 67, wherein the encoded active anthranilate synthase is insensitive to or has reduced sensitivity to Trp feedback inhibition.
69. A genetic construct comprising, in operable linkage:
   (i) a promoter that is functional in a plant cell; and
   (ii) the nucleic acid molecule of any one of embodiments 66-68.
70. A cell comprising the nucleic acid molecule of any one of embodiments 66-68, or the genetic construct of embodiment 69.
71. The cell of embodiment 70, wherein the cell is a plant cell.

The section headings used herein are for organizational purposes only and are not to be construed as limiting the subject matter described in any way.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. Certain features described in the context of various embodiments are not to be considered essential features of those embodiments, unless the embodiment is inoperative without those elements.

While various aspects of the invention are described herein, it is not intended that the invention be limited by any particular aspect. On the contrary, the invention encompasses various alternatives, modifications, and equivalents, as will be appreciated by those of skill in the art. Furthermore, where feasible, any of the aspects disclosed herein may be combined with each other (e.g., the feature according to one aspect may be added to the features of another aspect or replace an equivalent feature of another aspect) or with features that are well known in the art, unless indicated otherwise by context.

## Claims

1. A plant or plant part comprising a genetic mutation in at least one endogenous anthranilate synthase (*AS*) gene or homolog thereof encoding an anthranilate synthase and/or homocysteine S-methyltransferase *(HMT)* gene or homolog thereof encoding a homocysteine S-methyltransferase, wherein the mutated plant or plant part comprises increased tryptophan (Trp) and/or methionine (Met) amino acid content as compared to a control.

2. The plant or plant part of claim 1,
wherein the *AS* gene or homolog thereof comprises an anthranilate synthase alpha (*ASA*) gene or homolog thereof encoding an anthranilate synthase alpha subunit;
wherein the genetic mutation is located in an enzyme regulatory region of the encoded ASA subunit, optionally wherein the enzyme regulatory region comprises a region that binds Trp;
wherein the genetic mutation is located in a *ASA1* gene or homolog thereof
(i) comprising a nucleic acid sequence having at least 80% sequence identity to the nucleic acid sequence set forth as SEQ ID NO: 1 and encoding an active anthranilate synthase;
(ii) comprising the nucleic acid sequence set forth as SEQ ID NO: 1;
(iii) encoding an anthranilate synthase alpha subunit comprising an amino acid sequence having at least 80% sequence identity to an amino acid sequence set forth as SEQ ID NO: 2, wherein the anthranilate synthase alpha subunit is active; and/or
(iv) encoding an anthranilate synthase alpha subunit comprising an amino acid sequence set forth as SEQ ID NO: 2;
wherein the genetic mutation is located in a *HMT1* gene or homolog thereof
(v) comprising a nucleic acid sequence having at least 80% sequence identity to the nucleic acid sequence set forth as SEQ ID NO: 9 and encoding an active homocysteine S-methyltransferase;
(vi) comprising the nucleic acid sequence set forth as SEQ ID NO: 9;
(vii) encoding a homocysteine S-methyltransferase comprising an amino acid sequence having at least 80% sequence identity to an amino acid sequence set forth as SEQ ID NO: 10, wherein the homocysteine S-methyltransferase is active; and/or
(viii) encoding a homocysteine S-methyltransferase comprising an amino acid sequence set forth as SEQ ID NO: 10; and/or
wherein the genetic mutation is located in a HMT3 gene or homolog thereof
(ix) comprising a nucleic acid sequence having at least 80% sequence identity to the nucleic acid sequence set forth as SEQ ID NO: 13 and encodes an active homocysteine S-methyltransferase;
(x) comprising the nucleic acid sequence set forth as SEQ ID NO: 13;
(xi) encoding a homocysteine S-methyltransferase comprising an amino acid sequence having at least 80% sequence identity to an amino acid sequence set forth as SEQ ID NO: 14, wherein the homocysteine S-methyltransferase is active; and/or
(xii) encoding a homocysteine S-methyltransferase comprising an amino acid sequence set forth as SEQ ID NO: 14.

3. The plant or plant part of claim 1 or 2, wherein the plant or plant part comprises:
(i) an in-frame deletion of nucleotides 952 to 957 of ASA1 gene having the nucleic acid sequence set forth as SEQ ID NO: 1, or a nucleic acid sequence comprising SEQ ID NO: 3 or encoding an amino acid sequence of SEQ ID NO: 6 when the deletion of (i) is introduced;
(ii) an in-frame deletion of nucleotides 948 to 959 of ASA1 gene having the nucleic acid sequence set forth as SEQ ID NO: 1, or a nucleic acid sequence comprising SEQ ID NO: 4 or encoding an amino acid sequence of SEQ ID NO: 7 when the deletion of (ii) is introduced;
(iii) an in-frame deletion of nucleotides 938 to 961 of ASA1 gene having the nucleic acid sequence set forth as SEQ ID NO: 1, or a nucleic acid sequence comprising SEQ ID NO: 5 or encoding an amino acid sequence of SEQ ID NO: 8 when the deletion of (iii) is introduced;
(iv) a deletion of nucleotides 712 to 714 of HMT1 gene having the nucleic acid sequence set forth as SEQ ID NO: 9, or a nucleic acid sequence comprising SEQ ID NO: 11 when the deletion of (iv) is introduced;
(v) a deletion of nucleotides 695 to 760 of HMT1 gene having the nucleic acid sequence set forth as SEQ ID NO: 9, or a nucleic acid sequence comprising SEQ ID NO: 12 when the deletion of (v) is introduced;
(vi) a deletion of nucleotides 2783 to 2784 of HMT3 gene having the nucleic acid sequence set forth as SEQ ID NO: 13, or a nucleic acid sequence comprising SEQ ID NO: 15 when the deletion of (vi) is introduced; or
(vii) a deletion of nucleotides 2778 to 2790 of HMT3 gene having the nucleic acid sequence set forth as SEQ ID NO: 13, or a nucleic acid sequence comprising SEQ ID NO: 16 when the deletion of (vii) is introduced.

4. The plant or plant part of any one of claims 1-3 or a population of plants or plant parts comprising the plant or plant part of any one of claims 1-3,
wherein Trp and/or Met content in the plant, plant part, or the population of plants or plant parts is 1. 1-fold to 10-fold as compared to that of the control, and/or
wherein S-methylmethionine (SMM) level in vegetative tissue is increased as compared to the control,
optionally wherein the plant or plant part is a legume or is soybean (*Glycine max*) or pea (*Pisum sativum*)*.*

5. A method for increasing tryptophan (Trp) and/or methionine (Met) amino acid content produced by a plant, the method comprising:
introducing editing reagents into a plant, plant part, or plant cell to produce a mutated plant, plant part, or plant cell comprising a genetic mutation at at least one target site,
wherein the at least one target site comprises an endogenous anthranilate synthase (*AS*) gene or homolog thereof encoding an anthranilate synthase and/or an endogenous homocysteine S-methyltransferase (*HMT*) gene or homolog thereof encoding a homocysteine S-methyltransferase, and wherein Trp and/or Met content in a plant or plant part generated from the mutated plant part, plant part, or plant cell, is increased as compared to a control.

6. The method of claim 5, wherein the editing reagents comprise:
at least one RNA-guided nuclease, or a polynucleotide encoding the at least one RNA-guided nuclease; and
at least one guide RNA (gRNA), or a polynucleotide encoding the at least one gRNA,
wherein the at least one gRNA is complementary to genomic sequence of the endogenous AS gene or homolog thereof and/or the endogenous HMT gene or homolog thereof,
and wherein the at least one gRNA is capable of binding the at least one RNA-guided nuclease to form a ribonucleoprotein (RNP) complex, and/or
wherein the at least one RNA-guided nuclease comprises a CRISPR (Clustered Regularly Interspaced Palindromic Repeats) nuclease.

7. The method of claim 6, wherein the at least one RNA-guided nuclease comprises a CRISPR nuclease and a proto-adjacent motif (PAM) recognized by the CRISPR nuclease comprises a nucleic acid sequence of TTTA or TTTG;
wherein the CRISPR nuclease comprises a Type II CRISPR system nuclease or a Type V CRISPR system nuclease;
wherein the Type II CRISPR system nuclease comprises a Cas9 nuclease;
wherein the Type V CRISPR system nuclease comprises a Cas12a (Cpf1) nuclease or a Cms1 nuclease or an ortholog thereof, or is encoded by a nucleotide sequence set forth as SEQ ID NO: 25;
wherein the CRISPR nuclease has an amino acid sequence set forth as SEQ ID NO: 26;
wherein the at least one gRNA comprises:
(a) a nucleic acid sequence having at least 80% sequence identity to the nucleic acid sequence set forth as any one of SEQ ID NOs: 19-21; or
(b) a nucleic acid sequence having the nucleic acid sequence set forth as any one of SEQ ID NOs: 19-21; and/or
wherein the at least one target site comprises a nucleic acid sequence set forth as any one of SEQ ID NOs: 22-24.

8. The method of any one of claims 5-7,
wherein the genetic mutation is located in a ASA1 gene or homolog thereof
(i) comprising a nucleic acid sequence having at least 80% sequence identity to the nucleic acid sequence set forth as SEQ ID NO: 1 and encoding an active anthranilate synthase;
(ii) comprising the nucleic acid sequence set forth as SEQ ID NO: 1;
(iii) encoding an anthranilate synthase alpha subunit comprising an amino acid sequence having at least 80% sequence identity to an amino acid sequence set forth as SEQ ID NO: 2, wherein the anthranilate synthase alpha subunit is active; and/or
(iv) encoding an anthranilate synthase alpha subunit comprising an amino acid sequence set forth as SEQ ID NO: 2;
wherein the genetic mutation is located in an HMT1 gene or homolog thereof
(v) comprising a nucleic acid sequence having at least 80% sequence identity to the nucleic acid sequence set forth as SEQ ID NO: 9 and encoding an active homocysteine S-methyltransferase;
(vi) comprising the nucleic acid sequence set forth as SEQ ID NO: 9;
(vii) encoding a homocysteine S-methyltransferase comprising an amino acid sequence having at least 80% sequence identity to an amino acid sequence set forth as SEQ ID NO: 10, wherein the homocysteine S-methyltransferase is active; and/or
(viii) encoding a homocysteine S-methyltransferase comprising an amino acid sequence set forth as SEQ ID NO: 10; and/or
wherein the genetic mutation is located in an HMT3 gene or homolog thereof
(ix) comprising a nucleic acid sequence having at least 80% sequence identity to the nucleic acid sequence set forth as SEQ ID NO: 13 and encodes an active homocysteine S-methyltransferase;
(x) comprising the nucleic acid sequence set forth as SEQ ID NO: 13;
(xi) encoding a homocysteine S-methyltransferase comprising an amino acid sequence having at least 80% sequence identity to an amino acid sequence set forth as SEQ ID NO: 14, wherein the homocysteine S-methyltransferase is active; and/or
(xii) encoding a homocysteine S-methyltransferase comprising an amino acid sequence set forth as SEQ ID NO: 14.

9. The method of any one of claims 5-8, wherein the plant, plant part, or plant cell comprising the genetic mutation comprises:
(i) an in-frame deletion of nucleotides 952 to 957 of ASA1 having the nucleic acid sequence set forth as SEQ ID NO: 1, or a nucleic acid sequence comprising SEQ ID NO: 3 or encoding an amino acid sequence of SEQ ID NO: 6 when the deletion of (i) is introduced;
(ii) an in-frame deletion of nucleotides 948 to 959 of ASA1 having the nucleic acid sequence set forth as SEQ ID NO: 1, or a nucleic acid sequence comprising SEQ ID NO: 4 or encoding an amino acid sequence of SEQ ID NO: 7 when the deletion of (ii) is introduced;
(iii) an in-frame deletion of nucleotides 938 to 961 of ASA1 having the nucleic acid sequence set forth as SEQ ID NO: 1, or a nucleic acid sequence comprising SEQ ID NO: 5 or encoding an amino acid sequence of SEQ ID NO: 8 when the deletion of (iii) is introduced;
(iv) a deletion of nucleotides 712 to 714 of HMT1 having the nucleic acid sequence set forth as SEQ ID NO: 9, or a nucleic acid sequence comprising SEQ ID NO: 11 when the deletion of (iv) is introduced;
(v) a deletion of nucleotides 695 to 760 of HMT1 having the nucleic acid sequence set forth as SEQ ID NO: 9, or a nucleic acid sequence comprising SEQ ID NO: 12 when the deletion of (v) is introduced;
(vi) a deletion of nucleotides 2783 to 2784 of HMT3 having the nucleic acid sequence set forth as SEQ ID NO: 13, or a nucleic acid sequence comprising SEQ ID NO: 15 when the deletion of (vi) is introduced; or
(vii) a deletion of nucleotides 2778 to 2790 of HMT3 having the nucleic acid sequence set forth as SEQ ID NO: 13, or a nucleic acid sequence comprising SEQ ID NO: 16 when the deletion of (vii) is introduced, optionally
wherein the plant, plant part, or plant cell is from a legume or is soybean (*Glycine max*) or pea (*Pisum sativum*)*.*

10. A plant, plant part, plant cell, or population of plants, plant parts, or plant cells produced by the method of any one of claims 5-9, comprising increased Trp and/or Met content as compared to a control.

11. A seed or protein composition produced from the plant, plant part, plant cell, or population of plants, plant parts, or plant cells of any one of claims 1-4 and 10, wherein the seed or protein composition comprises increased Trp and/or Met content compared to a control.

12. The plant, plant part, plant cell, or population of plants, plant parts, or plant cells of claim 10, or the seed or protein composition of claim 11, wherein the Trp and/or Met content of the plant, plant part, plant cell, the population of plants, plant parts, plant cells, or the seed or protein composition is 1.1-fold to 10-fold as compared to the control and/or wherein S-methylmethionine (SMM) level in vegetative tissue of the plant, plant part, or the population of plants or plant parts is increased as compared to the control.

13. A nucleic acid molecule comprising a nucleic acid sequence of a mutated anthranilate synthase alpha subunit (ASA) gene,
wherein the mutated ASA gene comprises one or more insertions, substitutions, or deletions as compared to a corresponding ASA gene without the mutations,
wherein the nucleic acid sequence:
(i) comprises at least 80% sequence identity to a nucleic acid sequence set forth as SEQ ID NO: 1 and encodes an active ASA;
(ii) comprises the nucleic acid sequence set forth as SEQ ID NO: 1;
(iii) encodes an ASA comprising an amino acid sequence having at least 80% sequence identity to an amino acid sequence set forth as SEQ ID NO: 2, wherein the ASA is active; and/or
(iv) encodes an ASA comprising an amino acid sequence set forth as SEQ ID NO: 2 and a mutation therein;
wherein the mutated ASA gene comprises:
(v) an in-frame deletion of nucleotides 952 to 957 of ASA1 having the nucleic acid sequence set forth as SEQ ID NO: 1, or a nucleic acid sequence comprising SEQ ID NO: 3 when the deletion of (v) is introduced;
(vi) an in-frame deletion of nucleotides 948 to 959 of ASA1 having the nucleic acid sequence set forth as SEQ ID NO: 1, or a nucleic acid sequence comprising SEQ ID NO: 4 when the deletion of (vi) is introduced; or
(vii) an in-frame deletion of nucleotides 938 to 961 of ASA1 having the nucleic acid sequence set forth as SEQ ID NO: 1, or a nucleic acid sequence comprising SEQ ID NO: 5 when the deletion of (vii) is introduced; and/or
wherein the encoded active anthranilate synthase is insensitive to or has reduced sensitivity to Trp feedback inhibition.

14. A genetic construct comprising, in operable linkage:
(i) a promoter that is functional in a plant cell; and
(ii) the nucleic acid molecule of claim 13.

15. A cell comprising the nucleic acid molecule of claim 13, or the genetic construct of claim 14.
